# EUROPEAN PATENT SPECIFICATION

(11) **EP 3 142 690 B1**
(45) Date of publication and mention of the grant of the patent: **23.02.2022**
(21) Application number: 15733563.9
(22) Date of filing: 08.05.2015
(51) Int. Cl.: A61K 39/00, A61K 39/39

(54) **COMBINATION THERAPY FOR TREATING CANCER WITH A RECOMBINANT POXVIRUS EXPRESSING A TUMOR ANTIGEN AND AN IMMUNE CHECKPOINT MOLECULE ANTAGONIST OR AGONIST**
KOMBINATIONSTHERAPIE ZUR BEHANDLUNG VON KREBS MIT EINEM EIN TUMOR-ANTIGEN EXPRIMIERENDEN REKOMBINANTEN POCKENVIRUS UND EINEM IMMUM-CHECKPOINT-MOLEKÜLANTAGONIST ODER -AGONIST
POLYTHÉRAPIE DESTINÉE À TRAITER LE CANCER AVEC UN POXVIRUS RECOMBINANT EXPRIMANT UN ANTIGÈNE TUMORAL ET UN ANTAGONISTE OU AGONISTE D'UNE MOLÉCULE INHIBITRICE DE POINTS DE CONTRÔLE IMMUNITAIRES

(30) Priority: 13.05.2014 US 201461992788 P
(43) Date of publication of application: 22.03.2017
(73) Proprietor: Bavarian Nordic A/S, 3490 Kvistgaard (DK)
(72) Inventor: FOY, Susan, Mountain View, CA 94041 (US); MANDL, Stefanie, San Francisco, CA 94122 (US); ROUNTREE, Ryan, San Jose, CA 95118 (US); FRANZUSOFF, Alex, El Granada, CA 94018 (US)
(74) Representative: Bendiksen, Henrik
(86) International application number: PCT/US2015/029855
(87) International publication number: WO 2015/175334

(56) References cited:
- WO-A1-2006/121168
- ESPENSCHIED JONATHAN ET AL: "CTLA-4 blockade enhances the therapeutic effect of an attenuated poxvirus vaccine targeting p53 in an established murine tumor model.", JOURNAL OF IMMUNOLOGY, vol. 170, no. 6, 15 March 2003 (2003-03-15), pages 3401-3407, XP002443898, ISSN: 0022-1767
- MADAN RAVI A ET AL: "Combination of vaccine and immune checkpoint inhibitor is safe with encouraging clinical activity", ONCOIMMUNOLOGY, vol. 1, no. 7, October 2012 (2012-10), pages 1167-1168, XP002735512, ISSN: 2162-4011
- GAMEIRO S R ET AL: "Cancer vaccines targeting carcinoembryonic antigen: State-of-the-art and future promise", EXPERT REVIEW OF VACCINES 2013 EXPERT REVIEWS LTD. GBR, vol. 12, no. 6, June 2013 (2013-06), pages 617-629, XP008177293, ISSN: 1476-0584

## Description

### FIELD OF THE INVENTION

The invention relates to recombinant poxviruses encoding a tumor antigen in combination with antagonists of immune checkpoint molecules for use in the treatment of cancers.

### BACKGROUND OF THE INVENTION

Recombinant poxviruses have been used as vaccines for infectious organisms and, more recently, for tumors. Mastrangelo et al. J Clin Invest. 2000; 105(8): 1031-1034. Two of these poxvirus groups, avipoxvirus and orthopoxvirus, have been shown to be effective at battling tumors and have been involved with potential cancer treatments.

One exemplary avipoxvirus species, fowlpox, has been shown to be a safe vehicle for human administrations as fowlpox virus enters mammalian cells and expresses proteins, but replicates abortively. Skinner et al. Expert Rev Vaccines. 2005 Feb;4(1):63-76. Additionally, the use of fowlpox virus as a vehicle for expression is being evaluated in numerous clinical trials of vaccines against cancer, malaria, tuberculosis, and AIDS.

Vaccinia, the most well-known of the orthopoxviruses, was used in the worldwide eradication of smallpox and has shown usefulness as a vector and/or vaccine. Recombinant Vaccinia Vector has been engineered to express a wide range of inserted genes, including several tumor associated genes such as p97, HER-2/neu, p53 and ETA (Paoletti, et al., 1993).

A useful strain of orthopoxvirus is the Modified Vaccinia Ankara (MVA) virus. MVA was generated by 516 serial passages on chicken embryo fibroblasts of the Ankara strain of vaccinia virus (CVA) (for review see Mayr, A., et al. Infection 3, 6-14 (1975)). As a consequence of these long-term passages, the genome of the resulting MVA virus had about 31 kilobases of its genomic sequence deleted and, therefore, was described as highly host cell restricted for replication to avian cells (Meyer, H. et al., J. Gen. Virol. 72, 1031-1038 (1991)). It was shown in a variety of animal models that the resulting MVA was significantly avirulent (Mayr, A. & Danner, K., Dev. Biol. Stand. 41: 225-34 (1978)). Additionally, this MVA strain has been tested in clinical trials as a vaccine to immunize against the human smallpox disease (Mayr et al., Zbl. Bakt. Hyg. I, Abt. Org. B 167, 375-390 (1987); Stickl et al., Dtsch. med. Wschr. 99, 2386-2392 (1974)). In these human studies, MVA had diminished virulence or infectiousness as compared to vaccinia-based vaccines, while MVA still induced a good specific immune response.

In the following decades, MVA was engineered for use as a viral vector for recombinant gene expression or as a recombinant vaccine (Sutter, G. et al., Vaccine 12: 1032-40 (1994).

Even though Mayr et al. demonstrated during the 1970s that MVA is highly attenuated and avirulent in humans and mammals, certain investigators have reported that MVA is not fully attenuated in mammalian and human cell lines since residual replication might occur in these cells. (Blanchard et al., J Gen Virol 79, 1159-1167 (1998); Carroll & Moss, Virology 238, 198-211 (1997); Altenberger, U.S. Pat. No. 5,185,146; Ambrosini et al., J Neurosci Res 55(5), 569 (1999)). It is assumed that the results reported in these publications have been obtained with various known strains of MVA, since the viruses used essentially differ in their properties, particularly in their growth behavior in various cell lines. Such residual replication is undesirable for various reasons, including safety concerns in connection with use in humans.

Strains of MVA having enhanced safety profiles for the development of safer products, such as vaccines or pharmaceuticals, have been described. See International PCT publication WO2002042480 (see also e.g. U.S. Pat. Nos. 6,761,893 and 6,913,752). Such strains are capable of reproductive replication in non-human cells and cell lines, especially in chicken embryo fibroblasts (CEF), but are not capable of significant reproductive replication in certain human cell lines known to permit replication with known vaccinia strains. Such cell lines include a human keratinocyte cell line, HaCat (Boukamp et al. J Cell Biol 106(3): 761-71 (1988)), a human cervix adenocarcinoma cell line, HeLa (ATCC No. CCL-2), a human embryo kidney cell line, 293 (ECACC No. 85120602), and a human bone osteosarcoma cell line, 143B (ECACC No. 91112502). Such strains are also not capable of significant reproductive replication *in vivo,* for example, in certain mouse strains, such as the transgenic mouse model AGR 129, which is severely immune-compromised and highly susceptible to a replicating virus. See U.S. Pat. Nos. 6,761,893. One such MVA strain and its derivatives and recombinants, referred to as "MVA-BN," has been described.. See International PCT publication WO2002042480 (see also e.g. U.S. Pat. Nos. 6,761,893 and 6,913,752).

MVA and MVA-BN have each been engineered for use as a viral vector for recombinant gene expression or as a recombinant vaccine. *See, e.g.,* Sutter, G. et al., Vaccine 12: 1032-40 (1994), International PCT publication WO2002042480 (see also e.g U.S. Pat. Nos. 6,761,893 and 6,913,752).

Certain approaches to cancer immunotherapy have included vaccination with tumor-associated antigens. In certain instances, such approaches have included use of a delivery system to promote host immune responses to tumor-associated antigens. In certain instances, such delivery systems have included recombinant viral vectors. *See, e.g.,* Harrop et al., Front. Biosci. 11:804-817 (2006); Arlen et al., Semin. Oncol. 32:549-555 (2005); Liu et al., Proc. Natl. Acad. Sci. USA 101 (suppl. 2):14567-14571 (2004).

HER-2 is a tumor-associated antigen that is over-expressed in tumor cells of a number of cancer patients. Immunization with various HER-2 polypeptides has been used to generate an immune response against tumor cells expressing this antigen. *See, e.g.,* Renard et al., J. Immunology 171:1588-1595 (2003); Mittendorf et al., Cancer 106:2309-2317 (2006).

An MVA encoding a HER-2 antigen, MVA-BN-HER2, has been shown to exert potent anti-tumor efficacy in a murine model of experimental pulmonary metastasis, despite a strong tumor-mediated immunosuppressive environment characterized by a high frequency of regulatory T cells (T_{reg}) in the lungs. Mandl et al., Cancer Immunol Immunother (2012) 61:19-29. The recombinant MVA was reported to induce strongly Th1-dominated HER-2-specific antibody and T-cell responses. The anti-tumor activity was characterized by an increased infiltration of lungs with highly activated, HER-2-specific, CD8+CD11c+ T cells, and was accompanied by a decrease in the frequency of T_{reg} cells in the lung, resulting in a significantly increased ratio of effector T cells to T_{reg} cells.

MVA-BN-HER2 has also been shown to be safe and break tolerance to induce specific T and B cell responses in human clinical studies in a metastatic setting. Guardino et al., Cancer Research: December 15, 2009; Volume 69, Issue 24, Supplement 3.

Trastuzumab (Herceptin) is a humanized monoclonal antibody (mAb) targeting the extra-cellular domain of HER2, and has shown clinical efficacy in HER2-positive breast cancer. Wang et al., Cancer Res. 2012 September 1; 72(17): 4417-4428. However, a significant number of patients fail to respond to initial trastuzumab treatment and many trastuzumab-responsive tumors develop resistance after continuous treatment. #

Inhibitory receptors on immune cells are pivotal regulators of immune escape in cancer. Woo et al., Cancer Res; 72(4); 917-27, 2011. Among these inhibitory receptors, CTLA-4 (cytotoxic T-lymphocyte-associated protein 4) serves as a dominant off-switch while other receptors such as PD-1 (programmed death 1, CD279), LAG-3 (lymphocyte activation gene, CD223), and TIM-3 (T-cell immunoglobulin domain and mucin domain-3) seem to serve more subtle rheostat functions.

CTLA-4 is an immune checkpoint molecule, which is up-regulated on activated T-cells. Mackiewicz, Wspolczesna onkol 2012; 16 (5):363-370. CTLA-4 is a negative co-stimulatory molecule expressed on activated T-cells that inhibits their proliferation. *See* Mellman, Nature 2011; 480:480-9). An anti-CTLA4 mAb can block the interaction of CTLA-4 with CD80/86 and switch off the mechanism of immune suppression and enable continuous stimulation of T-cells by DCs. Two IgG mAb directed against CTLA-4, ipilimumab and tremelimumab, have been used in clinical trials in patients with melanoma.

While these recent studies have indicated that using IgG mAb directed against CTLA-4 can provide therapeutic benefit to melanoma patients, treatments with these anti-CTLA-4 antibodies have shown high levels of immune-related adverse events. Mellman et al. Nature 2011; 480(7378): 480-489. Such adverse events have been characterized as on-target toxicities where treated patients developed adverse effects including colitis and hypophysitis, as well as liver related problems. Madan et al., Oncoimmunology 2012; 1(7):1167-1168 presents a study to check the safety of coadministering ipilimumab with a MVA-BN vaccine encoding PSA.

Another human mAb modulating the immune system is BMS-936558 (MDX-1106) directed against the death-1 receptor (PD-1R), the ligand of which (PD-1L) can be directly expressed on melanoma cells. PD-1R is a part of the B7:CD28 family of co-stimulatory molecules that regulate T-cell activation and tolerance, and thus anti-PD-lR can play a role in breaking tolerance. Engagement of the PD-1/PD-L1 pathway results in inhibition of T-cell effector function, cytokine secretion and proliferation. Turnis et al., OncoImmunology 1:7, 1172-1174; 2012. High levels of PD-1 are associated with exhausted or chronically stimulated T cells. *Id.* Moreover, increased PD-1 expression correlates with reduced survival in cancer patients.

While these recent studies have suggested that PD-1 expression can be linked to survival rates in cancer, early studies with inhibition of PD-1 in treating cancers have shown a wide variety of adverse side effects. Mellman et al. Nature 2011; 480(7378): 480-489; see also Chow, Am Soc Clin Oncol Educ Book, 2013, "Exploring novel immune-related toxicities and endpoints with immune-checkpoint inhibitors in non-small cell lung cancer".

LAG-3 is a negative co-stimulatory molecule expressed on various lymphoid cells. Under inflammatory conditions (such as IFN-gamma stimulation) both LAG-3 and MHC class II are up-regulated (Triebel Trends Immunol 2003;24:619-22).

TIM-3, is expressed on T-cells during chronic infections and cancer (Jin Proc Natl Acad Sci 2010;107:14733-8, Baghadi Cancer Immunol Immunother 2013;62:629-37.

There is clearly a substantial unmet medical need for additional cancer treatments, including active immunotherapies and cancer vaccines. Furthermore, in view of the adverse side effects seen many current cancer therapies, such as checkpoint inhibitor therapy, a need in the art exists for therapies that retain efficacy at lower dosages. These lower dosage therapies can help to reduce and/or eliminate these adverse effects.

### BRIEF SUMMARY OF THE INVENTION

The invention encompasses compositions for use in treating human cancer patients.

In one embodiment, the invention encompasses a pharmaceutical combination or medicament for use in treating a human cancer patient, the combination or medicament comprises (a) a therapeutical effective amount of recombinant poxvirus encoding a polypeptide comprising at least one tumor-associated antigen (TAA) selected from the group consisting of carcinoembryonic (CEA), mucin 1 cell surface associated (MUC-1), prostate specific antigen (PSA), human epidermal growth factor 2 (HER-2), and Brachyury; and (b) a subtherapeutically effective amount of an immune checkpoint antagonist that is a PD-1 antagonist or a CTLA-4 antagonist, wherein the pharmaceutical combination or medicament comprises a PD-1 antagonist and a CTLA-4 antagonist, and wherein each of said PD-1 antagonist and CTLA-4 antagonist is an antibody, an antisense RNA, or an siRNA.

In one preferred embodiment, the recombinant poxvirus is a recombinant orthopoxvirus or a recombinant avipoxvirus.

In a more preferred embodiment, the recombinant orthopoxivirus is a recombinant vaccinia virus or a recombinant modified Vaccinia Ankara (MVA) virus. In another preferred embodiment, the recombinant orthopoxvirus is MVA-BN.

In another preferred embodiment, the recombinant avipoxvirus is a recombinant fowlpox virus.

In yet another embodiment, the cancer treatments described herein can be directed against cancers such as, but not limited to, breast cancer, lung cancer, gastric cancer, kidney cancer, liver cancer, melanoma, pancreatic cancer, prostate cancer, ovarian cancer, colorectal cancer, or combinations thereof.

Additional advantages of the invention will be set forth in part in the description which follows, and in part will be obvious from the description or may be learned by practice of the invention. The advantages of the invention will be realized and attained by means of the elements and combinations particularly pointed out in the appended claims.

The accompanying drawings serve to explain the principles of the invention.

### BRIEF DESCRIPTION OF THE DRAWINGS

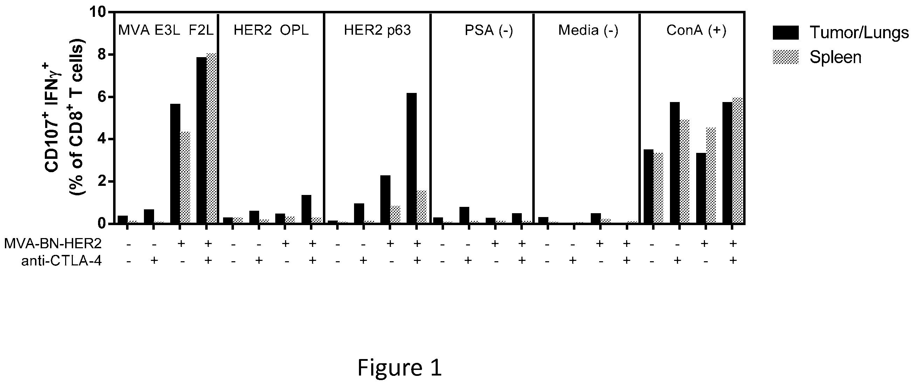

Figure 1. MVA-BN-HER2 and anti-CTLA-4 increase the tumor infiltrating antigen specific CD8+ T-cells. Mice were implanted as described in Example 2 and were either untreated or treated with 200 µg anti-CTLA-4 (in 100 µL PBS, i.p.) on day 3 and 18, and/or 1x107 Inf.U MV-BN-HER2 (7.1 µL, t.s.) on day 4 and 18. On day 25, tumor/lungs or spleens were pooled (4 mice/group) and re-stimulated overnight to measure virus and tumor antigen specific responses.

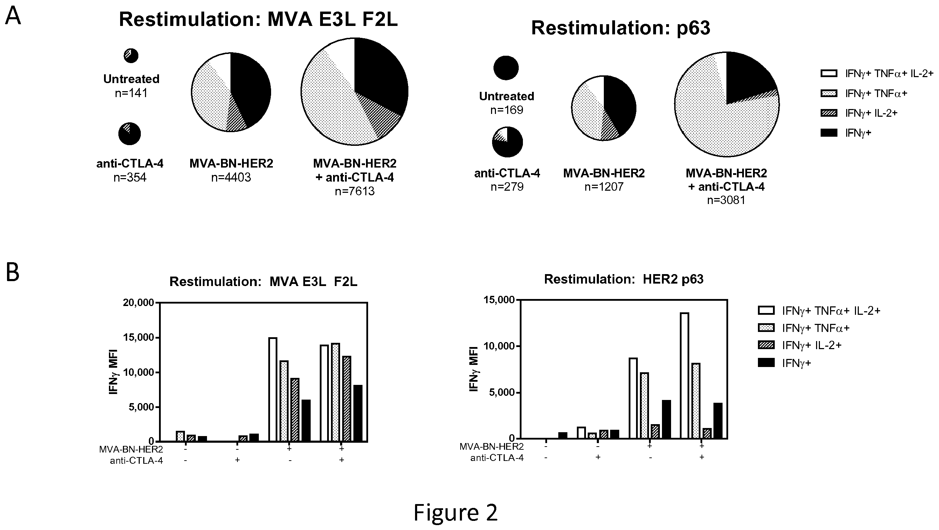

Figure 2. Treatment with MVA-BN-HER2 increased the magnitude and quality of tumor antigen and virus specific T-cells in the spleen. Mice were implanted as described in Example 3. A) Pie charts are area weighted to reflect the number of IFNγ+ cells per million CD8+ T-cells. B) IFNγ MFI increases with polyfunctional T-cells with combination therapy.

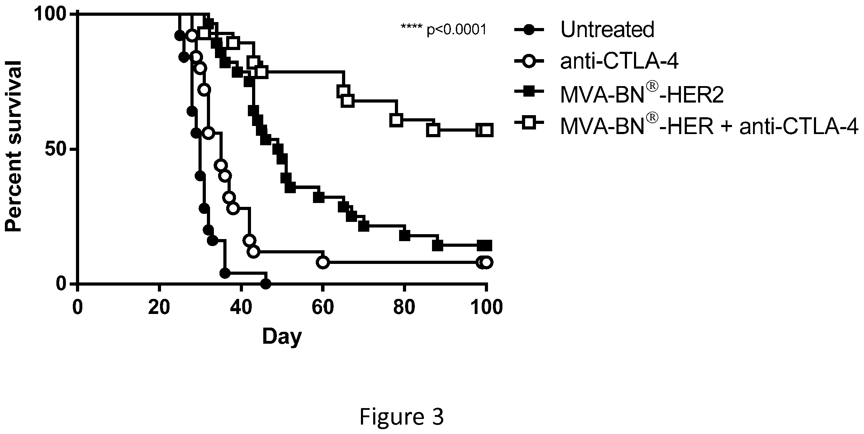

Figure 3. MVA-BN-HER2 synergizes with anti-CTLA-4 to eliminate tumors and increase survival in an experimental lung metastasis model. Mice were implanted on day 1 as described in Example 4 and treated with MVA-BN-HER2 on days 4 and 18, and anti-CTLA-4 on days 3 and 17. ^{∗∗∗∗} p<0.0001, Log-Rank Test.

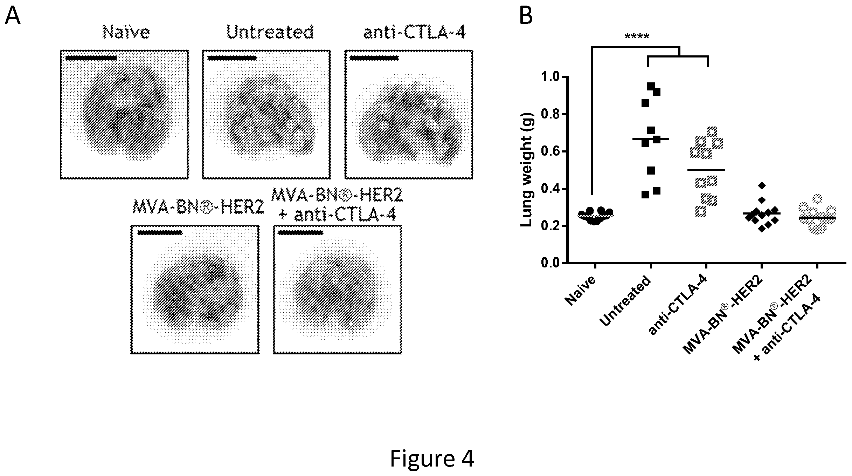

Figure 4. Mice with CT26-HER-2 lung tumors were treated as described in Example 5 and tumor burden analyzed on day 25. A) Mice were euthanized and perfused through the trachea with Trypan Blue. Lungs were removed and briefly submerged in Hydrogen Peroxide then PBS. Tumors are visible as small masses in Untreated and anti-CTLA-4 treated lungs. There were no visible tumors in mice treated with MVA-BN-HER2. Scale bar equals 1 cm. B) Mice treated with MVA-BN-HER2 have similar lung weight to Naive mice on day 25, while lung weight is significantly greater in Untreated and anti-CTLA-4 treated mice on day 25. ^{∗∗∗∗} p<0.0001, One-Way ANOVA with Dunnett's Multiple Comparisons test.

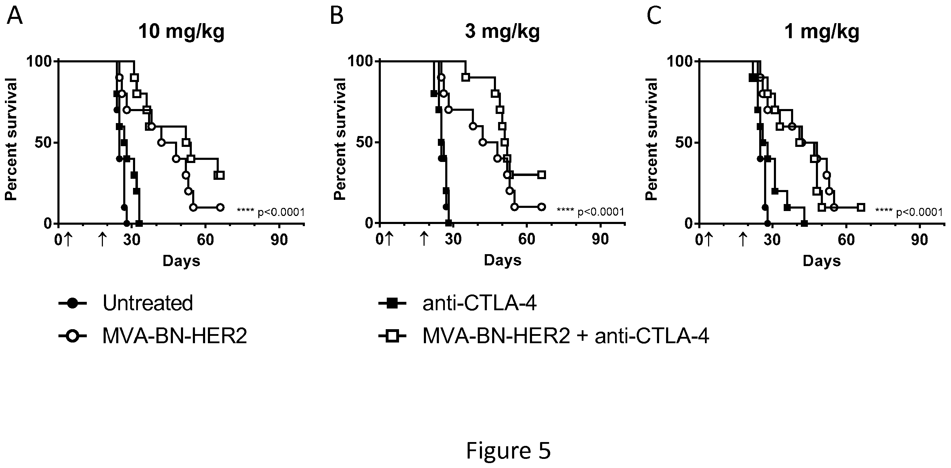

Figure 5. Mice were implanted with CT26-HER-2 tumors as described in Example 6. Mice were treated with MVA-BN-HER2 and anti-CTLA-4 on days 4 and 18 at 200 µg (A, 10 mg/kg), 66 ug (B, 3 mg/kg), or 22 µg (C, 1 mg/kg) i.p. in 100 µL PBS. ^{∗∗∗∗} p<0.0001, Log-Rank Test.

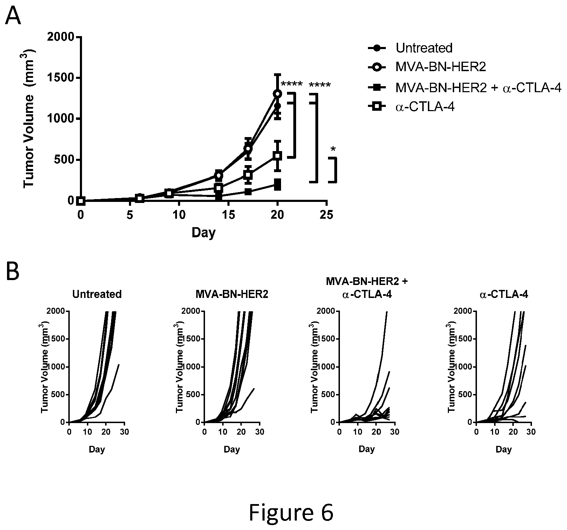

Figure 6. Mice were implanted as described in Example 7. Mice were treated on day 1 and 15 with MVA-BN-HER2 (1E7 Inf. U. in 100 µL TBS, s.c. at the tail base) and 22 µg anti-CTLA-4 (1 mg/kg) on days 1 and 15. Results demonstrate that MVA-BN-HER2 in combination with low dose anti-CTLA-4 significantly reduced tumor burden by day 20 compared to other treatments.

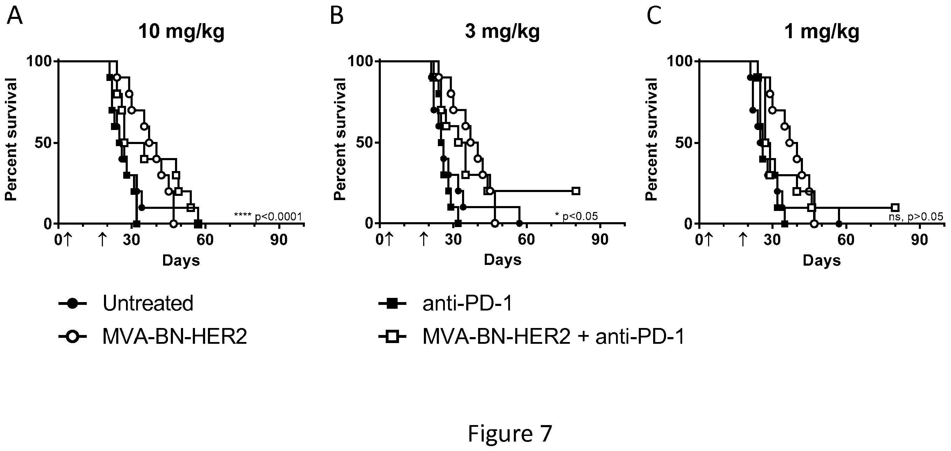

Figure 7. Mice were implanted with CT26-HER-2 tumors as described in Example 8. Mice were treated with MVA-BN-HER2 and anti-PD-1 on days 4 and 18 at 200 µg (A, 10 mg/kg), 66 ug (B, 3 mg/kg), or 22 µg (C, 1 mg/kg) i.p. in 100 µL PBS. ^{∗∗∗∗} p<0.0001, ^{∗} p<0.05, ns=not significant by Log-Rank Test.

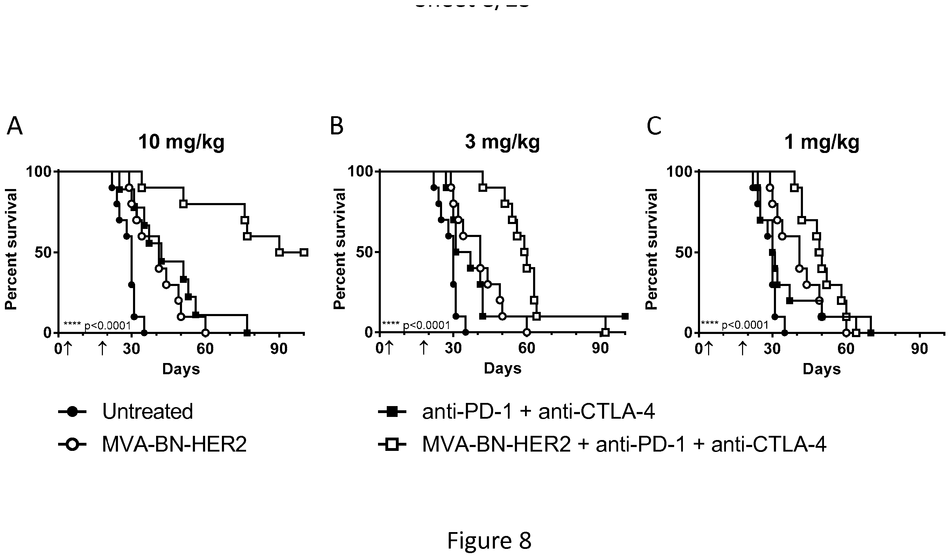

Figure 8. Female BALB/c mice (6-8 weeks old, ~ 20 g, Simonsen Laboratories, Gilroy,CA) were implanted as described in Example 9 . Mice were treated with MVA-BN-HER2 on day 4 and 18 and anti-CTLA-4 and anti-PD-1 on days 3 and 17 at 200 µg (A, 10 mg/kg), 66 ug (B, 3 mg/kg), or 22 µg (C, 1 mg/kg) each antibody i.p. in 100 µL PBS. ^{∗∗∗∗} p<0.0001, Log-Rank Test.

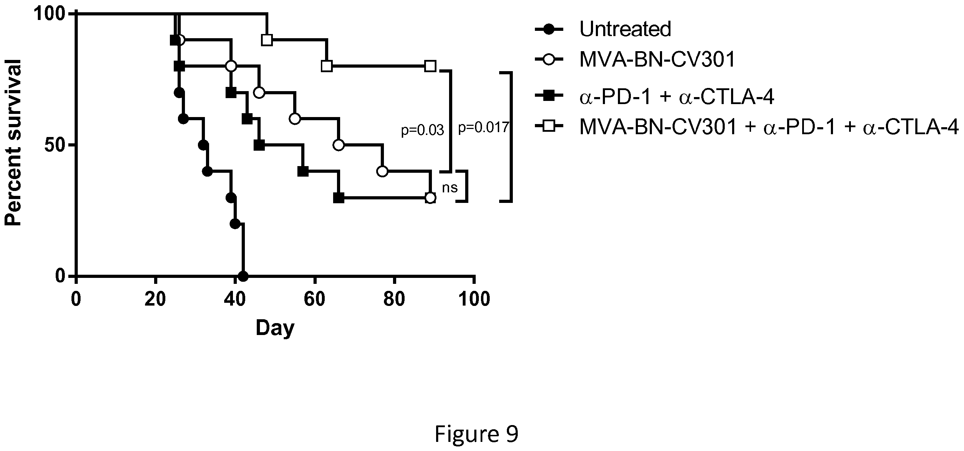

Figure 9. Female C57/BL6 mice (6-8 weeks old, ~ 20g, Simonsen Laboratories, Gilroy,CA) were implanted on day 1 as described in Example 10Mice were treated with MVA-BN-CV301 and treated with anti-CTLA-4 and anti-PD-1 (200 µg each) i.p. on days 4 and 18.

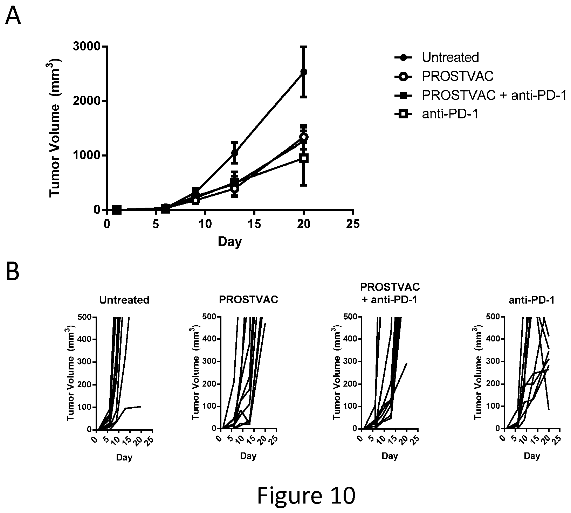

Figure 10. PROSTVAC and anti-PD-1 combination therapy in an E6 solid tumor model. Mice were implanted as described in Example 12. A) Mice were treated on day 1 with PROSTVAC-V, and days 8 and 15 with PROSTVAC-F. Anti-PD-1 was given on days 1 and 15. A) Average tumor volume in mice. B) Individual tumor growth in mice.

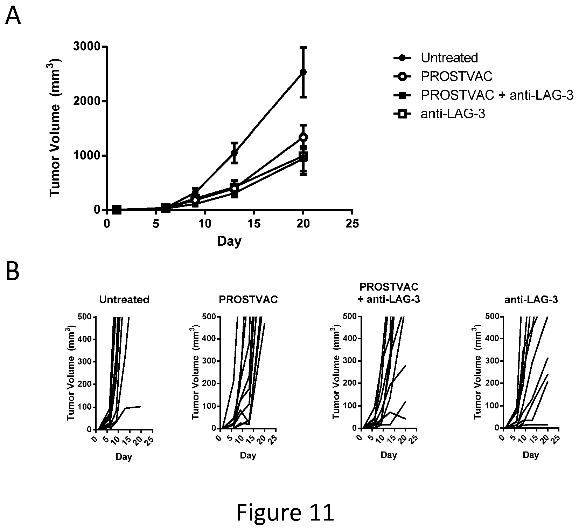

Figure 11. PROSTVAC and anti-LAG-3 combination therapy in an E6 solid tumor model. Mice were implanted as described in Example 13. A) Mice were treated on day 1 with PROSTVAC-V and days 8 and 15 with PROSTVAC-F. Anti-LAG-3 was given on days 1 and 15. A) Average tumor volume in mice. B) Individual tumor growth in mice.

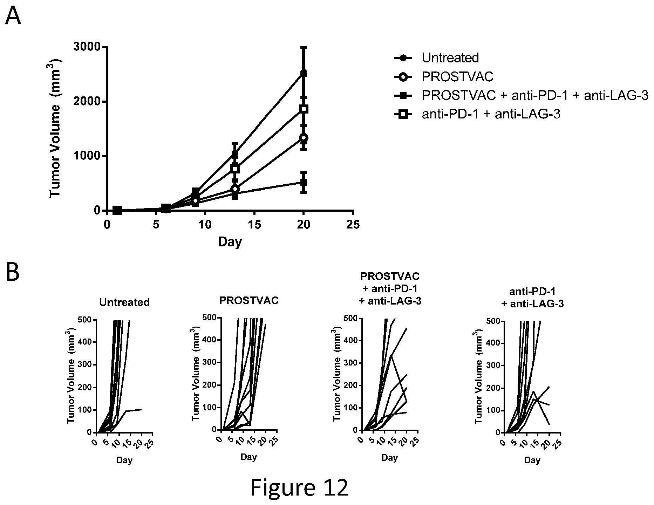

Figure 12. PROSTVAC in combination with anti-PD-1 and anti-LAG-3 in an E6 solid tumor model. Mice were implanted as described in Example 14. A) Mice were treated on day 1 with PROSTVAC-V and days 8 and 15 with PROSTVAC-F. Anti-PD-1 and anti-LAG-3 were given on days 1 and 15. A) Average tumor volume in mice. B) Individual tumor growth in mice.

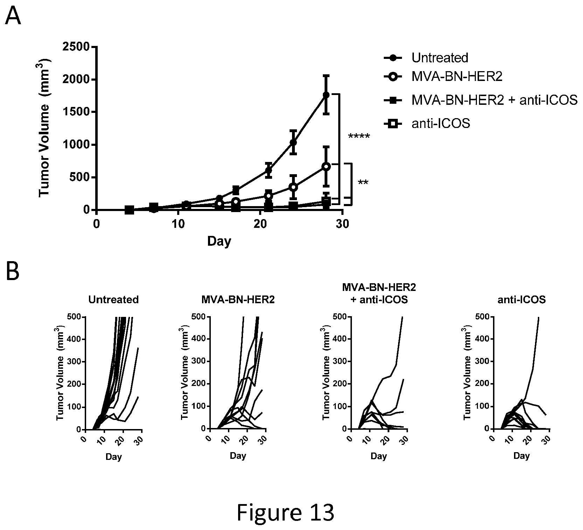

Figure 13. Mice were implanted as described in Example 15. Mice were treated with MVA-BN-HER2 on days 7 and 22 (1E7 Inf.U., t.s.), and anti-ICOS on days 1, 4, 8, 11, 15, 18, 22, 25 (200 µg i.p.). A) Average tumor growth. B) Tumor growth in individual mice.

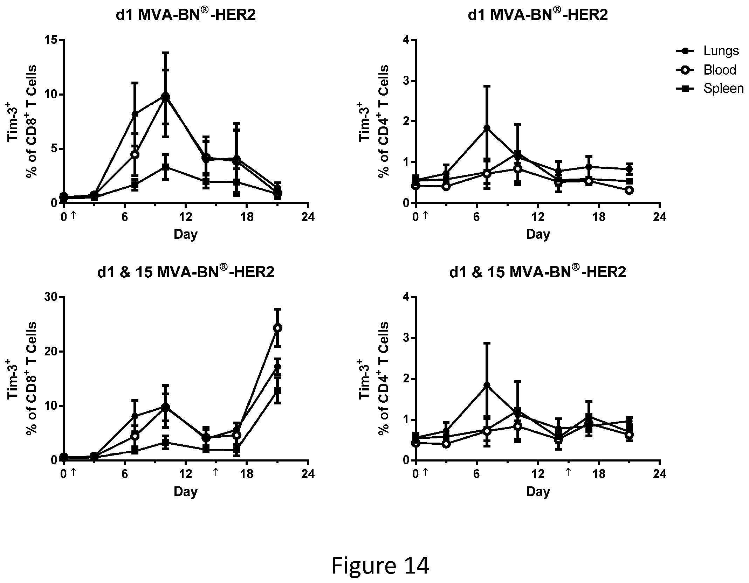

Figure 14. Tim-3 expression increases with MVA-BN-HER2 treatment. Mice were treated as described in Example 32. Tim-3 expression was measured in mice after day 1 and day 15 treatment with MVA-BN-HER2 (1E7 Inf.U., t.s.).

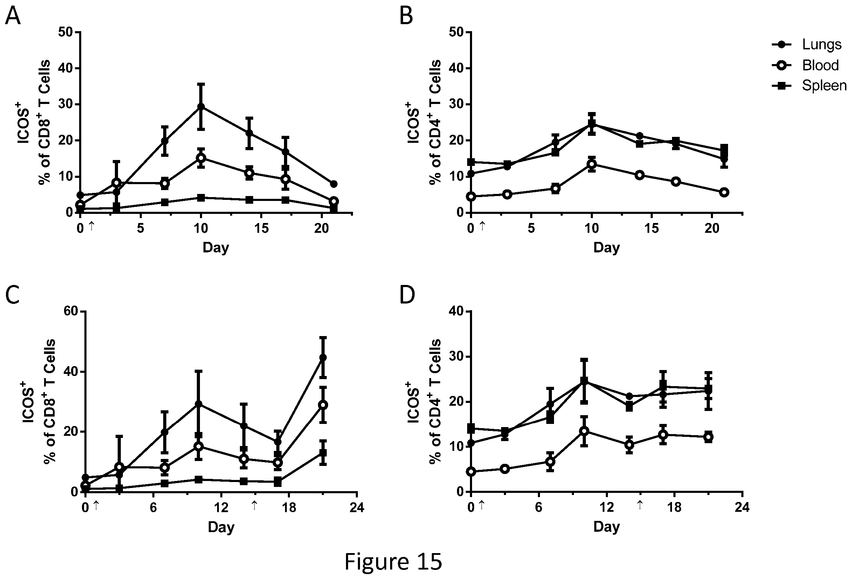

Figure 15. Mice were treated with as described in Example 33. ICOS increased in the lungs and blood on CD8+ T cells at day 10 (A) and in the lungs, blood, and spleen on CD4+ T-cells (B) after a single treatment with MVA-BN-HER2. With a second treatment of MVA-BN-HER2 on day 15, ICOS increased in the lungs, blood, and spleen on CD8+ T-cells (C), and CD4+ T-cells (D). Data shown as mean ± SEM, three mice per group at each time point.

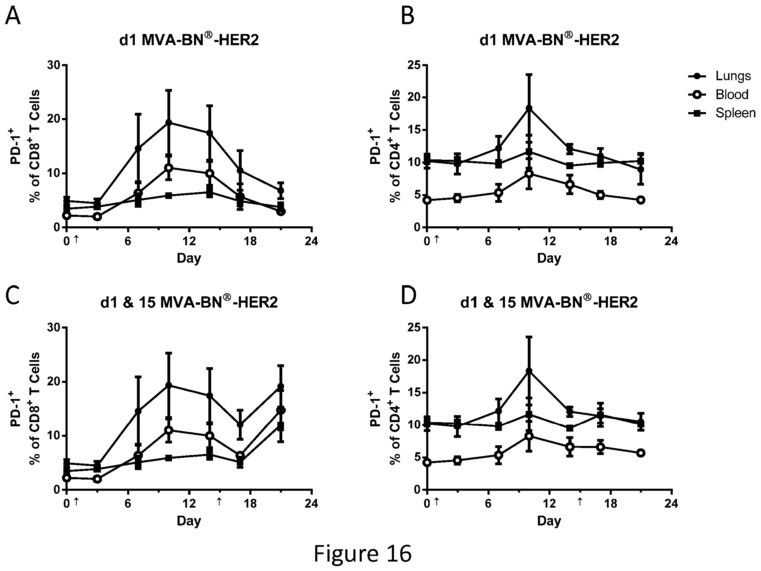

Figure 16. Mice were treated as described in Example 34. PD-1 expression increased on CD8+ T-cells in the lungs and blood with day 1 MVA-BN-HER2 Treatment (A). PD-1 expression increased further with a second treatment on Day 15 in the lungs, spleen and blood (C). PD-1 increased slightly on CD4+ T-cells in the lung after a single treatment (B) and remained stable after a second MVA-BN-HER2 treatment (D).

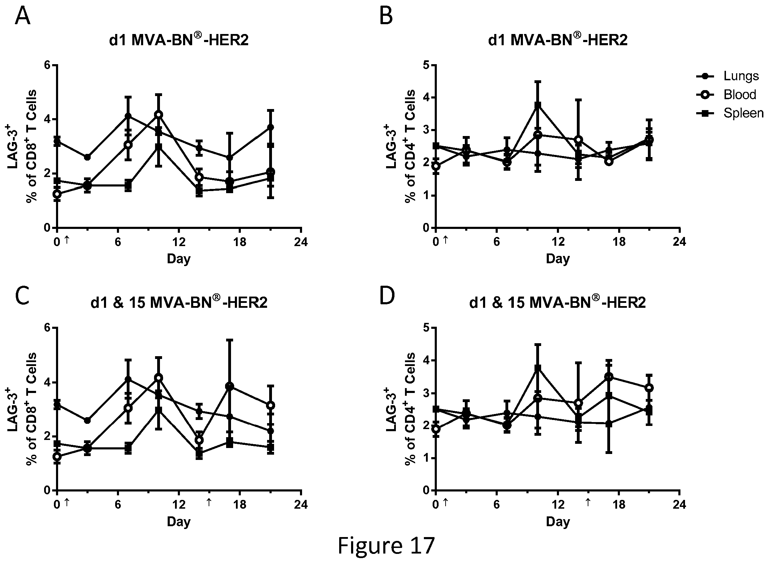

Figure 17. Mice were treated as described in Example 35 .LAG-3 expression increased in the lungs, spleen and blood on CD8+ T-cells after a d1 (A) or day 1 and 15 treatment (C) with MVA-BN-HER2. LAG-3 expression on CD4+ T-cells increased slightly after a day 1 (B) or day 1 and 15 (D) MVA-BN-HER2 treatment.

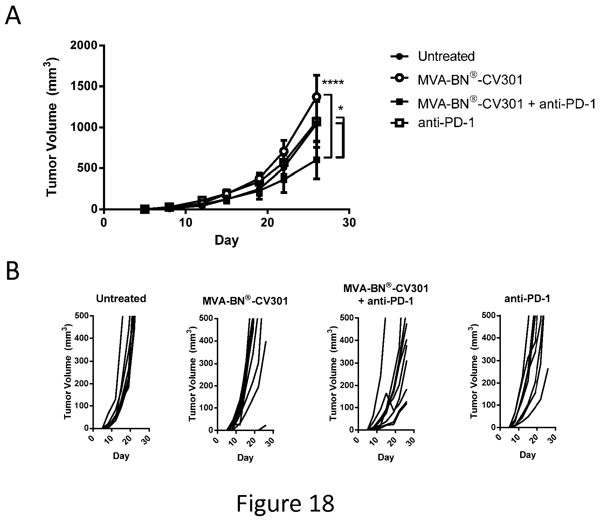

Figure 18. MVA-BN-CV301 and anti-PD-1 slow tumor growth in an MC38-CEA solid tumor model. Mice were implanted i.d. with MC38-CEA tumors and treated as described in Example 37. Mice were further treated with_MVA-BN-CV301 and anti-PD-1. A) Average tumor volume in mice. B) Individual tumor growth in mice.

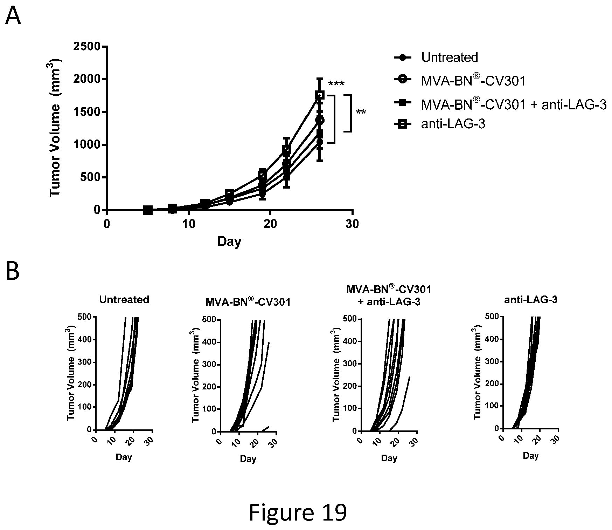

Figure 19. MVA-BN-CV301 and anti-LAG-3 combination therapy in an MC38-CEA solid tumor model. Mice were implanted i.d. with MC38-CEA tumors and treated as described in Example 38. Mice were further treated with_MVA-BN-CV301 and anti-LAG-3. A) Average tumor volume in mice. B) Individual tumor growth in mice.

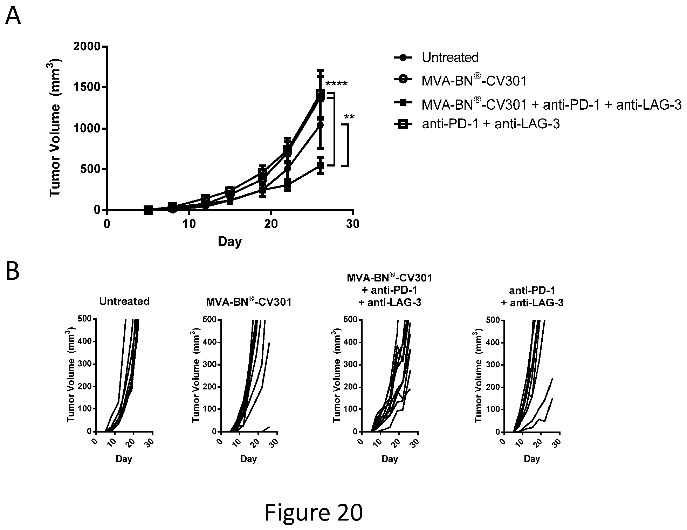

Figure 20. MVA-BN-CV-301 in combination with anti-PD-1 and anti-LAG-3. Mice were implanted i.d. with MC38-CEA tumors and treated as described in Example 39. Mice were further treated with_MVA-BN-CV-301 and anti-LAG-3 and anti-PDl. A) Average tumor volume in mice. B) Individual tumor growth in mice.

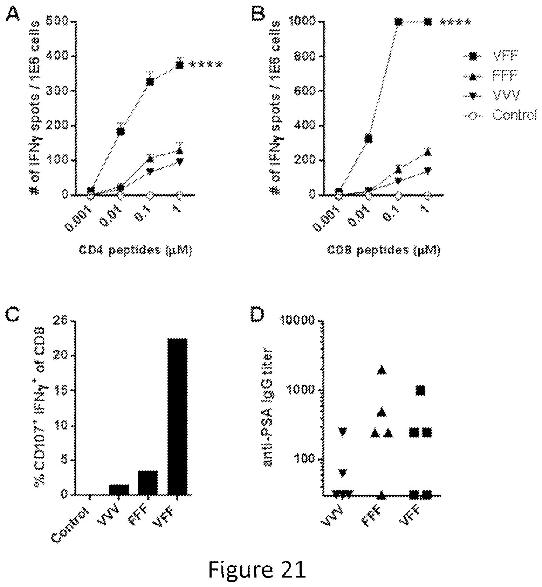

Figure 21. Mice were treated as described in Example 40. Pooled splenocytes were assayed for PSA-specific responses by IFN□ ELISPOT (A, B) and cytotoxic activity by flow cytometry (% CD107⁺ IFNγ⁺ CD8 T cells) (C). Anti-PSA IgG titers were determined by ELISA for each individual mouse (D). For ELISPOT, Graphs show representative data of four independently performed experiments.

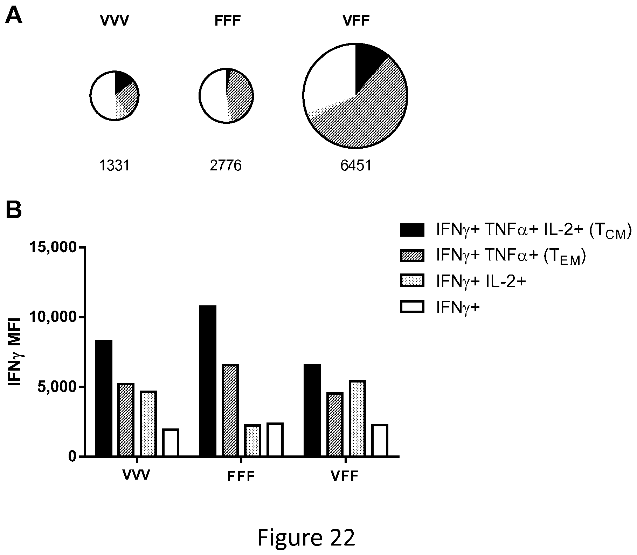

Figure 22. Mice were treated as described in Example 41. (A) The pie charts are weighted in size to reflect the numbers of detected cells (total numbers of PSA-specific CD8 per million T cells are indicated below each chart). (B) Amount of IFNγ production on a per cell basis as measured by mean fluorescence intensity (MFI). Graphs show representative data of two independently performed experiments.

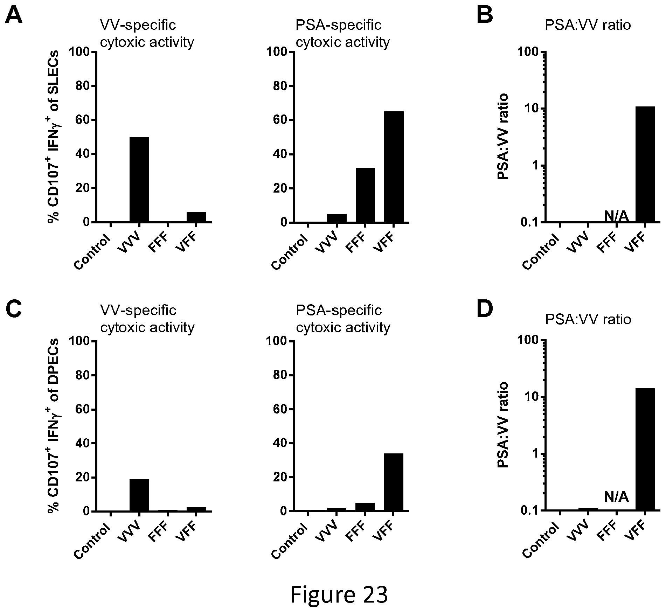

Figure 23. Mice were treated as described in Example 42. Pooled splenocytes were assayed for vaccinia virus (VV)-specific (A and C panels on left) or PSA-specific (A and C panels on right) cytotoxic activity by flow cytometry (% CD107+ IFNγ+ CD8 T cells) 14 days after the last treatment. Graphs show representative data of two independently performed experiments

### DETAILED DESCRIPTION OF THE INVENTION

A number of current clinical trial involve therapies employ vaccinia, Modified Vaccinia Ankara (MVA), and fowlpox-based vectors that were engineered to express one or more tumor-associated antigens (TAA). These vectors are used alone or in prime-boost strategies to generate an active immune response against a variety of cancers. PROSTVAC^{®} employs a prime-boost strategy using vaccinia and fowlpox expressing PSA and TRICOM^{™} and is currently in a global Phase III clinical trial (PROSPECT) for castration-resistant metastatic prostate cancer. CV301, or CV-301, employs a heterologous prime-boost strategy using vaccinia and fowlpox expressing MUC-1 antigen, CEA, and TRICOM^{™} and is currently in a Phase II clinical trial for Bladder Cancer.

MVA-BN-HER2 (Mandl et al, 2012), is in Phase I clinical trials for the treatment of HER-2⁺-breast cancer. This recombinant vector is derived from the highly attenuated Modified Vaccinia Ankara (MVA) virus stock known as MVA-BN. It expresses a modified form of HER-2 (designated HER2) consisting of the extracellular domain of HER-2 that has been engineered to include two universal T cell epitopes from tetanus toxin (TTp2 and TTp30) to facilitate the induction of effective immune responses against HER-2.

To further enhance the anti-tumor efficacy of the poxvirus-based immunotherapy, MVA-BN-HER2 was combined with a monoclonal antibody that blocks the activity of CTLA-4, an immune checkpoint protein that down-regulates T cell activation. In the CT26-HER-2 experimental lung metastasis model, the median survival time increased from 30 days in untreated mice to 49.5 days with MVA-BN-HER2 treatment while anti-CTLA-4 treatment by itself showed little survival benefit (median survival 35 days). In contrast, MVA-BN-HER2 in combination with anti-CTLA-4 significantly increased the survival to greater than 100 days (p<0.0001) in more than 50% of the mice. At 100 days, the lungs of the surviving mice were examined and there were no visible tumors.

PROSTVAC^{®} and MVA-BN-CV301 (MVA expressing CEA and MUC-1 with or without TRICOM) were each also tested in combination with various antagonist antibodies directed against PD-1 and LAG-3 in various tumor models. Combinations were found to enhance the effects of PROSTVAC^{®} and MVA-BN-CV301.

In view of the toxicity and adverse effects of cancer treatments involving antagonists of CTLA-4 and other immune checkpoint antagonists or agonists (*see,* e.g. Mellman et al. Nature 2011), dosage titration assays were conducted using a recombinant poxvirus encoding a tumor associated antigen such as MVA-BN-HER2 in combination with a monoclonal antibody that blocks the activity of CTLA-4. As illustrated and described herein, recombinant poxviral therapy such as MVA-BN-HER2 when combined with CTLA-4 inhibition, was able to achieve increased therapeutic effects when compared to cancer treatments using CTLA-4 inhibition alone or recombinant poxviral therapy alone. Most importantly, therapeutic efficacy was maintained by the combination treatment even at lower dosages.

To determine enhancement of anti-tumor efficacy of the poxvirus-based immunotherapy using additional immune checkpoint antagonists or agonists, MVA-BN-HER2 was combined with a monoclonal antibody that blocks the activity of CTLA-4, as well as an antagonistic antibody against PD-1. As illustrated and described herein, recombinant poxviral therapy when combined with a CTLA-4 antibody and PD-1 antagonist was able to achieve increased therapeutic effects when compared to cancer treatments using a combined CTLA-4 and PD-1 inhibition alone or recombinant poxviral therapy alone. Most importantly, therapeutic efficacy was maintained by the combination treatment even at lower dosages.

To further determine enhancement of anti-tumor efficacy of the poxvirus-based immunotherapy using additional immune checkpoint antagonists and agonists, dosage titration assays were conducted using a recombinant poxvirus encoding a tumor associated antigen, such as MVA-BN-HER2 in combination with various additional immune checkpoint antagonist or agonists as described herein. As illustrated and described herein, recombinant poxviral therapy when combined with immune checkpoint antagonists or agonist of the present application was able to achieve increased therapeutic effects. Most importantly, therapeutic efficacy was maintained by the combination treatment even at lower dosages.

In at least one aspect, the compositions of the present invention, together with their advantageous therapeutic efficacy also at low dosages, are designed to maximize the therapeutic benefits of immune checkpoint and poxviral cancer therapies while working to minimize the adverse side effects seen in the current cancer therapies. In one embodiment, these therapeutic benefits with reduced adverse effects are achieved through administering lower dosages of an immune checkpoint antagonist (e.g., CTLA-4 antagonist antibodies) in combination with a poxviral therapy. As provided for in the present invention, when combined with a poxviral therapy, immune checkpoint antagonists retain treatment efficacy even at lower dosages. Significantly, theses lower dosages where therapeutic efficacy was retained, can include dosages at which an immune checkpoint antagonist would be therapeutically ineffective when administered as a monotherapy.

In other embodiments, the present invention includes one or more dosing regimens and methods of administering the combination of a poxviral therapy and an immune checkpoint antagonist. In at least one aspect, the dosing regimens and methods of administration presented herein are designed to maximize the therapeutic benefits of immune checkpoint and poxviral cancer therapies while working to minimize the adverse side effects associated with cancer therapies.

### Poxvirus Encoding a Polypeptide Comprising a Tumor Antigen

In one embodiment, the recombinant poxvirus expressing a tumor antigen is preferably an orthopoxvirus such as, but not limited to, a vaccinia virus, a Modified Vaccinia Ankara (MVA) virus, or MVA-BN.

Examples of vaccinia virus strains are the strains Temple of Heaven, Copenhagen, Paris, Budapest, Dairen, Gam, MRIVP, Per, Tashkent, TBK, Tom, Bern, Patwadangar, BIEM, B-15, Lister, EM-63, New York City Board of Health, Elstree, Ikeda and WR. A preferred vaccinia virus (VV) strain is the Wyeth (DRYVAX) strain (U.S. Patent 7,410,644). Another preferred VV strain is a modified vaccinia virus Ankara (MVA) (Sutter, G. et al. [1994], Vaccine 12: 1032-40). Another preferred VV strain is MVA-BN.

Examples of MVA virus strains that are useful in the practice of the present invention and that have been deposited in compliance with the requirements of the Budapest Treaty are strains MVA 572, deposited at the European Collection of Animal Cell Cultures (ECACC), Vaccine Research and Production Laboratory, Public Health Laboratory Service, Centre for Applied Microbiology and Research, Porton Down, Salisbury, Wiltshire SP4 0JG, United Kingdom, with the deposition number ECACC 94012707 on January 27, 1994, and MVA 575, deposited under ECACC 00120707 on December 7, 2000. MVA-BN, deposited on Aug. 30, 2000 at the European Collection of Cell Cultures (ECACC) under number V00083008, and its derivatives, are additional exemplary strains.

Although MVA-BN is preferred for its higher safety (less replication competent), all MVAs are suitable for this invention. According to an embodiment of the present invention, the MVA strain is MVA-BN and its derivatives. A definition of MVA-BN and its derivatives is given in PCT/EP01/13628 which is incorporated by reference herein.

In one embodiment, the invention encompasses the use of recombinant orthopoxviruses, preferably a vaccinia virus (VV), a Wyeth strain, ACAM 1000, ACAM 2000, MVA, or MVA-BN for cancer therapy. Recombinant orthopoxviruses are generated by insertion of heterologous sequences into an orthopoxvirus.

In certain embodiments, the orthopoxvirus comprises at least one tumor-associated antigen (TAA) selected from a CEA, MUC-1, PSA, HER-2, or Brachyury antigen

In further embodiments, the tumor-associated antigen is modified to include one or more foreign T_{H} epitopes. Various cancer immunotherapeutic agents are described herein. In at least one aspect, the invention allows for the use of such agents in prime/boost vaccination regimens of humans and other mammals, including immunocompromised patients; and inducing both humoral and cellular immune responses, such as inducing a Th1 immune response in a pre-existing Th2 environment.

In certain embodiments, the MVA is MVA-BN, deposited on Aug. 30, 2000, at the European Collection of Cell Cultures (ECACC) under number V00083008, and described in International PCT publication WO2002042480 (see also e.g. U.S. Pat. Nos. 6,761,893 and 6,913,752) As described in those patent publications, MVA-BN does not reproductively replicate in cell lines 293, 143B, HeLa and HaCat. In particular, MVA-BN exhibits an amplification ratio of 0.05 to 0.2 in the human embryo kidney cell line 293. In the human bone osteosarcoma cell line 143B, MVA-BN exhibits an amplification ratio of 0.0 to 0.6. MVA-BN exhibits an amplification ratio of 0.04 to 0.8 in the human cervix adenocarcinoma cell line HeLa, and 0.02 to 0.8 in the human keratinocyte cell line HaCat. MVA-BN has an amplification ratio of 0.01 to 0.06 in African green monkey kidney cells (CV1: ATCC No. CCL-70).

The amplification ratio of MVA-BN is above 1 in chicken embryo fibroblasts (CEF: primary cultures) as described in International PCT publication WO2002042480 (see also e.g. U.S. Pat. Nos. 6,761,893 and 6,913,752). The virus can be easily propagated and amplified in CEF primary cultures with a ratio above 500.

In certain embodiments, a recombinant MVA is a derivative of MVA-BN. Such "derivatives" include viruses exhibiting essentially the same replication characteristics as the deposited strain (ECACC No. V00083008), but exhibiting differences in one or more parts of its genome. Viruses having the same "replication characteristics" as the deposited virus are viruses that replicate with similar amplification ratios as the deposited strain in CEF cells and the cell lines, HeLa, HaCat and 143B; and that show similar replication characteristics *in vivo,* as determined, for example, in the AGR129 transgenic mouse model.

In certain embodiments, the poxvirus is a recombinant vaccinia virus that contains additional nucleotide sequences that are heterologous to the poxvirus. In certain such embodiments, the heterologous sequences code for epitopes that induce a response by the immune system. Thus, in certain embodiments, the recombinant poxvirus is used to vaccinate against the proteins or agents comprising the epitope. In one embodiment, the epitope is a tumor-associated antigen, preferably, HER-2. In one embodiment, the HER-2 antigen comprises the sequence of SEQ ID NO:2.

In other embodiments, the epitope is a tumor-associated antigen selected from an antigen such as CEA, MUC-1, PSA, HER-2, or Brachyury.

In certain embodiments, a heterologous nucleic acid sequence encoding a tumor-associated antigen described herein is inserted into a non-essential region of the virus genome. In certain of those embodiments, the heterologous nucleic acid sequence is inserted at a naturally occurring deletion site of the MVA genome as described in PCT/EP96/02926. Methods for inserting heterologous sequences into the poxviral genome are known to a person skilled in the art.

In another embodiment, the recombinant poxvirus expressing a tumor antigen is an avipoxvirus, such as but not limited to a fowlpox virus.

The term "avipoxvirus" refers to any avipoxvirus, such as Fowlpoxvirus, Canarypoxvirus, Uncopoxvirus, Mynahpoxvirus, Pigeonpoxvirus, Psittacinepoxvirus, Quailpoxvirus, Peacockpoxvirus, Penguinpoxvirus, Sparrowpoxvirus, Starlingpoxvirus and Turkeypoxvirus. Preferred avipoxviruses are Canarypoxvirus and Fowlpoxvirus.

An example of a canarypox virus is strain Rentschler. A plaque purified Canarypox strain termed ALVAC (U.S. Pat. No. 5,766,598) was deposited under the terms of the Budapest treaty with the American Type Culture Collection (ATCC), accession number VR-2547. Another Canarypox strain is the commercial canarypox vaccine strain designated LF2 CEP 524 24 10 75, available from Institute Merieux, Inc.

Examples of a Fowlpox virus are strains FP-1, FP-5, TROVAC (U.S. Pat. No. 5,766,598), and POXVAC-TC (U.S. Patent 7,410,644). FP-1 is a Duvette strain modified to be used as a vaccine in one-day old chickens. The strain is a commercial fowlpox virus vaccine strain designated O DCEP 25/CEP67/2309 October 1980 and is available from Institute Merieux, Inc. FP-5 is a commercial fowlpox virus vaccine strain of chicken embryo origin available from American Scientific Laboratories (Division of Schering Corp.) Madison, Wis., United States Veterinary License No. 165, serial No. 30321.

Examples of vaccinia virus strains are the strains Temple of Heaven, Copenhagen, Paris, Budapest, Dairen, Gam, MRIVP, Per, Tashkent, TBK, Tom, Bern, Patwadangar, BIEM, B-15, Lister, EM-63, New York City Board of Health, Elstree, Ikeda and WR. A preferred vaccinia virus (VV) strain can include the Wyeth (DRYVAX) strain (U.S. Patent 7,410,644), ACAM 1000, or ACAM 2000. Another preferred VV strain is a modified vaccinia virus Ankara (MVA) (Sutter, G. et al. [1994], Vaccine 12: 1032-40). Another preferred VV strain is MVA-BN.

In certain embodiments, the avipox virus includes at least one tumor-associated antigen (TAA) selected from CEA, MUC-1, PSA, HER-2, or Brachyury antigen.

In other embodiments, the recombinant poxvirus expressing a tumor antigen is a combination of a vaccinia virus expressing a tumor antigen and an avipoxvirus, such as fowlpox, expressing a tumor antigen. It is contemplated that the vaccinia virus and fowlpox virus combination can be administered as a heterologous prime-boost regimen. In one non-limiting example, the heterologous prime-boost regimen is PROSTVAC^{®} or CV301.

For the preparation of vaccines, the poxvirus can be converted into a physiologically acceptable form. In certain embodiments, such preparation is based on experience in the preparation of poxvirus vaccines used for vaccination against smallpox, as described, for example, in Stickl, H. et al., Dtsch. med. Wschr. 99, 2386-2392 (1974).

An exemplary preparation follows. Purified virus is stored at -80°C with a titer of 5 x 10⁸ TCID₅₀/ml formulated in 10 mM Tris, 140 mM NaCl, pH 7.4. For the preparation of vaccine shots, e.g., 10²-10⁸ particles of the virus can be lyophilized in phosphate-buffered saline (PBS) in the presence of 2% peptone and 1% human albumin in an ampoule, preferably a glass ampoule. Alternatively, the vaccine shots can be prepared by stepwise, freeze-drying of the virus in a formulation. In certain embodiments, the formulation contains additional additives such as mannitol, dextran, sugar, glycine, lactose, polyvinylpyrrolidone, or other additives, such as, including, but not limited to, antioxidants or inert gas, stabilizers or recombinant proteins (e.g. human serum albumin) suitable for *in vivo* administration. The ampoule is then sealed and can be stored at a suitable temperature, for example, between 4°C and room temperature for several months. However, as long as no need exists, the ampoule is stored preferably at temperatures below -20°C.

In various embodiments involving vaccination or therapy, the lyophilisate is dissolved in 0.1 to 0.5 ml of an aqueous solution, preferably physiological saline or Tris buffer, and administered either systemically or locally, i.e., by parenteral, subcutaneous, intravenous, intramuscular, intranasal, intradermal, or any other path of administration known to a skilled practitioner. Optimization of the mode of administration, dose, and number of administrations is within the skill and knowledge of one skilled in the art.

In certain embodiments, attenuated vaccinia virus strains are useful to induce immune responses in immune-compromised animals, e.g., monkeys (CD4<400/µl of blood) infected with SIV, or immune-compromised humans. The term "immune-compromised" describes the status of the immune system of an individual that exhibits only incomplete immune responses or has a reduced efficiency in the defense against infectious agents.

### Certain Exemplary Tumor-Associated Antigens

In certain embodiments, an immune response is produced in a subject against a cell-associated polypeptide antigen. In certain such embodiments, a cell-associated polypeptide antigen is a tumor-associated antigen.

The term "polypeptide" refers to a polymer of two or more amino acids joined to each other by peptide bonds or modified peptide bonds. The amino acids may be naturally occurring as well as non-naturally occurring, or a chemical analogue of a naturally occurring amino acid. The term also refers to proteins, i.e. functional biomolecules comprising at least one polypeptide; when comprising at least two polypeptides, these may form complexes, be covalently linked, or may be non-covalently linked. The polypeptide(s) in a protein can be glycosylated and/or lipidated and/or comprise prosthetic groups.

Preferably, the tumor-associated antigen includes HER-2, PSA, CEA, MUC-1, or Brachyury alone or in combinations. Such exemplary combination may include CEA and MUC-1, also known as CV301.

Numerous tumor-associated antigens are known in the art. Exemplary tumor-associated antigens include, but are not limited to, 5 alpha reductase, alpha-fetoprotein, AM-1, APC, April, BAGE, beta-catenin, Bcl12, bcr-abl, CA-125, CASP-8/FLICE, Cathepsins, CD19, CD20, CD21, CD23, CD22, CD33 CD35, CD44, CD45, CD46, CD5, CD52, CD55, CD59, CDC27, CDK4, CEA, c-myc, Cox-2, DCC, DcR3, E6/E7, CGFR, EMBP, Dna78, farnesyl transferase, FGF8b, FGF8a, FLK-1/KDR, folic acid receptor, G250, GAGE-family, gastrin 17, gastrin-releasing hormone, GD2/GD3/GM2, GnRH, GnTV, GP1, gp100/Pmel17, gp-100-in4, gp15, gp75/TRP-1, hCG, heparanse, Her2/neu, HMTV, Hsp70, hTERT, IGFR1, IL-13R, iNOS, Ki67, KIAA0205, K-ras, H-ras, N-ras, KSA, LKLR-FUT, MAGE-family, mammaglobin, MAP17, melan-A/MART-1, mesothelin, MIC A/B, MT-MMPs, mucin, NY-ESO-1, osteonectin, p15, P170/MDR1, p53, p97/melanotransferrin, PAI-1, PDGF, uPA, PRAME, probasin, progenipoientin, PSA, PSM, RAGE-1, Rb, RCAS1, SART-1, SSX-family, STAT3, STn, TAG-72, TGF-alpha, TGF-beta, Thymosin-beta-15, TNF-alpha, TYRP-, TYRP-2, tyrosinase, VEGF, ZAG, p16INK4, and glutathione-S-transferase.

A preferred PSA antigen comprises the amino acid change of isoleucine to leucine at position 155. U.S. Patent 7,247,615, which is incorporated herein by reference.

One exemplary tumor-associated antigen is HER-2. HER-2 is a member of the epidermal growth factor receptor family (c-erbB) which consists of four different receptors to date: c-erbB-1 (EGFr), c-erbB-2 (HER-2, c-Neu), c-erbB-3 and c-erbB-4 (Salomon et al, 1995). C-erbB-3 and c-erbB-4 are less well characterized than EGFr and HER-2. HER-2 is an integral membrane glycoprotein. The mature protein has a molecular weight of 185 kD with structural features that closely resemble the EGFr receptor (Prigent et al, 1992). EGFr is also an integral membrane receptor consisting of one subunit. It has an apparent molecular weight of 170 kD and consists of a surface ligand-binding domain of 621 amino acids, a single hydrophobic transmembrane domain of 23 amino acids, and a highly conserved cytoplasmic tyrosine kinase domain of 542 amino acids. The protein is N-glycosylated (Prigent et al, 1994).

All proteins in this family are tyrosine kinases. Interaction with the ligand leads to receptor dimerization, which increases the catalytic action of the tyrosine kinase (Bernard. 1995, Chantry 1995). The proteins within the family are able to homo- and heterodimerise, which is important for their activity. The EGFr conveys growth promoting effects and stimulates uptake of glucose and amino acids by cells (Prigent et al 1992). HER-2 also conveys growth promoting signals.

The epidermal growth factor receptor is expressed on normal tissues in low amounts, but it is overexpressed in many types of cancers. EGFr is overexpressed in breast cancers (Earp et al, 1993, Eppenberger 1994), gliomas (Schlegel et al, 1994), gastric cancer (Tkunaga et al, 1995), cutaneous squamous carcinoma (Fujii 1995), ovarian cancer (van Dam et al, 1994) and others. HER-2 is also expressed on few normal human tissues in low amount, most characteristically on secretory epithelia. Over-expression of HER-2 occurs in about 30% of breast, gastric, pancreatic, bladder and ovarian cancers.

The expression of these receptors varies depending on the degree of differentiation of the tumors and the cancer type, e.g., in breast cancer, primary tumors overexpress both receptors; whereas in gastric cancer, the overexpression occurs at a later stage in metastatic tumours (Salomon et al, 1995). The number of overexpressed receptors on carcinoma cells is greater than 10⁶/cell for several head and neck cancers, vulva, breast and ovarian cancer lines isolated from patients (Dean et al, 1994).

There are several reasons why the EGFr family of receptors constitutes suitable targets for tumor immunotherapy. First, they are overexpressed in many types of cancers, which should direct the immune response towards the tumor. Second, the tumors often express or overexpress the ligands for this family of receptors and some are hypersensitive to the proliferative effects mediated by the ligands. Third, patients with tumors that overexpress growth factor receptors often have a poor prognosis. The overexpression has been closely linked with poor prognosis especially in breast cancer, lung cancer, and bladder cancer and can be associated with invasive/metastatic phenotypes, which are rather insensitive to conventional therapies (Eccles et al, 1994).

### Modified Tumor-Associated Antigens

In certain embodiments, a cell-associated polypeptide antigen is modified such that a CTL response is induced against a cell which presents epitopes derived from a polypeptide antigen on its surface, when presented in association with an MHC Class I molecule on the surface of an APC. In certain such embodiments, at least one first foreign TH epitope, when presented, is associated with an MHC Class II molecule on the surface of the APC. In certain such embodiments, a cell-associated antigen is a tumor-associated antigen.

Exemplary APCs capable of presenting epitopes include dendritic cells and macrophages. Additional exemplary APCs include any pino- or phagocytizing APC, which is capable of simultaneously presenting 1) CTL epitopes bound to MHC class I molecules and 2) T_{H} epitopes bound to MHC class II molecules.

In certain embodiments, modifications to one or more of the tumor-associated antigens (TAAs) presented herein, such as, but not limited to, CEA, MUC-1, PSA, HER-2, or Brachyury,are made such that, after administration to a subject, polyclonal antibodies are elicited that predominantly react with the one or more of the TAAs described herein . Such antibodies could attack and eliminate tumor cells as well as prevent metastatic cells from developing into metastases. The effector mechanism of this anti-tumor effect would be mediated via complement and antibody dependent cellular cytotoxicity. In addition, the induced antibodies could also inhibit cancer cell growth through inhibition of growth factor dependent oligo-dimerisation and internalization of the receptors. In certain embodiments, such modified TAAs could induce CTL responses directed against known and/or predicted TAA epitopes displayed by the tumor cells.

In certain embodiments, a modified TAA polypeptide antigen comprises a CTL epitope of the cell-associated polypeptide antigen and a variation, wherein the variation comprises at least one CTL epitope of a foreign T_{H} epitope. Certain such modified TAAs can include in one non-limiting example one or more HER-2 polypeptide antigens comprising at least one CTL epitope and a variation comprising at least one CTL epitope of a foreign T_{H} epitope, and methods of producing the same, are described in U.S. Patent No. 7,005,498 and U.S. Patent Pub. Nos. 2004/0141958 and 2006/0008465.

In certain embodiments, a foreign T_{H} epitope is a naturally-occurring "promiscuous" T-cell epitope. Such promiscuous T-cell epitopes are active in a large proportion of individuals of an animal species or an animal population. In certain embodiments, a vaccine comprises such promiscuous T-cell epitopes. In certain such embodiments, use of promiscuous T-cell epitopes reduces the need for a very large number of different CTL epitopes in the same vaccine. Exemplary promiscuous T-cell epitopes include, but are not limited to, epitopes from tetanus toxin, including but not limited to, the P2 and P30 epitopes (Panina-Bordignon et al., 1989), diphtheria toxin, Influenza virus hemagluttinin (HA), and P. falciparum CS antigen.

Additional promiscuous T-cell epitopes include peptides capable of binding a large proportion of HLA-DR molecules encoded by the different HLA-DR. See, e.g., WO 98/23635 (Frazer IH et al., assigned to The University of Queensland); Southwood S et. al, 1998, J. Immunol. 160: 3363 3373; Sinigaglia F et al., 1988, Nature 336: 778 780; Rammensee HG et al., 1995, Immunogenetics 41: 4 178 228; Chicz RM et al., 1993, J. Exp. Med 178: 27 47; Hammer J et al., 1993, Cell 74: 197 203; and Falk K et al., 1994, Immunogenetics 39: 230 242. The latter reference also deals with HLA-DQ and -DP ligands. All epitopes listed in these references are relevant as candidate natural epitopes as described herein, as are epitopes which share common motifs with these.

In certain other embodiments, the promiscuous T-cell epitope is an artificial T-cell epitope which is capable of binding a large proportion of haplotypes. In certain such embodiments, the artificial T-cell epitope is a pan DR epitope peptide ("PADRE") as described in WO 95/07707 and in the corresponding paper Alexander J et al., 1994, Immunity 1: 751 761.

### mHER2

Various modified HER-2 polypeptide antigens and methods for producing the same are described in U.S. Patent No. 7,005,498 and U.S. Patent Pub. Nos. 2004/0141958 and 2006/0008465. Those documents describe various modified HER-2 polypeptide antigens comprising promiscuous T-cell epitopes at different positions in the HER-2 polypeptide.

The human HER-2 sequence can be divided into a number of domains based solely on the primary structure of the protein. Those domains are as follows. The extracellular (receptor) domain extends from amino acids 1-654 and contains several subdomains as follows: Domain I (N-terminal domain of mature polypeptide) extends from amino acids 1-173; Domain II (Cysteine rich domain, 24 cysteine residues) extends from amino acids 174-323; Domain III (ligand binding domain in the homologous EGF receptor) extends from amino acids 324-483; and Domain IV (Cysteine rich domain, 20 cysteine residues) extends from amino acids 484-623. The transmembrane residues extend from amino acids 654-675. The intracellular (Kinase) domain extends from amino acids 655-1235 and contains the tyrosine kinase domain, which extends from amino acids 655-1010 (core TK domain extends from 725-992); and the C-terminal domain, which extends from amino acids 1011-1235.

Selection of sites in the amino acid sequence of HER-2 to be displaced by either the P2 or P30 human T helper epitopes is described in U.S. Patent No. 7,005,498 and U.S. Patent Pub. Nos. 2004/0141958 and 2006/0008465. To summarize, the following parameters were considered:
1. Known and predicted CTL epitopes;
2. Homology to related receptors (EGFR in particular);
3. Conservation of cysteine residues;
4. Predicted loop, α-helix and β-sheet structures;
5. Potential N-glycosylation sites;
6. Prediction of exposed and buried amino acid residues;
7. Domain organization.

The CTL epitopes appear to be clustered in domain I, domain III, the TM domain and in two or three "hot spots" in the TK domain. As described in U.S. Patent No. 7,005,498 and U.S. Patent Pub. Nos. 2004/0141958 and 2006/0008465, these should be largely conserved.

Regions with a high degree of homology with other receptors are likely to be structurally important for the "overall" tertiary structure of HER-2, and hence for antibody recognition, whereas regions with low homology possibly can be exchanged with only local alterations of the structure as the consequence.

Cysteine residues are often involved in intramolecular disulphide bridge formation and are thus involved in the tertiary structure and should not be changed. Regions predicted to form alpha-helix or beta-sheet structures should be avoided as insertion points of foreign epitopes, as these regions are thought to be involved in folding of the protein.

Potential N-glycosylation sites should be conserved if mannosylation of the protein is desired.

Regions predicted (by their hydrophobic properties) to be interior in the molecule preferably should be conserved as these could be involved in the folding. In contrast, solvent exposed regions could serve as candidate positions for insertion of the model T_{H} epitopes P2 and P30.

Finally, the domain organization of the protein should be taken into consideration because of its relevance for protein structure and function.

As described in U.S. Patent No. 7,005,498 and U.S. Patent Pub. Nos. 2004/0141958 and 2006/0008465, the focus of the strategy has been to conserve the structure of the extracellular part of HER-2 as much as possible, because this is the part of the protein which is relevant as a target for neutralizing antibodies. By contrast, the intracellular part of native membrane bound HER-2 on the surface of cancer cells is inaccessible for the humoral immune system.

Various exemplary constructs using the P2 and P30 epitopes of tetanus toxin inserted in various domains of HER-2 are provided in U.S. Patent No. 7,005,498 and U.S. Patent Pub. Nos. 2004/0141958 and 2006/0008465. One exemplary modified HER-2 polypeptide antigen, referred to as "mHER2," comprises the extracellular domains and nine amino acids of the transmembrane domain; the P2 epitope inserted in Domain II between amino acid residues 273 to 287 of the modified HER-2 polypeptide; and the P30 epitope inserted in Domain IV between amino acid residues 655 to 675 of the modified HER-2 polypeptide.

### Recombinant MVA-BN-mHER2

In an embodiment, recombinant MVA comprising a tumor-associated antigen, e.g., MVA-BN-mHER2, is constructed as follows. The initial virus stock is generated by recombination in cell culture using a cell type permissive for replication, e.g., CEF cells. Cells are both inoculated with an attenuated vaccinia virus, e.g., MVA-BN, and transfected with a recombination plasmid (e.g., pBN146) that encodes the tumor-associated antigen, e.g., mHER2, sequence and flanking regions of the virus genome. In one non-limiting embodiment, the plasmid pBN146 contains sequences which are also present in MVA-BN (the 14L and 15L open reading frames). The mHER2 sequence is inserted between the MVA-BN sequences to allow for recombination into the MVA-BN viral genome. In certain embodiments, the plasmid also contains a selection cassette comprising one or more selection genes to allow for selection of recombinant constructs in CEF cells. In a preferred embodiment, the recombinant MVA encodes a polypeptide comprising SEQ ID NO:2.

Simultaneous infection and transfection of cultures allows homologous recombination to occur between the viral genome and the recombination plasmid. Insert-carrying virus is then isolated, characterized, and virus stocks prepared. In certain embodiments, virus is passaged in CEF cell cultures in the absence of selection to allow for loss of the region encoding the selection genes, gpt and EGFP.

### Antagonists of Immune Checkpoint Molecules

As described herein, at least in one aspect, the invention encompasses the use of immune checkpoint antagonists. Such immune checkpoint antagonists include antagonists of immune checkpoint molecules such as Cytotoxic T-Lymphocyte Antigen 4 (CTLA-4) and Programmed Cell Death Protein 1 (PD-1). An antagonist of CTLA-4 or PD-1 interferes with CTLA-4 or PD-1 function, respectively.

Such antagonists of CTLA-4 or PD-1 include antibodies which specifically bind to CTLA-4 or PD-1, respectively and inhibit and/or block biological activity and function.

Other antagonists of CTLA-4 or PD-1 include antisense nucleic acids RNAs that interfere with the expression of CTLA-4 or PD-1; small interfering RNAs that interfere with the expression of CTLA-4 or PD-1; and small molecule inhibitors of CTLA-4 or PD-1.

Candidate antagonists of CTLA-4 or PD-1 can be screened for function by a variety of techniques known in the art and/or disclosed within the instant application, such as ability to interfere with CTLA-4 or PD-1 function in an in vitro or mouse model.

### Antibodies

In one embodiment, the antagonist of CTLA-4 or PD-1 is an antibody. Antibodies can be synthetic, monoclonal, or polyclonal and can be made by techniques well known in the art. Such antibodies specifically bind to CTLA-4 or PD-1 via the antigen-binding sites of the antibody (as opposed to non-specific binding). CTLA-4 or PD-1 polypeptides, fragments, variants, fusion proteins, etc., can be employed as immunogens in producing antibodies immunoreactive therewith. More specifically, the polypeptides, fragment, variants, fusion proteins, etc. contain antigenic determinants or epitopes that elicit the formation of antibodies.

These antigenic determinants or epitopes can be either linear or conformational (discontinuous). Linear epitopes are composed of a single section of amino acids of the polypeptide, while conformational or discontinuous epitopes are composed of amino acids sections from different regions of the polypeptide chain that are brought into close proximity upon protein folding (C. A. Janeway, Jr. and P. Travers, Immuno Biology 3:9 (Garland Publishing Inc., 2nd ed. 1996)). Because folded proteins have complex surfaces, the number of epitopes available is quite numerous; however, due to the conformation of the protein and steric hinderances, the number of antibodies that actually bind to the epitopes is less than the number of available epitopes (C. A. Janeway, Jr. and P. Travers, Immuno Biology 2:14 (Garland Publishing Inc., 2nd ed. 1996)). Epitopes can be identified by any of the methods known in the art.

Antibodies, including scFV fragments, which bind specifically to CTLA-4 or PD-1 and either block its function ("antagonist antibodies") or enhance/ activate its function ("agonist antibodies"), are encompassed by the disclosure. Such antibodies can be generated by conventional means.

In one embodiment, the invention encompasses monoclonal antibodies against CTLA-4 or PD-1 that block ("antagonist antibodies") function of the CTLA-4 or PD-1 immune checkpoint molecules, respectively. Exemplary blocking monoclonal antibodies against PD-1 are described in WO 2011/041613.

Antibodies are capable of binding to their targets with both high avidity and specificity. They are relatively large molecules (~150kDa), which can sterically inhibit interactions between two proteins (e.g. PD-1 and its target ligand) when the antibody binding site falls within proximity of the protein-protein interaction site. The invention further encompasses antibodies that bind to epitopes within close proximity to a CTLA-4 or PD-1 ligand binding site.

In various embodiments, the invention encompasses antibodies that interfere with intermolecular interactions (e.g. protein-protein interactions), as well as antibodies that perturb intramolecular interactions (e.g. conformational changes within a molecule). Antibodies can be screened for the ability to block or enhance/activate the biological activity of CTLA-4 or PD-1 or the binding of CTLA-4 or PD-1 to a ligand, and/or for other properties.

Both polyclonal and monoclonal antibodies can be prepared by conventional techniques.

CTLA-4 or PD-1 and peptides based on the amino acid sequence of CTLA-4 or PD-1 can be utilized to prepare antibodies that specifically bind to CTLA-4 or PD-1. The term "antibodies" is meant to include polyclonal antibodies, monoclonal antibodies, fragments thereof, such as F(ab')2 and Fab fragments, single-chain variable fragments (scFvs), single-domain antibody fragments (VHHs or Nanobodies), bivalent antibody fragments (diabodies), as well as any recombinantly and synthetically produced binding partners.

Antibodies are defined to be specifically binding if they bind CTLA-4 or PD-1 polypeptide with a Ka of greater than or equal to about 10⁷ M⁻¹. Affinities of binding partners or antibodies can be readily determined using conventional techniques, for example those described by Scatchard et al., Ann. N.Y. Acad. Sci., 51:660 (1949).

Polyclonal antibodies can be readily generated from a variety of sources, for example, horses, cows, goats, sheep, dogs, chickens, rabbits, mice, or rats, using procedures that are well known in the art. In general, purified CTLA-4 or PD-1 or a peptide based on the amino acid sequence of CTLA-4 or PD-1 that is appropriately conjugated is administered to the host animal typically through parenteral injection. The immunogenicity of CTLA-4 or PD-1 can be enhanced through the use of an adjuvant, for example, Freund's complete or incomplete adjuvant. Following booster immunizations, small samples of serum are collected and tested for reactivity to CTLA-4 or PD-1 polypeptide. Examples of various assays useful for such determination include those described in Antibodies: A Laboratory Manual, Harlow and Lane (eds.), Cold Spring Harbor Laboratory Press, 1988; as well as procedures, such as countercurrent immuno-electrophoresis (CIEP), radioimmunoassay, radio-immunoprecipitation, enzyme-linked immunosorbent assays (ELISA), dot blot assays, and sandwich assays. See U.S. Pat. Nos. 4,376,110 and 4,486,530.

Monoclonal antibodies can be readily prepared using well known procedures. See, for example, the procedures described in U.S. Pat. Nos. RE 32,011, 4,902,614, 4,543,439, and 4,411,993; Monoclonal Antibodies, Hybridomas: A New Dimension in Biological Analyses, Plenum Press, Kennett, McKeam, and Bechtol (eds.), 1980.

For example, the host animals, such as mice, can be injected intraperitoneally at least once and preferably at least twice at about 3 week intervals with isolated and purified CTLA-4 or PD-1 or conjugated CTLA-4 or PD-1 peptide, optionally in the presence of adjuvant. Mouse sera are then assayed by conventional dot blot technique or antibody capture (ABC) to determine which animal is best to fuse. Approximately two to three weeks later, the mice are given an intravenous boost of CTLA-4 or PD-1 or conjugated CTLA-4 or PD-1, peptide. Mice are later sacrificed and spleen cells fused with commercially available myeloma cells, such as Ag8.653 (ATCC), following established protocols. Briefly, the myeloma cells are washed several times in media and fused to mouse spleen cells at a ratio of about three spleen cells to one myeloma cell. The fusing agent can be any suitable agent used in the art, for example, polyethylene glycol (PEG). Fusion is plated out into plates containing media that allows for the selective growth of the fused cells. The fused cells can then be allowed to grow for approximately eight days. Supernatants from resultant hybridomas are collected and added to a plate that is first coated with goat anti-mouse Ig. Following washes, a label, such as a labeled CTLA-4 or PD-1 polypeptide, is added to each well followed by incubation. Positive wells can be subsequently detected. Positive clones can be grown in bulk culture and supernatants are subsequently purified over a Protein A column (Pharmacia).

The monoclonal antibodies of the invention can be produced using alternative techniques, such as those described by Alting-Mees et al., "Monoclonal Antibody Expression Libraries: A Rapid Alternative to Hybridomas", Strategies in Molecular Biology 3:1-9 (1990), which is incorporated herein by reference. Similarly, binding partners can be constructed using recombinant DNA techniques to incorporate the variable regions of a gene that encodes a specific binding antibody. Such a technique is described in Larrick et al., Biotechnology, 7:394 (1989).

Antigen-binding fragments of such antibodies, which can be produced by conventional techniques, are also encompassed by the present invention. Examples of such fragments include, but are not limited to, Fab and F(ab')2 fragments. Antibody fragments and derivatives produced by genetic engineering techniques are also provided.

The monoclonal antibodies of the present invention include chimeric antibodies, e.g., humanized versions of murine monoclonal antibodies. Such humanized antibodies can be prepared by known techniques, and offer the advantage of reduced immunogenicity when the antibodies are administered to humans. In one embodiment, a humanized monoclonal antibody comprises the variable region of a murine antibody (or just the antigen binding site thereof) and a constant region derived from a human antibody. Alternatively, a humanized antibody fragment can comprise the antigen binding site of a murine monoclonal antibody and a variable region fragment (lacking the antigen-binding site) derived from a human antibody. Procedures for the production of chimeric and further engineered monoclonal antibodies include those described in Riechmann et al. (Nature 332:323, 1988), Liu et al. (PNAS 84:3439, 1987), Larrick et al. (Bio/Technology 7:934, 1989), and Winter and Harris (TIPS 14:139, May, 1993). Procedures to generate antibodies transgenically can be found in GB 2,272,440, U.S. Pat. Nos. 5,569,825 and 5,545,806.

Antibodies produced by genetic engineering methods, such as chimeric and humanized monoclonal antibodies, comprising both human and non-human portions, which can be made using standard recombinant DNA techniques, can be used. Such chimeric and humanized monoclonal antibodies can be produced by genetic engineering using standard DNA techniques known in the art, for example using methods described in Robinson et al. International Publication No. WO 87/02671; Akira, et al. European Patent Application 0184187; Taniguchi, M., European Patent Application 0171496; Morrison et al. European Patent Application 0173494; Neuberger et al. PCT International Publication No. WO 86/01533; Cabilly et al. U.S. Pat. No. 4,816,567; Cabilly et al. European Patent Application 0125023; Better et al., Science 240:1041 1043, 1988; Liu et al., PNAS 84:3439 3443, 1987; Liu et al., J. Immunol. 139:3521 3526, 1987; Sun et al. PNAS 84:214 218, 1987; Nishimura et al., Canc. Res. 47:999 1005, 1987; Wood et al., Nature 314:446 449, 1985; and Shaw et al., J. Natl. Cancer Inst. 80:1553 1559, 1988); Morrison, S. L., Science 229:1202 1207, 1985; Oi et al., BioTechniques 4:214, 1986; Winter U.S. Pat. No. 5,225,539; Jones et al., Nature 321:552 525, 1986; Verhoeyan et al., Science 239:1534, 1988; and Beidler et al., J. Immunol. 141:4053 4060, 1988.

In connection with synthetic and semi-synthetic antibodies, such terms are intended to cover but are not limited to antibody fragments, isotype switched antibodies, humanized antibodies (e.g., mouse-human, human-mouse), hybrids, antibodies having plural specificities, and fully synthetic antibody-like molecules.

For therapeutic applications, "human" monoclonal antibodies having human constant and variable regions are often preferred so as to minimize the immune response of a patient against the antibody. Such antibodies can be generated by immunizing transgenic animals which contain human immunoglobulin genes. See Jakobovits et al. Ann NY Acad Sci 764:525-535 (1995).

Human monoclonal antibodies against CTLA-4 or PD-1 polypeptides can also be prepared by constructing a combinatorial immunoglobulin library, such as a Fab phage display library or a scFv phage display library, using immunoglobulin light chain and heavy chain cDNAs prepared from mRNA derived from lymphocytes of a subject. See, e.g., McCafferty et al. PCT publication WO 92/01047; Marks et al. (1991) J. Mol. Biol. 222:581 597; and Griffths et al. (1993) EMBO J 12:725 734. In addition, a combinatorial library of antibody variable regions can be generated by mutating a known human antibody. For example, a variable region of a human antibody known to bind CTLA-4 or PD-1 can be mutated, by for example using randomly altered mutagenized oligonucleotides, to generate a library of mutated variable regions which can then be screened to bind to CTLA-4 or PD-1. Methods of inducing random mutagenesis within the CDR regions of immunoglobin heavy and/or light chains, methods of crossing randomized heavy and light chains to form pairings and screening methods can be found in, for example, Barbas et al. PCT publication WO 96/07754; Barbas et al. (1992) Proc. Nat'1 Acad. Sci. USA 89:4457 4461.

An immunoglobulin library can be expressed by a population of display packages, preferably derived from filamentous phage, to form an antibody display library. Examples of methods and reagents particularly amenable for use in generating antibody display library can be found in, for example, Ladner et al. U.S. Pat. No. 5,223,409; Kang et al. PCT publication WO 92/18619; Dower et al. PCT publication WO 91/17271; Winter et al. PCT publication WO 92/20791; Markland et al. PCT publication WO 92/15679; Breitling et al. PCT publication WO 93/01288; McCafferty et al. PCT publication WO 92/01047; Garrard et al. PCT publication WO 92/09690; Ladner et al. PCT publication WO 90/02809; Fuchs et al. (1991) Bio/Technology 9:1370 1372; Hay et al. (1992) Hum Antibod Hybridomas 3:81 85; Huse et al. (1989) Science 246:1275 1281; Griffths et al. (1993) supra; Hawkins et al. (1992) J Mol Biol 226:889 896; Clackson et al. (1991) Nature 352:624 628; Gram et al. (1992) PNAS 89:3576 3580; Garrad et al. (1991) Bio/Technology 9:1373 1377; Hoogenboom et al. (1991) Nuc Acid Res 19:4133 4137; and Barbas et al. (1991) PNAS 88:7978 7982. Once displayed on the surface of a display package (e.g., filamentous phage), the antibody library is screened to identify and isolate packages that express an antibody that binds a CTLA-4 or PD-1 polypeptide. In a preferred embodiment, the primary screening of the library involves panning with an immobilized CTLA-4 or PD-1 polypeptide and display packages expressing antibodies that bind immobilized CTLA-4 or PD-1 polypeptide are selected.

In addition to the CTLA-4 or PD-1 antagonists and agonists already described herein, it is contemplated that the antagonists and agonists can include those known in the art. For example, Ipilimumab^{®} and tremelimumab, are known CTLA-4 antibodies. Additionally, lambrolizumab, Amplimmune-224 (AMP-224), Amplimmune -514 (AMP-514), Nivolumab, MK-3475, and B7H1 are known PD-1 antibodies.

It is also envisioned by the present disclosure that immune checkpoint antagonists or agonists can be embodied in small molecules, peptides, soluble receptor proteins, and other types of fusion proteins.

### Combination Therapy with a Poxvirus Expressing a Tumor Antigen and an Immune Checkpoint Antagonist

In one embodiment, patients with a cancer mediated by cells over-expressing the tumor-associated antigen HER-2 (e.g., breast cancer) can be treated by the combination of: an orthopoxvirus (e.g., vaccinia virus, Wyeth, ACAM1000, ACAM2000, MVA, or MVA-BN) or an avipoxvirus (e.g.,fowlpoxvirus, POXVAC-TC), encoding the tumor-associated antigens a HER-2 antigen with one or more immune checkpoint antibodies, or antagonists according to the invention. In a preferred embodiment, the MVA is MVA-BN. In a particularly preferred embodiment, the MVA encodes a polypeptide comprising SEQ ID NO:2.

In one embodiment, patients with a prostate cancer can be treated by the combination of an orthopoxvirus, for example a vaccinia virus (such as but not limited to, vaccinia virus, Wyeth, ACAM1000, ACAM2000, MVA, or MVA-BN) or a avipoxvirus (e.g., fowlpoxvirus, e.g. POXVAC-TC), encoding a PSA antigen, with one or more antibodies, or antagonists according to the invention. In a particularly preferred embodiment, the Vaccinia virus is part of PROSTVAC^{®}.

In one embodiment, patients with a cancer mediated by cells over-expressing the tumor-associated antigen CEA and/or MUC-1 (e.g., breast, colorectal, lung, and ovarian cancer) can be treated by the combination of an orthopoxvirus, for example a vaccinia virus (e.g., vaccinia virus, Wyeth, ACAM1000, ACAM2000, MVA, or MVA-BN Wyeth, or MVA) or an avipoxvirus (e.g., fowlpoxvirus, (e.g. POXVAC-TC), encoding a CEA and/or MUC-1 antigen, with one or more antibodies, or antagonists according to the invention..

The recombinant poxvirus can be administered either systemically or locally, i.e., by parenteral, subcutaneous, intravenous, intramuscular, intranasal, intradermal, scarification, or any other path of administration known to a skilled practitioner. Preferably, the administration is via scarification. More preferably, the administration is via subcutaneous injection. In one embodiment, 10⁵⁻10¹⁰ TCID₅₀ of the recombinant poxvirus are administered to the patient. Preferably, 10⁷⁻10¹⁰ TCID₅₀ of the recombinant poxvirus are administered to the patient. More preferably, 10⁸⁻10¹⁰ TCID₅₀ of the recombinant poxvirus are administered to the patient. Most preferably, 10⁸⁻10⁹ TCID₅₀ of the recombinant poxvirus are administered to the patient.

The cancer preferably includes, but is not limited to, a prostate cancer, breast cancer, a lung cancer, a gastric cancer, a pancreatic cancer, a bladder cancer, or an ovarian cancer. In a preferred embodiment, the cancer is a metastatic breast cancer.

The cancer patient can be any mammal, including a mouse or rat. Preferably, the cancer patient is a primate, most preferably, a human.

In one or more embodiments, the recombinant poxvirus is for administration on the same day or within 1, 2, 3, 4, 5, 6, or 7, days of immune checkpoint agonist and/or antagonist administration. The recombinant poxvirus can be administered before or after the immune checkpoint agonist and/or antagonist.

In one embodiment, one or more immune checkpoint antibodies, agonist or antagonist, according to the invention and the poxvirus encoding a polypeptide comprising a tumor antigen are administered at the same time. The combination treatment is superior to either treatment alone.

In one or more preferred embodiments, the recombinant poxvirus is for administration prior to the administration of the immune checkpoint antagonists and agonists.

### Combination Therapy Using Homologous/Heterologous Prime-Boost Regimens

It is possible to induce an immune response with a single administration of the recombinant poxvirus as defined above. The poxvirus according to the present invention may also be used as part of a homologous prime-boost regimen. In the homologous prime-boost, a first priming vaccination is given followed by one or more subsequent boosting vaccinations. The boosting vaccinations are configured to boost the immune response generated in the first vaccination by administration of the same recombinant poxvirus that was used in the first vaccination.

The recombinant poxvirus according to the present invention may also be used in heterologous prime-boost regimens in which one or more of the initial prime vaccinations are done with a poxvirus as defined herein and in which one or more subsequent boosting vaccinations is done with a different vaccine, e.g., another virus vaccine, a protein or a nucleic acid vaccine.

In one embodiment, the one or more subsequent boosting vaccinations of a heterologous prime-boost regimen are selected from poxviruses of a different genus than the initial prime vaccinations. In one non-limiting example, when the first or initial pox virus vaccine includes vaccinia, the second and subsequent poxvirus vaccines are selected from the poxviruses from a different genus such as suipox, avipox, capripox or an orthopox immunogenically different from vaccinia.

In one exemplary embodiment a homologous prime-boost regimen may be employed wherein a poxvirus such as an MVA-BN expressing one or more Tumor Associated Antigens (TAAs), such as but not limited to HER2, is administered in a first dosage in combination with one or more immune checkpoint antagonists or agonists. One or more subsequent administrations of MVA-BN expressing one or more TAAs, such as but not limited to HER2, in combination with one or more immune checkpoint antagonists or agonists can be given to boost the immune response provided in the first administration. Preferably, the one or more TAAs in the second and subsequent MVA-BNs are the same or similar TAAs to those of the first administration.

In another exemplary embodiment, a heterologous prime-boost may be employed wherein a poxvirus, such as vaccinia, expressing one or more TAAs is administered in a first dose in combination with one or more immune checkpoint antagonists. This first dose is followed by one or more administrations of different poxvirus, such as fowlpox, expressing one or more TAAs. Preferably, the one or more TAAs in the fowlpox virus are the same or similar TAAs to those included in the vaccinia virus of the first administration. Further description of additional exemplary heterologous prime-boost regimens can be found in US Patents 6,165,460; 7,598,225; and 7,247,615.

In one preferred embodiment, the one or more TAAs in the heterologous prime-boost regimen include prostate specific antigen (PSA). In a more preferred embodiment, the PSA antigen can include those PSA antigens found in US Patents 7,247,615 and 7,598,225. In one non-limiting example, the heterologous prime-boost including PSA is PROSTVAC^{®}.

In yet another preferred embodiment, the one or more TAAs in the heterologous prime-boost regimen include A mucin 1, cell surface associated (MUC1) antigen and a carcinoembryonic antigen (CEA). In a more preferred embodiment, the MUC1 and the CEA antigens can include those found in US Patents 7,118,738; 7,723,096; and PCT application No. PCT/US2013/020058. In one non-limiting example, the heterologous prime-boost regimen including a MUC-1 antigen and CEA is CV301.

In yet another exemplary embodiment, a heterologous prime-boost may be employed wherein a poxvirus, such as MVA or MVA-BN, expressing one or more TAAs is administered in a first dose in combination with one or more immune checkpoint antagonists. This first dose is followed by one or more administrations of different poxvirus, such as fowlpox, expressing one or more TAAs. Preferably, the one or more TAAs in the fowlpox virus are the same or similar TAAs to those included in the MVA or MVA-BN virus of the first administration.

It is contemplated by the present disclosure that the homologous and heterologous prime-boost regimens described herein can incorporate one or more of the dosage administration embodiments of the present invention. By way of example, in one or of both the homologous and heterologous prime-boost regimens a sub-therapeutically effective amount or dosage of an immune checkpoint antagonist can be administered, as described herein.

Additionally, by way of example, in one or of both the homologous and heterologous prime-boost regimens an immune checkpoint antagonist can be administered after a recombinant poxvirus encoding a TAA, as described herein.

Also by way of example, in one or of both the homologous and heterologous prime-boost regimens a second dosage or administration of an immune checkpoint antagonist can be increased as compared to a first dosage or administration.

In certain embodiments, the one or more boosting vaccinations are administered at intervals comprising days, weeks or months after administration of the initial priming vaccination. In certain embodiments, the one or more boosting vaccinations are administered at intervals of 1, 2, 3, 4, 5, 6, 7 or more days after administration of the initial priming vaccination. In certain embodiments, the one or more boosting vaccinations are administered at intervals of 1, 2, 3, 4, 5, 6, 7, 8 or more weeks after administration of the initial priming vaccination. In certain embodiments, the one or more boosting vaccinations are administered at intervals of 1, 2, 3, 4, 5, 6, 7, 8, 9, 10, 11, 12 or more months after administration of the initial priming vaccination. In certain embodiments, the one or more boosting vaccinations are administered at any combination of intervals after administration of the initial the priming vaccination)(e.g., 1, 2, 3, 4, 5, 6, 7 or more days, 1, 2, 3, 4, 5, 6, 7, 8 or more weeks, or 1, 2, 3, 4, 5, 6, 7, 8, 9, 10, 11, 12 or more months).

### Dosage Administrations of Immune Checkpoint Antagonists in Combination with a Recombinant Poxviral Treatment

As described and illustrated herein, treatment of a cancer using a recombinant poxviral vector in combination with one or more immune checkpoint antagonists can be effective at a variety of dosages. Significantly, and in contrast to what was understood in the art, the present disclosure demonstrates that in combination with a recombinant poxviral vector encoding a tumor associated antigen, immune checkpoint antagonists can be effective at treating cancer even when the immune checkpoint antagonists are administered at lower dosages. These lower dosage treatments can reduce and/or eliminate many of the adverse side effects that have been seen with current cancer treatments using immune checkpoint antagonists.

In various aspects of the present disclosure, the dosage of antagonist administered to a patient as described herein can be about 0.1 mg/kg to about 100 mg/kg of the patient's body weight. Preferably, the dosage administered to a patient is between about 0.1 mg/kg and about 20 mg/kg of the patient's body weight, more preferably about 1 mg/kg to about 10 mg/kg of the patient's body weight, more preferably about 3 mg/kg to about 10 mg/kg of the patient's body weight, and most preferably about 1 mg/kg to about 3 mg/kg of the patient's body weight. In view of the adverse effects of the treatment of humans with higher dosages of immune checkpoint antagonists to humans, a most preferred dosage of antagonist is about 1mg/kg to about 3mg/kg of a patient's body weight.

In view of the efficacy of the immune checkpoint antagonists at lower dosages when combined with a recombinant poxvirus therapy, in alternative embodiments, the dosage of the antagonist is about 0.1 mg/kg, to about 3 mg/kg of the patient's body weight, about 0.1 mg/kg to about 2 mg/kg of the patient's body weight, or about 0.1 mg/kg to about 1mg/kg of the patient's body weight. In additional embodiments, the dosage of the antagonist is about 0.1 mg/kg, about 0.5mg/kg, about 1mg/kg, about 1.5 mg/kg, about 2 mg/kg, about 2.5 mg/kg, or about 3mg/kg of the patient's body weight.

In additional aspects of the present disclosure, the dosage of antagonist administered to a patient as described herein can be 0.1 mg/kg to 100 mg/kg of the patient's body weight. Preferably, the dosage administered to a patient is between 0.1 mg/kg and 20 mg/kg of the patient's body weight, more preferably 1 mg/kg to 10 mg/kg of the patient's body weight, more preferably 3 mg/kg to 10 mg/kg of the patient's body weight, and most preferably 1 mg/kg to 3 mg/kg of the patient's body weight. In view of the adverse effects of the treatment of humans with higher dosages of immune checkpoint antagonist to humans, a most preferred dosage of antagonist is 1mg/kg to 3mg/kg of a patient's body weight.

In view of the efficacy of the immune checkpoint antagonists at lower dosages when combined with a recombinant poxvirus therapy, in alternative embodiments, the dosage of the antagonist is 0.1 mg/kg to 3 mg/kg of the patient's body weight, 0.1 mg/kg to 2 mg/kg of the patient's body weight, or 0.1 mg/kg to about 1mg/kg of the patient's body weight. In additional embodiments, the dosage of the antagonist is 0.1 mg/kg,0.5mg/kg, 1mg/kg, 1.5 mg/kg, 2 mg/kg, 2.5 mg/kg, or 3mg/kg of the patient's body weight.

The quantities of active ingredient necessary for effective therapy will depend on many different factors, including means of administration, target site, physiological state of the patient, and other medicaments administered. Thus, treatment dosages should be titrated to optimize safety, tolerability and efficacy. Typically, dosages used in vitro can provide useful guidance in the amounts useful for in situ administration of the active ingredients. Animal testing of effective doses for treatment of particular disorders will provide further predictive indication of human dosage. Various considerations are described, for example, in Goodman and Gilman's the Pharmacological Basis of Therapeutics, 7th Edition (1985), MacMillan Publishing Company, New York, and Remington's Pharmaceutical Sciences 18th Edition, (1990) Mack Publishing Co, Easton Pa. Methods for administration are discussed therein, including oral, intravenous, intraperitoneal, intramuscular, transdermal, nasal, iontophoretic administration, and the like.

The compositions of the invention can be administered in a variety of unit dosage forms depending on the method of administration. For example, unit dosage forms suitable for oral administration include solid dosage forms such as powder, tablets, pills, capsules, and dragees, and liquid dosage forms, such as elixirs, syrups, and suspensions. The active ingredients can also be administered parenterally in sterile liquid dosage forms. Gelatin capsules contain the active ingredient and as inactive ingredients powdered carriers, such as glucose, lactose, sucrose, mannitol, starch, cellulose or cellulose derivatives, magnesium stearate, stearic acid, sodium saccharin, talcum, magnesium carbonate and the like. Examples of additional inactive ingredients that can be added to provide desirable color, taste, stability, buffering capacity, dispersion or other known desirable features are red iron oxide, silica gel, sodium lauryl sulfate, titanium dioxide, edible white ink and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain coloring and flavoring to increase patient acceptance.

The concentration of the compositions of the invention in the pharmaceutical formulations can vary widely, i.e., from less than about 0.1%, usually at or at least about 2% to as much as 20% to 50% or more by weight, and will be selected primarily by fluid volumes, viscosities, etc., in accordance with the particular mode of administration selected.

For solid compositions, conventional nontoxic solid carriers can be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more compositions of the invention of the invention, and more preferably at a concentration of 25%-75%.

For aerosol administration, the compositions of the invention are preferably supplied in finely divided form along with a surfactant and propellant. Preferred percentages of compositions of the invention are 0.01%-20% by weight, preferably 1-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides can be employed. The surfactant can constitute 0.1%-20% by weight of the composition, preferably 0.25-5%. The balance of the composition is ordinarily propellant. A carrier can also be included, as desired, as with, e.g., lecithin for intranasal delivery.

The constructs of the invention can additionally be delivered in a depot-type system, an encapsulated form, or an implant by techniques well-known in the art. Similarly, the constructs can be delivered via a pump to a tissue of interest.

Any of the foregoing formulations can be appropriate in treatments and therapies in accordance with the present invention, provided that the active agent in the formulation is not inactivated by the formulation and the formulation is physiologically compatible.

In certain embodiments, the recombinant poxviruses of the present invention can be embodied in one or more pharmaceutical compositions. In addition to a recombinant poxvirus encoding a TAA and one or more immune checkpoint antagonists , pharmaceutical compositions may comprise one or more pharmaceutically acceptable and/or approved carriers, additives, antibiotics, preservatives, adjuvants, diluents and/or stabilizers. Such additives include, for example, but not limited to, water, saline, glycerol, ethanol, wetting or emulsifying agents, and pH buffering substances. Exemplary carriers are typically large, slowly metabolized molecules such as proteins, polysaccharides, polylactic acids, polyglycolic acids, polymeric amino acids, amino acid copolymers, lipid aggregates, or the like.

### Dosage Regimens of Immune Checkpoint Antagonists

In additional embodiments of the present invention, immune checkpoint antagonist dosage concentrations and dosing regimens are configured based upon one or more cancer related factors such as tumor size, tumor volume, cancer stage of a cancer patient or group of cancer patients (such as pre or post metastatic cancer). Additionally, one or more of the immune checkpoint antagonist dosage concentrations and dosing regimens can be configured by monitoring and reviewing efficacy by use of an associated biomarker or antibody such as but not limited to a Forssman or sCD27 antibody. In accordance with this aspect, more effective dosing regimens can be configured for human cancer patients or groups of human cancer patients. At least in one aspect, providing more effective dosing regimens, as described herein, can reduce instances where a patient or group of patients is administered too much or too little of an immune checkpoint antagonist in treatments.

As defined herein, "dosing regimens" can be defined as the schedule of doses of a therapeutic agent per unit of time, including: the time between doses or the time when the doses are to be given, and the amount of medicine or therapeutic agent to be given at each specific time.

At least in one aspect, the methods disclosed herein, for example particular the dosing regimens, are configured to maximize the therapeutic benefit of the combinations of the present disclosure while minimizing the adverse side effects. In one embodiment, an immune checkpoint antagonist is administered to the patient on the same day as an administration of the recombinant poxvirus encoding one or more TAAs. In additional embodiments, as described in the paragraphs that follow, an immune checkpoint antagonist is administered to the patient after an administration of a recombinant poxvirus encoding one or more TAAs.

### Administering an Immune Checkpoint Antagonist after a Recombinant Poxvirus Therapy Increases Efficacy of Cancer Treatments

Shown in Figures 14-17 and described in Examples 32-35, subjects were administered a recombinant poxvirus including a TAA after which expression levels of immune checkpoint molecules such as TIM -3, LAG-3, ICOS, and PD-1 were measured at regular intervals. In response to the treatment, there was an initial period of very little increase in immune checkpoint expression. However, after approximately 1-2 days, T-cells increased expression of immune checkpoint molecules. From about day 3 to about day 12 immune checkpoint expression increased dramatically. Increased expression was still seen until about day 18. Even more significant, this increase in immune checkpoint expression was more profound in CD8 T-cells as compared to CD4 T-cells. (See Figures 14-17).

In view of the results of Figures 14-17 and Examples 32-35, the most effective time period for administration of an immune checkpoint antagonist or agonist occurs after treatment with a recombinant poxvirus. For example, shown in Figure 14, after treatment with MVA-BN-HER2 expression levels of the immune checkpoint molecule TIM-3 increased from about days 1-3 to about 18, with the most significant increases occurring from about day 3 to about day 14. In accordance with this, a more effective time period in which to administer a TIM-3 antagonist is after treatment with a recombinant poxvirus during those periods where increased expression occurs.

Shown in Figures 15-17, after treatment with MVA-BN-HER-2, similar increased expression results were seen with the immune checkpoint molecules ICOS, PD-1, and LAG-3. In accordance with these expression results, to achieve more therapeutic efficacy, each immune checkpoint antagonist (e.g., TIM-3, PD-1, PDL-1, and LAG-3) or agonist (e.g., ICOS) is administered to a cancer patient after being treated with a recombinant poxvirus.

At least in one aspect, greater therapeutic efficacy occurs during these periods of increased immune checkpoint molecule expression at least in part because the increased expression provides more substrates to which the administered immune checkpoints antagonist or agonist can bind, thereby providing either greater blocking or activation of the immune checkpoint molecules.

In another aspect, the results shown in Figure 14-17 indicate that administering an immune checkpoint antagonist or agonist after a recombinant poxvirus treatment is important for enhancing efficacy. This importance is clearly demonstrated by the periods of increased immune checkpoint molecule expression after treatment with a recombinant poxvirus.

In view of the teachings of the present disclosure, in one embodiment, there is a method for treating cancer comprising administering to the patient a dosage of a therapeutic cancer vaccine such as, but not limited to, a recombinant poxvirus, the recombinant poxvirus comprising at least one tumor-associated antigen (TAA); and (b) administering to the patient a dosage of at least one immune checkpoint antagonist; wherein the dosage of the at least one immune checkpoint antagonist is administered after the dosage of the therapeutic cancer vaccine.

It is contemplated that in one embodiment, the dosage of the at least one immune checkpoint antagonist is administered after the dosage of the therapeutic cancer vaccine on the same day. It is additionally contemplated that the dosage of the at least one immune checkpoint antagonist is administered from about 1 to about 18 days, from about 2 to about 17 days, from about 3 to about 16 days, from about 3 to about 14 days, from about 3 to about 12 days, from about 3 to about 10 days, from about 3 to about 8 days, from about 3 to about 7 days, from about 4 to about 15 days, from about 4 to about 14 days, from about 4 to about 13 days, or from about 4 to about 12 days after administering the dosage of the therapeutic cancer vaccine. In a more preferred embodiment, the dosage of the at least one immune checkpoint antagonist is administered from about 3 to about 15 days after administering the dosage of the therapeutic cancer vaccine. In a more preferred embodiment, the dosage of the at least one immune checkpoint antagonist is administered from about 3 to about 7 days after administering the dosage of the therapeutic cancer vaccine. In still a more preferred embodiment, the dosage of the at least one immune checkpoint antagonist is administered 3 days or 7 days after administering the dosage of the therapeutic cancer vaccine.

It is additionally contemplated that the dosage of the at least one immune checkpoint antagonist is administered on the same day or from 1 to 18 days, from 2 to 17 days, from 3 to 16 days, from 3 to 15 days, from 3 to 14 days, from 3 to 12 days, from 4 to 15 days, from 4 to 14 days, from 4 to 13 days, or from 4 to 12 days after administering the dosage of the therapeutic cancer vaccine. In a more preferred embodiment, the dosage of the at least one immune checkpoint antagonist is administered from 3 to 15 days after administering the dosage of the therapeutic cancer vaccine. In a more preferred embodiment, the dosage of the at least one immune checkpoint antagonist is administered from 3 to 7 days after administering the dosage of the therapeutic cancer vaccine. In still a more preferred embodiment, the dosage of the at least one immune checkpoint antagonist is administered 3 days or 7 days after administering the dosage of the therapeutic cancer vaccine.

In an additional embodiments, the present disclosure includes administering to the patient a subsequent (relative to the dosage in the previous paragraph) or a second dosage of a therapeutic cancer vaccine, such as but not limited to, a recombinant poxvirus comprising at least one tumor-associated antigen (TAA); and (b) administering to the patient a subsequent (relative to the dosage in the previous paragraph) or a second dosage of at least one immune checkpoint antagonist; wherein the subsequent dosage of the at least one immune checkpoint antagonist is administered after the subsequent dosage of the therapeutic cancer vaccine. It is contemplated that the subsequent immune checkpoint antagonist dosage can be administered at similar intervals after the subsequent dosage of the therapeutic cancer vaccine (e.g., from about 1 to about 18 days , from about 2 to about 17 days, from about 3 to about 16 days, from about 3 to about 15 days, from about 3 to about 14 days, from about 3 to about 12 days, from about 4 to about 15 days, from about 4 to about 14 days, from about 4 to about 13 days, or from about 4 to about 12 days after administering the dosage of the therapeutic cancer vaccine).

The enhanced treatment efficacy resulting from administering an immune checkpoint antagonist after administering a therapeutic cancer vaccine, such as a recombinant poxviral therapy, is further demonstrated by Examples 26 through 31.

In one embodiment, the therapeutic cancer vaccine comprises a recombinant poxvirus that includes at least one TAA. In another preferred embodiment, the recombinant poxvirus comprises an orthopoxvirus or avipoxvirus that includes at least one TAA. In another preferred embodiment, the orthopoxvirus comprises vaccinia, MVA, or MVA-BN. In another embodiment, the avipoxvirus comprise a fowlpox virus.

In another embodiment, it is contemplated that a CTLA-4 antagonist is administered on the same day of, or about 1-3 days after administering the dosage of the therapeutic cancer vaccine.

In yet another embodiment, it is contemplated that a PD-1 antagonist is administered on from about 2 to 18 days after administering the dosage of the therapeutic cancer vaccine.

### Increasing the Therapeutically Effective Amount of a Second Administration of an Immune Checkpoint Antagonist Increases Efficacy of Cancer Treatments

Shown in Figures 14-17 and described in Examples 32-35, subjects were administered a recombinant poxvirus including a TAA on day 1 and day 15. Expression levels of immune checkpoint molecules such as TIM -3, LAG-3, ICOS, and PD-1 were measured at regular intervals. In response to the day 1 treatment, there was an initial period of very little increase in immune checkpoint expression. However, approximately 1-2 days, after day 1 treatment, there was an increase in T-cell expression of immune checkpoint molecules. From about day 3 to about day 12 immune checkpoint expression increased dramatically. Increased expression was still seen until about day 18.

Most notably, after the second or subsequent treatment of recombinant poxvirus at day 15 an even greater increase in expression of immune checkpoint molecules by T-cells occurred. Even more significant, this increase in immune checkpoint expression was more profound in CD8 T-cells as compared to CD4 T-cells. (See Figures 14-17). For example, shown in Figure 12, after treatment with MVA-BN-HER-2 at day 15, there was a slight increase in expression, after which on day 18 expression levels of TIM-3 increased significantly. In accordance with these TIM-3 expression results, increasing the second or subsequent dosage of a TIM-3 antagonist as compared to the first dosage achieves an even greater treatment efficacy.

Shown in Figures 14-17, after the second recombinant poxvirus treatment with MVA-BN-HER-2 at day 15, similar increased expression of immune checkpoint molecules ICOS, PD-1, and LAG-3 occurred. Further, the increased expression was significantly higher as compared to after the first MVA-BN-HER2 treatment. In accordance with these expression results, an increased dosage of immune checkpoint antagonist (e.g., TIM-3, PD-1, PDL-1, LAG-3) or agonist (e.g., ICOS) is administered to a cancer patient during a time point after a second or subsequent recombinant poxvirus.

At least in one aspect, greater therapeutic efficacy is achieved by increasing the dosage or amount of the immune checkpoint antagonist in a second or subsequent treatment as compared to the first dosage of immune checkpoint antagonist . This is realized at least in part because increased expression after the second treatment provides more substrates to which the administered immune checkpoint antagonist or agonist can bind, thereby providing either greater blocking or activation of the immune checkpoint molecules.

In another aspect, the results shown in Figure 14-17 indicate that increasing a second or subsequent dosage of an immune checkpoint antagonist or agonist as compared to a first dosage is important to achieving increased efficacy of immune checkpoint antagonist treatment when administered in combination with a recombinant poxviral therapy. This importance is supported by the periods of increased immune checkpoint molecule expression after a second treatment with a recombinant poxvirus described and illustrated herein.

In a further aspect, increasing the dosage of a second or subsequent administration of an immune checkpoint antagonist or agonist as described herein greatly enhances treatment efficacy in view of recent data describing immune checkpoint antagonist T cell receptor occupancy (RO). After a first dosage of an immune checkpoint antagonist, an anti-PDl antibody, receptor occupancy (RO) was measured on surface of T cells by FACS (CD45+, CD3 gate) (data not shown). The data indicates that at various dosages of the immune checkpoint antagonist PD-1 (e.g., .1mg/kg, .4 mg/kg, 1.4 mg/kg, and 5 mg/kg) there was a high percentage of PD-1 RO on T cells in the blood stream (RO of .1mg/kg = 65%, RO of.4 mg/kg = 84%, RO of 1.4 mg/kg = 96%, and RO of 5 mg/kg = 89%). When compared with the RO percentages in the blood, the RO of T cells found in tumors was lower (RO of .1mg/kg = 9%, RO of.4 mg/kg = 41%, RO of 1.4 mg/kg = 58%, and RO of 5 mg/kg = 65%).

During an initial dosage of an immune checkpoint antagonist or agonist in combination with a recombinant poxvirus, it is believed that a larger percentage of tumor specific T-cells (CD8 T cells) are typically present in the peripheral areas such as the blood and lymph nodes. In view of increased RO in immune checkpoint antagonist on T cells in the blood, a first administration of an immune checkpoint antagonist or agonist is more effective at lower dosages. For increased efficacy in a second or subsequent dosage of an immune checkpoint antagonist or agonist in combination with a recombinant poxvirus, an increased dosage as compared to the first dosage is administered in view of lower RO on T cells in tumors.

In view of the teachings of the present disclosure in additional embodiments, the present disclosure provides a method for treating a human cancer patient using a therapeutic cancer vaccine in combination with an immune checkpoint antagonist, the method comprising (a) providing the patient a first administration of a therapeutic cancer vaccine, such as a recombinant poxvirus, in combination with a dosage of at least one immune checkpoint antagonist; and (b) providing a second administration of a therapeutic cancer vaccine in combination with a dosage of at least one immune checkpoint antagonist , wherein the dosage of the at least one immune checkpoint antagonist or agonist of the second administration is increased as compared to the dosage of the at least one immune checkpoint antagonist or agonist of the first administration.

In various embodiments, the dosage (or amount) of the second administration of the immune checkpoint antagonist or agonist is increased by a factor of: from about 2 to about 100, from about 3 to about 100, from about 5 to about 100, from about 10 to about 100, from about 5 to about 90, from about 10 to about 80, from about 10 to about 70, from about 10 to about 60, from about 10 to about 50 as compared to the dosage of the first administration. In preferred embodiments, the dosage of the second administration is increased by a factor of about 10 to about 100 as compared to the dosage of the first administration. In another preferred embodiment, the dosage of second administration is increased by a factor of about 10 to about 50 as compared to the dosage of the first administration. In still another more preferred embodiment, the dosage of the second administration is increase by a factor of: about 3, about 10, about 30, or about 100.

In various additional embodiments, the dosage of the at least one immune checkpoint antagonist of the second administration is increased by a factor of: from 2 to 100, from 3 to 100, from 5 to 100, from 10 to 100, from 5 to 90, from 10 to 80, from 10 to 70, from 10 to 60, from 10 to 50 as compared to the dosage of the first administration. In preferred embodiments, the dosage of the second administration is increased by a factor of 10 to 100 as compared to the dosage of the first administration. In another more preferred embodiment, the dosage of second administration is increased by a factor of 10 to 50 as compared to the dosage of the first administration. In still another more preferred embodiment, the dosage of the second administration is increase by a factor of 3, 10, 30, or 100.

It is contemplated that in some exemplary embodiments, a first administration of an immune checkpoint antagonist with a therapeutic cancer vaccine, such as but not limited to a recombinant poxvirus, includes a sub-therapeutic or sub-therapeutically effective dosage after which a second or subsequent administration of an immune checkpoint antagonist with a therapeutic cancer vaccine, such as but not limited to a recombinant poxvirus, includes an increased dosage as set forth in the dosing regimens described herein. In one non-limiting example, a first administration of an immune checkpoint antagonist includes a dosage of about 0.1 mg/kg to about 1mg/kg. This first administration is followed with one or more subsequent administrations with an increased dosage of about 3mg/kg to about 10mg/kg.

In one preferred embodiment, the therapeutic cancer vaccine comprises a recombinant poxvirus that includes at least one TAA. In another preferred embodiment, the recombinant poxvirus comprises an orthopoxvirus or avipoxvirus that includes at least one TAA. In another preferred embodiment, the orthopoxvirus comprises vaccinia, MVA, or MVA-BN. In another embodiment, the avipoxvirus comprise a fowlpox virus. The enhanced treatment efficacy resulting from administering an increased second or subsequent dosage of an immune checkpoint antagonist or agonist as compared to a first dosage is further demonstrated by Examples 26 through 31.

A therapeutic cancer vaccine is a vaccine that displays one or more unique immunological properties that stimulates a patient's or group of patient's own immune system to specifically target a tumor and/or tumor cells. These unique immunological properties are typically antigens that are associated with a tumor or tumor cell. In at least one aspect, therapeutic cancer vaccines function to delay or stop cancer cell growth; to cause tumor shrinkage; to prevent cancer from coming back; or to eliminate cancer cells that have not been killed by other forms of treatment.

In one preferred embodiment, the therapeutic cancer vaccine is virus based. Some non-limiting example of viruses useful as therapeutic cancer vaccines include: Poxvirus, Adenovirus, Alphavirus, Measles virus, Herpes Simplex virus (HSV), Parvovirus, Reo Virus, and Vesicular Stomatis virus (VSV). In a more preferred embodiment, a therapeutic cancer vaccine is a Poxvirus based therapeutic.

Shown in Table 1 are some non-limiting examples of viral based therapeutic cancer vaccines currently in development.

**Table 1**

| Virus | Therapeutic Cancer Vaccine (Company developing vaccine) |
|---|---|
| Poxvirus | MVA-BN, Vaccinia, PROSTVAC, CV-301,(Bavarian |
| | Nordic, Inc.) |
| Adenovirus | AdhTAP (Tap Immune), ColoAd-1(PsiOxus) |
| Alpha virus | AVX-701 (AlphaVax Inc.) |
| Lentivirus | ID-LV(Immune Design Corp.) |
| Herpes Simplex Virus | OncoVEX (Amgen) |
| Parvovirus | H-IPV (OryxGmbH & Co. KG) |
| Measles Virus | MV-CEA and MV-NIS (Mayo Clinic) |
| Polio (Sabin I) | PVS-RIPO (Duke Univ.) |
| Seneca Valley Virus | NTX-10 (Neotropix Inc.) |
| Reovirus | REOLYSIN² (Oncolytics,Biotech, Inc.) |

In another embodiment, a therapeutic cancer vaccine can be yeast based. *See, e.g.,* Cancer Immunol Immunother. 2014 Mar; 63(3):225-34. In one non-limiting example, a yeast-based therapeutic cancer vaccine includes a recombinant yeast-CEA vaccine (GI-6207) which is currently in phase II clinical trials. *See* J Clin Oncol 31, 2013 (suppl; abstr TPS3127).

In still another embodiment, a therapeutic cancer vaccine can be bacterial based. In one non-limiting example, the bacterial based cancer vaccine includes a Coley vaccine. See Clin Cancer Res. 2012 Oct 1; 18 (19):5449-59.

### Administering an Immune Checkpoint Antagonist after Boost Dosages in Heterologous Prime-Boost Increases Efficacy of Cancer Treatments

PROSTVAC^{®} comprises a heterologous prime-boost regimen that includes a single prime administration with PROSTVAC-V (Vaccinia virus expressing PSA and TRICOM^{™}) followed by one or more consecutive boosting doses of PROSTVAC-F (Fowlpoxvirus expressing PSA and TRICOM^{™} ); also described in J Clin Oncol 2010, 28:1099-1105, which is incorporated by reference herein..

As shown and described in Figures 21-23 and Examples 40-43, in a cancer treatment a heterologous PROSTVAC^{®} dosing regimen greatly enhances the magnitude and quality of the PSA-specific T cell response as compared to homologous dosing with the same vector. Additionally, the figures and examples demonstrate that priming with PROSTVAC-V and boosting with PROSTVAC-F provides the added benefit of focusing the highly functional CD8 CTL immune response towards PSA, the target tumor antigen, and away from the vaccinia vector.

In at least in one aspect, when one or more dosages of a recombinant poxvirus are administered as part of a cancer treatment, greater therapeutic efficacy is achieved by administering one or more immune checkpoint antagonists in combination with a second or subsequent recombinant poxvirus dosage or administration.

In a more particular aspect, when one or more dosages of a recombinant poxvirus of the present invention are administered as part of a cancer treatment as part of a heterologous prime-boost regimen greater therapeutic efficacy is achieved by administering at least one checkpoint antagonist in combination with the second or subsequent boost dosages of recombinant poxvirus encoding at least one TAA. This greater therapeutic efficacy is realized, at least in part, because during and after the second or subsequent boost dosages of a heterologous prime-boost regimen a patient's immune T-cell response is more focused on the tumor antigen as compared to the recombinant poxvirus. Accordingly, at least in one aspect, an administration of an immune checkpoint antagonist during the boosting dosages functions to enhance a patient's immune response to the tumor antigen, and thereby increase a patient's immune response more specifically to the tumor.

In at least another aspect, as part of a cancer treatment involving a heterologous prime-boost regimen, administering at least one immune checkpoint antagonist in combination with the second or subsequent boost dosages of recombinant poxvirus maximizes therapeutic benefits of the immune checkpoint antagonist while minimizing adverse side effects that have been seen in the immune checkpoint treatments.

In view of the teachings of the present disclosure, in additional embodiments, the present invention includes a method for treating a human cancer patient, the method comprising administering to the patient: (a) a first therapeutic cancer vaccine, such as but not limited to, a first recombinant poxvirus, the poxvirus comprising at least one tumor-associated antigen (TAA); and (b) a second therapeutic cancer vaccine, such as but not limited to, a second recombinant poxvirus, the second recombinant poxvirus comprising at least one tumor-associated antigen (TAA); wherein the second recombinant poxvirus is administered in combination with at least one immune checkpoint antagonist. In an additional embodiment, the second recombinant poxvirus is different than the first recombinant poxvirus. In other embodiments, the second recombinant poxvirus is from a different genus than the first recombinant poxvirus.

In another embodiment, the first and second recombinant poxviruses are different or are of a different genus and are administered as a heterologous prime-boost regimen, the heterologous prime-boost regimen comprising: a) administering the first recombinant poxvirus as a first prime dose; and b) administering the second recombinant poxvirus as one or more boost doses in combination with at least one immune checkpoint antagonist. In a preferred embodiment, the heterologous prime boost regimen is selected from PROSTVAC^{®}, CV301 or MVA-BN-CV301.

In yet another embodiment, it is contemplated that the first recombinant poxvirus or the recombinant poxvirus of the initial or prime dose does not include an immune checkpoint antagonist.

It is additionally contemplated that the first and second recombinant poxviruses can be any poxvirus, such as but not limited to, those described in the present disclosure. It is further contemplated that the at least one tumor-associated antigen (TAA) can be any TAA, such as but not limited to, those TAAs described in the present disclosure.

In one or more embodiments, at least one immune checkpoint antagonist is administered on the same day or within 1, 2, 3, 4, 5, 6, or 7, days of the second or subsequent dosages of a recombinant poxvirus encoding at least one TAA. In a preferred embodiment, at least one immune checkpoint antagonist is administered as part of a heterologous prime-boost regimen, and is administered on the same day or within 1, 2, 3, 4, 5, 6, or 7, days of the second or subsequent boost dosages of a recombinant poxvirus encoding at least one TAA.

In one or more embodiments, at least one immune checkpoint antagonist is administered after the second or subsequent dosages of a recombinant poxvirus encoding at least one TAA is administered. In a preferred embodiment, at least one immune checkpoint antagonist is administered as part of a heterologous prime-boost regimen, and is administered after the second or subsequent boost dosages of a recombinant poxvirus encoding at least one TAA. It is contemplated that, after the second or subsequent boost dosages of a recombinant poxvirus, the time intervals at which at least one immune checkpoint antagonist is administered can include those time intervals described in the present disclosure.

It is additionally contemplated that when administered in combination with a second or one or more subsequent boost dosages of a recombinant poxvirus encoding at least one TAA, at least one immune checkpoint antagonist can be administered at a dosage or concentration as provided in the present disclosure.

### Therapeutic Compositions and Uses

The present invention further relates to the use of the poxvirus vectors according to the invention for the preparation of therapeutic compositions or vaccines which are capable of inducing or contributing to the occurrence or improvement of an immunological reaction against tumor epitopes. The present invention thus provides vectors that are useful as a medicament or vaccine.

Accordingly, the invention relates to an immunogenic composition comprising a MVA vector according to the invention in combination with one or more antibody, antagonist according to the invention.

Thus, the MVA vectors according to the invention can be used for the preparation of therapeutic composition for the treatment of cancer.

The invention encompasses a composition for use in prophylactic and/or therapeutic vaccination protocols, for the treatment of tumors and especially as anti-cancer treatment.

In one embodiment, the invention encompasses a composition for administration to or treatment of a cancer patient, particularly those cancers such as, but not limited to, breast cancer, lung cancer, gastric cancer, bladder cancer, kidney cancer, liver cancer, pancreatic cancer, prostate cancer, ovarian cancer, or colorectal cancer.

In one embodiment, the invention encompasses use of a composition for administration to or treatment of a cancer patient, particularly those cancers selected from breast cancer, lung cancer, gastric cancer, bladder cancer, kidney cancer, liver cancer, pancreatic cancer, prostate cancer, ovarian cancer, or colorectal cancer.

In additional embodiments, the invention encompasses a composition or use of a composition for administration to or treatment of a cancer patient, particularly a breast cancer patient.

In one embodiment the composition for simultaneous or sequential administration comprising an MVA vector according to the invention and one or more antibodies, antagonists according to the invention.

In still additional embodiments, the invention encompasses a composition or use of a composition for administration to or treatment of a cancer patient, particularly a prostate cancer patient.

In one embodiment the composition for simultaneous or sequential administration comprising a PROSTVAC^{®} vector according to the invention and one or more antibodies, or antagonists according to the invention.

In accordance with the various embodiments of the present invention, in cancer treatment, when an immune checkpoint antagonist is combined with a poxviral vector encoding a TAA, the dosage at which the immune checkpoint antagonist is therapeutically effective is less or is decreased as compared to either 1) a therapeutically effective dosage of an immune checkpoint antagonist without the poxviral vector or 2) a therapeutically effective dosage of an immune checkpoint antagonist by itself. As such, therapeutically effective dosages of immune checkpoint antagonists can be administered at lower dosages, thereby reducing and/or eliminating the toxic and adverse side effects of the treatments found in the prior art.

A "therapeutically effective amount" is a quantity of a composition sufficient to achieve a desired therapeutic or clinical effect in a subject being treated. For example, a therapeutically effective amount of a poxviral vector comprising a tumor associated antigen (TAA) nucleic acid (such as, but not limited to, CEA, MUC-1, PSA, HER-2, or Brachyury antigen) operably linked to an expression control sequence would be an amount sufficient to elicit a TAA-specific immune response, to reduce tumor size or burden, to reduce the number of tumor metastases, to delay progression of a cancer, or to increase overall survival of a patient or population of patients having cancer.

Also, by way of example, a therapeutically effective amount of an immune checkpoint antagonist or agonist would be an amount sufficient to inhibit the function of the targeted immune checkpoint molecule, to bind to the targeted immune checkpoint molecule to reduce tumor size or burden, to reduce the number of tumor metastases, to delay progression of a cancer, or to increase overall survival of a patient or population of patients having cancer.

A "therapeutic effect" is a desired therapeutic or clinical effect in a subject. A "therapeutic effect" in a cancer treatment can be characterized by: a reduction in tumor size, tumor mass, or tumor burden; a reduction in the number of tumor metastases; a delay in the progression of a cancer, or an increase in the overall survival of patient or population of patients having cancer.

### Combination Cancer Treatment of an Immune Checkpoint Antagonist with a Recombinant Poxvirus enables Therapeutic Efficacy at Low Dosages of the Antagonist or Agonist

As illustrated and described herein, when an immune checkpoint antagonist is combined with a poxviral vector encoding a TAA, the dosage at which the immune checkpoint antagonist or agonist is therapeutically effective is less or is decreased. In various additional embodiments, the present disclosure includes administering to a human cancer patient a combination of: (a) a therapeutically effective amount of a recombinant poxvirus, the poxvirus comprising at least one tumor-associated antigen (TAA); and (b) a sub-therapeutically effective amount of at least one immune checkpoint antagonist. In this embodiment, the sub-therapeutically effective amount of the immune checkpoint antagonist achieves an increased "therapeutic effect" as compared to an administration of either the poxvirus comprising at least one TAA alone or the sub-therapeutically effective amount of the at least one immune checkpoint antagonist alone or in combination with other immune checkpoint antagonists .

In at least one aspect, the sub-therapeutically effective dosages are configured to maximize therapeutic benefits while minimizing adverse side effects that have been seen in the immune checkpoint treatments in the prior art.

A "sub-therapeutically effective amount" is a dosage or amount at which the immune checkpoint antagonist is ineffective for a "therapeutic effect" by itself, when administered as a mono-therapeutic or monotherapy, or when administered in conjunction with other or multiple immune checkpoint antagonists or agonists.

In one embodiment, the sub-therapeutically effective amount of the at least one immune checkpoint antagonist is from about 99% to about 5%, about 90% to about 5%, about 85% to about 5%, about 80% to about 5%, 75% to about 5%, from about 70% to about 10%, from about 65% to about 15%, from about 60% to about 20%, from about 55% to about 25%, from about 50% to about 30%, or from about 50% to about 10% of a therapeutically effective amount of an immune checkpoint antagonist. In a preferred embodiment the sub-therapeutically effective amount of the at least one immune checkpoint antagonist is from about a 99% decrease to about a 30% decrease of a therapeutically effective amount of an immune checkpoint antagonist . In another preferred embodiment, the sub-therapeutically effective amount of the at least one immune checkpoint antagonist is from about a 90% decrease to about a 30% decrease of a therapeutically effective amount of an immune checkpoint antagonist or agonist. In still another preferred embodiment, the sub-therapeutically effective amount of the at least one immune checkpoint antagonist or agonist is about a 99% decrease, about a 90% decrease, or about a 30% decrease of a therapeutically effective amount of an immune checkpoint antagonist.

In another embodiment, the sub-therapeutically effective amount of the at least one immune checkpoint antagonist is from 99% to 5%, 90% to 5%, 85% to 5%, 80% to 5%, 75% to 5%, from 70% to 10%, from 65% to 15%, from 60% to 20%, from 55% to 25%, from 50% to 30%, or from 50% to 10% of a therapeutically effective amount of an immune checkpoint antagonist. In a preferred embodiment the sub-therapeutically effective amount of the at least one immune checkpoint antagonist is from a 99% decrease to a 30% decrease of a therapeutically effective amount of an immune checkpoint antagonist or agonist. In another preferred embodiment, the sub-therapeutically effective amount of the at least one immune checkpoint antagonist is from about a 90% decrease to about a 30% decrease of a therapeutically effective amount of an immune checkpoint antagonist. In still another preferred embodiment, the sub-therapeutically effective amount of the at least one immune checkpoint antagonist is a 99% decrease, a 90% decrease, or a 30% decrease of a therapeutically effective amount of an immune checkpoint antagonist.

In one or more embodiments a sub-therapeutically effective amount of at least one immune checkpoint antagonist may be used in combination with a recombinant poxvirus including at least one TAA as part of a heterologous or homologous prime-boost regimen as described herein.

### Additional Embodiments for Combinations or Medicaments

In additional embodiments, the present disclosure encompasses a combination or medicament for use in treating a human cancer patient. The combination or medicament comprises a recombinant poxvirus vector, the poxvirus vector comprising at least one tumor associated antigen (TAA); (b) a PD-1 antagonist; and (c) a CTLA-4 antagonist. The PD-1 antagonist and the CTLA-4 antagonist can include an anti-PD-1 antagonist antibody and an anti-CTLA-4 antibody, respectively.

In another embodiment, the present disclosure can include a combination or medicament for use in increasing overall survival rate in a human cancer patient, the combination or medicament comprising: (a) a poxvirus including at least one tumor associated antigen (TAA); (b) a PD-1 antagonist; and (c) a CTLA-4 antagonist. The PD-1 antagonist and the CTLA-4 antagonist can include an anti-PD-1 antagonist antibody and an anti-CTLA-4 antibody, respectively.

In still another embodiment, the present disclosure can include a combination or medicament for use in increasing overall survival rate in a human cancer patient, the combination or medicament comprising: (a) a poxvirus including at least one tumor associated antigen (TAA); and (b) a therapeutically effective amount of at least one immune checkpoint antagonist; wherein the therapeutically effective amount of the at least one immune checkpoint antagonist combined with the poxvirus vector has an increased therapeutic effect as compared to an administration of either a poxvirus vector comprising at least one TAA alone or at least one immune checkpoint antagonist alone or in combination with other immune checkpoint antagonists.

In still an additional embodiment, the present disclosure can include a combination or medicament for use in treating a human cancer patient, the combination or medicament comprising: (a) a therapeutically effective amount of a recombinant poxvirus, the poxvirus comprising at least one tumor-associated antigen (TAA); and (b) a sub-therapeutically effective amount of at least one immune checkpoint antagonist, wherein the sub-therapeutically effective amount of the at least one immune checkpoint antagonist is such that the therapeutic effect of the combination is increased as compared to an administration of either the poxvirus comprising at least one TAA alone or the sub-therapeutically effective amount of the at least one immune checkpoint antagonist or agonist alone or in combination with other immune checkpoint antagonists. It is contemplated that the combination or medicament can include an immune checkpoint antagonist or agonist selected from a CTLA-4 antagonist or a PD-1 antagonist. It is contemplated that the various immune checkpoint antagonists can be embodied in one or more antibodies.

In still an additional embodiment, the present disclosure can include a combination or medicament for use in treating a human cancer patient, the combination or medicament comprising: (a) a therapeutically effective amount of a recombinant poxvirus, the poxvirus vector comprising at least one tumor associated antigen (TAA); and (b) a therapeutically effective amount or a sub-therapeutically effective amount of at least one immune checkpoint antagonist; wherein the therapeutically effective amount or the sub-therapeutically effective amount of at least one immune checkpoint antagonist is configured to be administered after the therapeutically effective of a recombinant poxvirus; and wherein the therapeutically effective amount and sub-therapeutically effective amount of the at least one immune checkpoint antagonist combined with the poxvirus vector has an increased therapeutic effect as compared to an administration of either a poxvirus vector comprising at least one TAA alone or a therapeutically effective amount or sub-therapeutically effective amount of at least one immune checkpoint antagonist alone or in combination with other immune checkpoint antagonists .

In yet another embodiment, the present disclosure can include a combination or medicament for use in treating a human cancer patient, the combination or medicament comprising at least a first and second administration, each administration comprising: (a) a therapeutically effective amount of a recombinant poxvirus, the poxvirus comprising at least one tumor associated antigen (TAA); and (b) a therapeutically effective amount or a sub-therapeutically effective amount of at least one immune checkpoint antagonist ; wherein the second administration of a therapeutically effective amount or sub-therapeutically effective amount of at least one immune checkpoint antagonist is increased as compared to the first administration of therapeutically effective amount or sub-therapeutically effective amount of at least one immune checkpoint antagonist ; and wherein the therapeutically effective amount or sub-therapeutically effective amount of the at least one immune checkpoint antagonist or agonist combined with the poxvirus has an increased therapeutic effect as compared to an administration of either a poxvirus comprising at least one TAA alone or a therapeutically effective amount or sub-therapeutically effective amount of at least one immune checkpoint antagonist alone or in combination with other immune checkpoint antagonists.

In still another embodiment, the recombinant poxvirus encoding a TAA in the combination or medicaments described herein can be PROSTVAC^{®}. In yet another embodiment, the recombinant poxvirus encoding a TAA in the combination or medicaments described herein can be CV301.

In still an additional embodiment, the present disclosure can include use of: (a) a recombinant poxvirus, the poxvirus comprising at least one tumor associated antigen (TAA); (b) a PD-1 antagonist; and (c) a CTLA-4 antagonist in the preparation of a pharmaceutical composition or medicament. The PD-1 antagonist and the CTLA-4 antagonist can include an anti-PD-1 antagonist antibody and an anti-CTLA-4 antibody, respectively. In an additional embodiment, the use of the disclosed pharmaceutical composition or medicament can be for the treatment of a human cancer patient.

In still an additional embodiment, the present disclosure can include use of: (a) a poxvirus comprising at least one tumor associated antigen (TAA); and (b) a therapeutically effective amount of at least one immune checkpoint antagonist in the preparation of a pharmaceutical composition or medicament; wherein the therapeutically effective amount of the at least one immune checkpoint antagonist combined with the poxvirus vector has an increased therapeutic effect as compared to an administration of either a poxvirus vector comprising at least one TAA alone or at least one immune checkpoint antagonist alone or in combination with other immune checkpoint antagonists.

In still an additional embodiment, the present disclosure can include use of (a) a therapeutically effective amount of a recombinant poxvirus, the poxvirus comprising at least one tumor-associated antigen (TAA); and (b) a sub-therapeutically effective amount of at least one immune checkpoint antagonist in the preparation of a pharmaceutical composition or medicament; wherein the sub-therapeutically effective amount of the at least one immune checkpoint antagonist is such that the therapeutic effect of the combination is increased as compared to an administration of either the poxvirus comprising at least one TAA alone or the sub-therapeutically effective amount of the at least one immune checkpoint antagonist alone. It is contemplated that the at least one immune checkpoint antagonist can be selected from a CTLA-4 antagonist or a PD-1 antagonist. It is contemplated that the various immune checkpoint antagonists can be embodied in one or more antibodies.

In still an additional embodiment, the present disclosure can include use of: (a) a therapeutically effective amount of a recombinant poxvirus, the poxvirus comprising at least one tumor associated antigen (TAA); and (b) a therapeutically effective amount or a sub-therapeutically effective amount of at least one immune checkpoint antagonist in the preparation of a pharmaceutical composition or medicament; wherein the therapeutically effective amount or sub-therapeutically effective amount of at least one immune checkpoint antagonist is configured to be administered after the therapeutically effective amount of a recombinant poxvirus; and wherein the therapeutically effective amount or sub-therapeutically effective amount of the at least one immune checkpoint antagonist combined with the poxvirus vector has an increased therapeutic effect as compared to an administration of either a poxvirus vector comprising at least one TAA alone or a therapeutically effective amount or sub-therapeutically effective amount of at least one immune checkpoint antagonist alone or in combination with other immune checkpoint antagonists.

In yet another embodiment, the present disclosure can include use of at least a first and second dosage administration, each dosage administration comprising: (a) a therapeutically effective amount of a recombinant poxvirus, the poxvirus comprising at least one tumor associated antigen (TAA); and (b) a therapeutically effective amount or a sub-therapeutically effective amount of at least one immune checkpoint antagonist in the preparation of a pharmaceutical composition or medicament; wherein the therapeutically effective amount or sub-therapeutically effective amount of at least one immune checkpoint antagonist of the second administration is increased as compared to the first administration; and wherein the therapeutically effective amount or sub-therapeutically effective amount of the at least one immune checkpoint antagonist combined with the poxvirus vector has an increased therapeutic effect as compared to an administration of either a poxvirus vector comprising at least one TAA alone or a therapeutically effective amount or sub-therapeutically effective amount of at least one immune checkpoint antagonist alone or in combination with other immune checkpoint antagonists.

### EXAMPLES

### Example 1

### Construction of MVA-BN-mHER2

Simultaneous infection and transfection of cultures allowed homologous recombination to occur between the viral genome and the recombination plasmid. Insert-carrying virus was isolated, characterized, and virus stocks were prepared.

Plasmid pBN146 contains sequences which are also present in MVA-BN (the 14L and 15L open reading frames). The mHER2 sequence was inserted between the MVA-BN sequences to allow for recombination into the MVA-BN viral genome. Thus, a plasmid was constructed that contained the mHER2 sequence downstream of a poxvirus promoter, specifically the cowpox virus A-type inclusion body gene promoter. The plasmid also contained a selection cassette comprising a synthetic vaccinia virus promoter (Ps), a drug resistance gene (guanine-xanthine phosphoribosyltransferase; Ecogpt), an internal ribosomal entry site (IRES), and the enhanced green fluorescent protein (EGFP). Both selection genes (gpt and EGFP) were encoded by a single bicistronic transcript.

The HER-2 sequence was modified by addition of nucleotides sequences encoding tetanus toxin epitopes of p2 and p30 to increase the immune response against it. After mHER2 was inserted into the MVA-BN genome, the virus "insert region" had the following structure:

ATI promoter - mHER2 sequence - Ps promoter - gpt - IRES - EGFP. The insert region was flanked by MVA-BN I4L intergenic region sequences (F1 and F2) in the bacterial recombination plasmid pBN146. The nucleotide sequence of the construct is shown below.

AGTATGCATTTTTACGGATGGAGTCTCGGTCTAAAAACGGAATGTACTATCTACGTACG
AAACCCGCATCCGCTCCCATTCAATTCACATTGGACAAGGATAAAATAAAACCACTGGTG
GTTTGCGATTCCGAAATCTGTACATCATGCAGTGGTTAAACAAATCTAGAACTAGTTTAA
TTAAGGAGCTGTTTTGAATAAAATTTTTTTATAATAAATCTAGAACTAGTGGATCCCCCG
GGCTGCAGGAATTCGATCTAGCCGCCACCATGGAGCTGGCGGCCTTGTGCCGCTGGGGGC
TCCTCCTCGCCCTCTTGCCCCCCGGAGCCGCGAGCACCCAAGTGTGCACCGGCACAGACA
TGAAGCTGCGGCTCCCTGCCAGTCCCGAGACCCACCTGGACATGCTCCGCCACCTCTACC
AGGGCTGCCAGGTGGTGCAGGGAAACCTGGAACTCACCTACCTGCCCACCAATGCCAGCT
TAAGTTTCCTGCAGGATATCCAGGAGGTGCAGGGCTACGTGCTCATCGCTCACAACCAAG
TGAGGCAGGTCCCACTGCAGAGGCTGCGGATTGTGCGAGGCACCCAGCTCTTTGAGGACA
ACTATGCCCTGGCCGTGCTAGACAATGGAGACCCGCTGAACAATACCACCCCTGTCACAG
GGGCCTCCCCAGGAGGCCTGCGGGAGCTGCAGCTTCGAAGCCTCACAGAGATCTTGAAAG
GAGGGGTCTTGATCCAGCGGAACCCCCAGCTCTGCTACCAGGACACGATTTTGTGGAAGG
ACATCTTCCACAAGAACAACCAGCTGGCTCTCACACTGATAGACACCAACCGCTCTCGGG
CCTGCCACCCCTGTTCTCCGATGTGTAAGGGCTCCCGCTGCTGGGGAGAGAGTTCTGAGG
ATTGTCAGAGCCTGACGCGCACTGTCTGTGCCGGTGGCTGTGCCCGCTGCAAGGGGCCAC
TGCCCACTGACTGCTGCCATGAGCAGTGTGCTGCCGGCTGCACGGGCCCCAAGCACTCTG
ACTGCCTGGCCTGCCTCCACTTCAACCACAGTGGCATCTGTGAGCTGCACTGCCCAGCCC
TGGTCCAGTACATCAAAGCTAACTCCAAATTCATCGGTATCACCGAGCTGCGGTATACAT
TCGGCGCCAGCTGTGTGACTGCCTGTCCCTACAACTACCTTTCTACGGACGTGGGATCCT
GCACCCTCGTCTGCCCCCTGCACAACCAAGAGGTGACAGCAGAGGATGGAACACAGCGGT
GTGAGAAGTGCAGCAAGCCCTGTGCCCGAGTGTGCTATGGTCTGGGCATGGAGCACTTGC
GAGAGGTGAGGGCAGTTACCAGTGCCAATATCCAGGAGTTTGCTGGCTGCAAGAAGATCT

TTGGGAGCCTGGCATTTCTGCCGGAGAGCTTTGATGGGGACCCAGCCTCCAACACTGCCC
CGCTCCAGCCAGAGCAGCTCCAAGTGTTTGAGACTCTGGAAGAGATCACAGGTTACCTAT
ACATCTCAGCATGGCCGGACAGCCTGCCTGACCTCAGCGTCTTCCAGAACCTGCAAGTAA
TCCGGGGACGAATTCTGCACAATGGCGCCTACTCGCTGACCCTGCAAGGGCTGGGCATCA
GCTGGCTGGGGCTGCGCTCACTGAGGGAACTGGGCAGTGGACTGGCCCTCATCCACCATA
ACACCCACCTCTGCTTCGTGCACACGGTGCCCTGGGACCAGCTCTTTCGGAACCCGCACC
AAGCTCTGCTCCACACTGCCAACCGGCCAGAGGACGAGTGTGTGGGCGAGGGCCTGGCCT
GCCACCAGCTGTGCGCCCGAGGGCACTGCTGGGGTCCAGGGCCCACCCAGTGTGTCAACT
GCAGCCAGTTCCTTCGGGGCCAGGAGTGCGTGGAGGAATGCCGAGTACTGCAGGGGCTCC
CCAGGGAGTATGTGAATGCCAGGCACTGTTTGCCGTGCCACCCTGAGTGTCAGCCCCAGA
ATGGCTCAGTGACCTGTTTTGGACCGGAGGCTGACCAGTGTGTGGCCTGTGCCCACTATA
AGGACCCTCCCTTCTGCGTGGCCCGCTGCCCCAGCGGTGTGAAACCTGACCTCTCCTACA
TGCCCATCTGGAAGTTTCCAGATGAGGAGGGCGCATGCCAGCCTTGCCCCATCAACTGCA
CCCACTCCTGTGTGGACCTGGATGACAAGGGCTGCCCCGCCGAGCAGAGAGCCAGCCCTC
TGACGTCCTTCAACAACTTCACCGTGAGCTTCTGGCTGCGCGTGCCCAAGGTGAGCGCCA
GCCACCTGGAGATCGTCTCTGCGGTGGTTGGCATTCTGTAGAAGCTTGGTACCGAGCTCG
GATCCACTAGTCCAGTGTGGTGGAATTCTGCAGATATCCAGCACAGTGGCGGCCATCAAG
CTTATCGATACCGTCGACCTCGAGGGGGGGCCCGGTACCCAGTTAATTAAGGATCCCCCG
GGCTGCAGGAATTCCATTTTTATTCTCAAATGAGATAAAGTGAAAATATATATCATATAT
ACAAAGTA

(SEQ ID NO:1).

HER2 start and stop codons are indicated in bold. Flanking sequences are indicated in italics.

Translation of the encoded mHER2 polypeptide is shown below:

MELAALCRWGLLLALLPPGAASTQVCTGTDMKLRLPASPETHLDMLRHLYQGCQVVQGNL
ELTYLPTNASLSFLQDIQEVQGYVLIAHNQVRQVPLQRLRIVRGTQLFEDNYALAVLDNG
DPLNNTTPVTGASPGGLRELQLRSLTEILKGGVLIQRNPQLCYQDTILWKDIFHKNNQLA
LTLIDTNRSRACHPCSPMCKGSRCWGESSEDCQSLTRTVCAGGCARCKGPLPTDCCHEQC
AAGCTGPKHSDCLACLHFNHSGICELHCPALVQYIKANSKFIGITELRYTFGASCVTACP
YNYLSTDVGSCTLVCPLHNQEVTAEDGTQRCEKCSKPCARVCYGLGMEHLREVRAVTSAN
IQEFAGCKKIFGSLAFLPESFDGDPASNTAPLQPEQLQVFETLEEITGYLYISAWPDSLP
DLSVFQNLQVIRGRILHNGAYSLTLQGLGISWLGLRSLRELGSGLALIHHNTHLCFVHTV
PWDQLFRNPHQALLHTANRPEDECVGEGLACHQLCARGHCWGPGPTQCVNCSQFLRGQEC
VEECRVLQGLPREYVNARHCLPCHPECQPQNGSVTCFGPEADQCVACAHYKDPPFCVARC
PSGVKPDLSYMPIWKFPDEEGACQPCPINCTHSCVDLDDKGCPAEQRASPLTSFNNFTVS
FWLRVPKVSASHLEIVSAVVGIL.

(SEQ ID NO:2).

The tetanus toxin epitopes of p2 and p30 sequences are indicated in bold.

CEF cultures were inoculated with MVA-BN and also transfected with pBN146 plasmid DNA. In turn, samples from these cell cultures were inoculated into CEF cultures in medium containing selection drugs, and EGFP-expressing viral clones were isolated by plaque purification. Virus stocks which grew in the presence of the selection drugs and expressed EGFP were designated MVA-BN-mHER2. Generation of MVA-BN-mHER2 and preparation of the virus stock involved twelve (12) sequential passages, including five (5) plaque purifications.

MVA-BN-mHER2 was passaged in CEF cell cultures in the absence of selection drugs. The absence of selection drugs allowed loss of the region encoding the selection genes, gpt and EGFP and the associated promoter (the selection cassette) from the inserted sequence. Recombination resulting in loss of the selection cassette is mediated by the F1 I4L region and a subsection of that region, the F1 repeat (F1 rpt), which flank the selection cassette in plasmid pBN146. These duplicated sequences were included to mediate recombination that results in loss of the selection cassette, leaving only the mHER2 sequence inserted in the I4L intergenic region.

Plaque-purified virus lacking the selection cassette was prepared. Such preparation involved fifteen (15) passages including five (5) plaque purifications.

The presence of the mHER2 sequence and absence of parental MVA-BN virus in MVA-BN-mHER2 stocks was confirmed by PCR analysis, and nested PCR was used to verify the absence of the selection cassette (the gpt and EGFP genes).

Expression of the mHER2 protein was demonstrated in cells inoculated with MVA-BN-mHER2 *in vitro.*

### Example 2

### Increase in IFNγ as a result of treatment with MVA-BN-mHER2 and anti-CTLA4

Female BALB/c mice (6-8 weeks old, ~ 20 g) were purchased from Simonsen Laboratories, Gilroy, CA. For the experimental lung metastasis model, mice were implanted i.v. on day 1 with 5.0×10⁴ CT26-HER-2 cells in 300 µL DPBS which forms tumors in the lungs.

The following antibodies were purchased from Bio X Cell (West, Lebanon, NH): anti-ICOS agonistic Antibody (Clone 17G9), anti-CTLA-4 (9D9), anti-PD-1 (RMP1-14), and anti-LAG-3 (C9B7W). All antibodies were injected i.p. at 200 µg per mouse in 100 µL PBS on the days 3 and 17 unless otherwise indicated. For virus treatments, mice were treated with 7.1 µL of 1.0×10⁷ Inf. U. MVA-BN-HER2 by tail scarification (t.s., produced by Bavarian Nordic [BN], Martinsried, Germany) on the days days 4 and 18 unless otherwise indicated.

On day 25, whole blood, tumor/lungs or spleens were pooled (4 mice/group) for flow cytometric analysis. Splenocytes were prepared by pressing the spleens between two frosted glass slides, and lysing the red blood cells with ACK lysis buffer (Life Technologies, Grand Island, NY). Lungs and associated tumors were diced to ~1-2 mm³ pieces and further digested to single cell suspensions for 1 h at 37 °C in DMEM with 10% FBS, 50 U/mL DNAse I and 250 U/mL Collagenase I (Worthington Biochemical Corporation, Lakewood, NJ). The red blood cells in both the lungs and whole blood were lysed with RBC Lysis Buffer (eBiosceince).

Antibodies against the following proteins were purchased from BD Bioscience (San Jose, CA): CD3e (500A2), CD4 (RM4-5), CD8a (53-6.7), CD107a (1D4B), IFN-γ (XMG1.2); BioLegend (San Diego, CA): CD3e (145-2C11), IL-2 (JES6-5H4), LAG-3 (C9B7W), PD-1 (CD279, 29F.1A12), Tim-3 (RMT3-23), TNF-α (MP6-XT22); or eBioscience (San Diego, CA): ICOS (7E.17G9), CD16/CD32 (93).

To identify degranulating T-cells, single cell suspensions of splenocytes (2×10⁶ cells/well) or tumor/lungs (1×10⁶ cells/well) were re-suspended in RPMI-10 (10% FBS, 1% Penstrep, and 0.1% β-mercaptoethanol) and restimulated overnight at 37 °C in the presence of anti-CD107a antibody and Golgi Stop (BD Bioscience). The following peptides were used for the restimulation: MVA E3L and F2L (VGPSNSPTF and SPGAAGYDL, 1 µM each), HER2 p63 (TYLPTNASL, 1 µM), HER2 ECD overlapping peptide library (HER2 OPL, 1 µM), and PSA (HPQKVTKFML, 1 µM) (5, 9-11). Concanavalin A (ConA) was used at 5 µg/mL as a positive control. The next day, cells were washed, blocked with anti-CD16/CD32 antibodies, and stained for surface markers. Cells were then washed, fixed/permeabilized with BD Cytofix/Cytoperm Buffer, and stained intracellularly for IFN-γ.

Additional intracellular cytokine staining was performed on splenocytes as described above except the anti-CD107a antibody was omitted, Golgi Stop and Golgi Plug were added, and cells were stained intracellularly for IFN-γ, IL-2, and TNF-α.

All FACS samples were acquired on the BD LSRII or Fortessa and analyzed using FlowJo version 9.6.2 (TreeStar Inc., Ashland, OR).

All statistical analyses were performed using GraphPad Prism version 6.01 for Windows (GraphPad Software, La Jolla, CA).

Results are shown in Figure 1. Tumor antigen specific degranulating cells (HER2 p63 CD107+IFNγ+) increased in the tumor/lungs and spleen of mice treated with MVA-BN-HER2 and CTLA-4 combination therapy. Virus specific degranulating cells (MVA E3L F2L CD107+ IFNγ+) were high in both the tumor/lungs and spleen of mice treated with MVA-BN-HER2.

### Example 3

### Increase in IFNγ and Cytokine Production as a result of treatment with MVA-BN-mHER2 and anti-CTLA4

Treatment with MVA-BN-HER2 increased the magnitude and quality of tumor antigen and virus specific T-cells in the spleen. Mice were implanted with 5×10⁴ CT26-HER-2 cells, and treated with MVA-BN-HER2 and anti-CTLA-4, as described in Example 2. On day 25, tumor/lungs or spleens were pooled (4 mice/group) and re-stimulated overnight to measure virus and tumor antigen specific responses as described in Example 2.

Results are shown in Figure 2, A) Pie charts are area weighted to reflect the number of IFNγ+ cells per million CD8+ T-cells. B) IFNγ MFI increases with tumor antigen specific (HER2 p63) polyfunctional T-cells with combination therapy.

In both Examples 2 and 3, as a result of treatment with the combination of MVA-BN-HER2 and anti-CTLA-4, the number and quality of antigen and virus specific T-cells were increased. Furthermore, the combination treatment increased the numbers of antigen and virus specific T-cells that were present in the tumor. As shown by the Figures 1 and 2, the increase of antigen and virus specific T-cells at tumor locations is marked improvement over treatments using either MVA-BN-HER2 or anti-CTLA-4 treatment alone. Such increases in the quantity and specificity of antigen and virus specific T-cells can provide better and more effective treatments for human cancers.

Additionally, shown in Figure 2, MVA-BN-HER2 induces tumor antigen specific T cells that produce IFNγ. It is contemplated by the present disclosure that virus induced TILs (tumor infiltrating lymphocytes) that secrete IPNγ may lead to increased PD-L1 on tumor cells; supporting blockade of this pathway in combination with virus treatment.

### Example 4

Mice were implanted i.v. with 5×10⁴ CT26-HER-2 cells, and treated with MVA-BN-HER2 and anti-CTLA-4 as described in Example 2. ^{∗∗∗∗} p<0.0001, Log-Rank Test. *Results.* Shown in Figure 3, the results demonstrate that treatment with MVA-BN-HER2 and anti-CTLA-4 increases the overall survival rate of subjects significantly as compared to treatment of cancers with either MVA-BN-HER2 or anti-CTLA-4 alone.

### Example 5

### MVA-BN-HER2 Significantly Reduces Pulmonary Tumor Burden by Day 25

Mice were implanted i.v. with 5×10⁴ CT26-HER-2 cells, and treated with MVA-BN-HER2 and anti-CTLA-4 as described in Example 2. A) Mice were euthanized and perfused through the trachea with Trypan Blue. Lungs were removed and briefly submerged in Hydrogen Peroxide and washed in PBS. Tumors visible as small masses in Untreated and anti-CTLA-4 treated mice. There were no visible tumors in the lungs of mice treated with MVA-BN-HER2. Scale bar equals 1 cm. B) Lung weight on Day 25. ^{∗∗∗∗} p<0.0001, One-Way ANOVA with Dunnett's Multiple Comparisons test.

*Results.* Shown in Figure 4, the results demonstrate that treatment with MVA-BN-HER2 alone or in combination with anti-CTLA-4 significantly decreased tumor burden by day 25 as compared to treatment of cancers with no treatment or anti-CTLA-4 alone.

### Example 6

### MVA-BN-HER2 and anti-CTLA-4 Treatment Increases Overall Survival Rate

Mice were implanted i.v. with 5×10⁴ CT26-HER-2 cells and treated with MVA-BN-HER2 as described in Example 2. Mice were treated with anti-CTLA-4 on days 4 and 18 at 200 µg (A, 10 mg/kg), 66 µg (B, 3 mg/kg), or 22 µg (C, 1 mg/kg) i.p. in 100 µL PBS. ^{∗∗∗∗} p<0.0001, Log-Rank Test.

*Results.* Shown in Figure 5, the results demonstrate that MVA-BN-HER2 in combination with anti-CTLA-4 increased overall survival rate of subjects at each dosage concentration as compared to treatment no treatment or anti-CTLA-4 alone.

### Example 7

### MVA-BN-HER2 and anti-CTLA-4 Decreases Tumor Burden

In the solid tumor model, female BALB/c mice were implanted on day 1 with CT26-HER-2 cells (1.0×10^5, i.d. in the dorsal flank). Mice were treated on day 1 and 15 with MVA-BN-HER2 (1E7 Inf. U. in 100 µL TBS, s.c. at the tail base) and 22 µg anti-CTLA-4 (1 mg/kg) on days 1 and 15. Tumors were measured twice weekly and the tumor volume calculated according to the formula: tumor volume (mm3) = (length × width2)/2.

Results. Shown in Figure 6, the results demonstrate that MVA-BN-HER2 in combination with low dose anti-CTLA-4 significantly reduced tumor burden by day 20 compared to other treatments. ^{∗∗∗∗} p<0.0001, ^{∗} p<0.05, Two way ANOVA.

### Example 8

### MVA-BN-HER2 and anti-PD-1 Treatment

Mice were implanted i.v. with 5×10⁴ CT26-HER-2 cells and treated with MVA-BN-HER2 as described in Example 2. Mice were treated with anti-PD-1 on days 4 and 18 at 200 µg (A, 10 mg/kg), 66 ug (B, 3 mg/kg), or 22 µg (C, 1 mg/kg) i.p. in 100 µL PBS. ^{∗∗∗∗} p<0.0001, ^{∗} p<0.05, ns=not significant by Log-Rank Test.

*Results.* In Figure 7, the results show MVA-BN-HER2 in combination with anti-PD-1.

### Example 9

### MVA-BN-HER2 in Combination with anti-CTLA-4 and anti-1 PD-1 Treatment Increases Overall Survival Rate at lower dosages

Mice were implanted i.v. with 5×10⁴ CT26-HER-2 cells and treated with MVA-BN-HER2 as described in Example 2. Mice were treated anti-CTLA-4 and anti-PD-1 on days 3 and 17 at 200 µg (A, 10 mg/kg), 66 µg (B, 3 mg/kg), or 22 µg (C, 1 mg/kg) each antibody i.p. in 100 µL PBS. ^{∗∗∗∗} p<0.0001, Log-Rank Test.

*Results.* Shown in Figure 8, the results demonstrate that MVA-BN-HER2 in combination with anti-CTLA-4 and anti-PD-1 significantly increased overall survival rate of subjects at each dosage concentration as compared to treatment of cancers with either MVA-BN-HER2 or anti-CTLA-4 and anti-PD-1 alone. More importantly, survival rates were increased at the lower dosages of 3 mg/kg and 1 mg/kg antibodies for the MVA-BN-HER2 in combination with anti-CTLA-4 and anti-PD-1 as compared to MVA-BN-HER2 or anti-CTLA-4 and anti-PD-1 alone.

### Example 10

### MVA-BN-CV301 with anti-CTLA-4 and anti-PD-1 Increases Overall Survival Rate

Female C57/BL6 mice (6-8 weeks old, ~ 20 g, Simonsen Laboratories, Gilroy,CA) were implanted on day 1 i.v. with 1.0×10^6 MC38-MUC1 cells in 300 µL DPBS which forms tumors in the lungs. Mice were treated with MVA-BN-CV301 (4E5 Inf.U. subcutaneously, s.c. above the tail base) and treated with anti-CTLA-4 and anti-PD-1 (200 µg each) i.p. on days 4 and 18.

*Results.* Shown in Figure 9, the results demonstrate that MVA-BN-CV301 in combination with anti-CTLA-4 and anti-PD-1 significantly increased overall survival rate of subjects as compared to treatment of cancers with either MVA-BN-CV301 or anti-CTLA-4 and anti-PD-1 alone.

### Example 11

### Induction of an anti-tumor response in mice treated with PROSTVAC and antibodies

Male BALB/c mice (6-8 weeks old, -20 g, Simonsen Laboratories, Gilroy CA) were implanted on day 1 with E6 cells (1.5×10^5, i.d. in the dorsal flank), which forms solid tumors. Mice were treated on day 1 with PROSTVAC-V (2E7 Inf. U., s.c. at the tail base), and on days 8 and 15 with PROSTVAC-F (1E8 Inf. U., s.c. at the tail base). Mice were treated i.p. with anti-PD-1 and or (200 µg) on days 1 and 15. Tumors were measured twice weekly and the tumor volume calculated according to the formula: tumor volume (mm3) = (length × width2)/2.

### Example 12

### Induction of an anti-tumor response in mice treated with PROSTVAC and anti-PD-1

Mice were implanted i.d. with E6 tumors and treated with PROSTVAC and anti-PD-1 as described in Example 11. The results are shown in Figure 10.

### Example 13

### Induction of an anti-tumor response in mice treated with PROSTVAC and anti-LAG-3

Mice were implanted i.d. with E6 tumors and treated with PROSTVAC and anti-LAG-3 as described in Example 11. The results are shown in Figure 11.

### Example 14

### Induction of an anti-tumor response in mice treated with PROSTVAC and anti-PD-1 and anti-LAG-3

Mice were implanted i.d. with E6 tumors and treated with PROSTVAC, anti-PD-1 and anti-LAG-3 as described in Example 11. The results are shown in Figure 12.

### Example 15

### MVA-BN-HER2 and anti-CTLA-4 Decreases Tumor Burden

Mice were implanted on day 1 with CT26-HER-2 cells i.d as described in Example 7. Mice were treated with MVA-BN-HER2 on days 7 and 22 (1E7 Inf.U., t.s.), and anti-ICOS on days 1, 4, 8, 11, 15, 18, 22, 25 (200 µg i.p.). A) Average tumor growth. B) Tumor growth in individual mice. ^{∗∗∗∗}p<0.0001, ^{∗∗} p<0.01, Two way ANOVA. The results are shown in Figure 13.

### Example 16

### MVA-BN-HER2 in Combination with anti-PD-1 reduces tumor burden at lower dosages

Mice are implanted with CT26-HER-2 cells and treated with MVA-BN-HER2 as described in Example 7. Mice are treated with anti-PD-1 on days 1 and 15 at 200 µg (10 mg/kg), 66 µg (3 mg/kg), or 22 µg (1 mg/kg) i.p. in 100 µL PBS. ^{∗∗∗∗} p<0.0001, ^{∗∗∗} p<0.001, ^{∗∗} p <0.01, ^{∗} p<0.05, Two way ANOVA.

### Example 17

### MVA-BN-HER2 in Combination with anti-ICOS reduces tumor burden at lower dosages

Mice are implanted with CT26-HER-2 cells and treated with MVA-BN-HER2 as described in Example 7. Mice are treated with anti-ICOS on days 1 and 15 at 200 µg (10 mg/kg), 66 µg (3 mg/kg), or 22 µg (1 mg/kg) i.p. in 100 µL PBS. ^{∗∗∗∗} p<0.0001, ^{∗∗∗} p<0.001, ^{∗∗} p <0.01, ^{∗} p<0.05, Two way ANOVA.

### Example 18

### MVA-BN-HER2 in Combination with anti-PD-1 and anti-LAG-3 reduces tumor burden at lower dosages

Mice are implanted with CT26-HER-2 cells and treated with MVA-BN-HER2 as described in Example 7. Mice are treated with anti-PD-1 and anti-LAG-3 on days 1 and 15 at 200 µg (10 mg/kg), 66 µg (3 mg/kg), or 22 µg (1 mg/kg) for each antibody i.p. in 100 µL PBS. ^{∗∗∗∗} p<0.0001, ^{∗∗∗} p<0.001, ^{∗∗} p <0.01, ^{∗} p<0.05, Two way ANOVA.

### Example 19

### MVA-BN-HER2 in Combination with anti-CTLA-4and anti-ICOS reduces tumor burden at lower dosages

Mice are implanted with CT26-HER-2 cells and treated with MVA-BN-HER2 as described in Example 7. Mice are treated with anti-CTLA-4 and anti-ICOS on days 1 and 15 at 200 µg (10 mg/kg), 66 µg (3 mg/kg), or 22 µg (1 mg/kg) for each antibody i.p. in 100 µL PBS. ^{∗∗∗∗} p<0.0001, ^{∗∗∗} p<0.001, ^{∗∗} p <0.01, ^{∗} p<0.05, Two way ANOVA.

### Example 20

### MVA-BN-HER2 in Combination with anti-CTLA-4 reduces tumor burden with an increased second dosage

Mice are implanted with CT26-HER-2 cells and treated with MVA-BN-HER2 as described in Example 7. Mice are treated with anti-CTLA-4 on day 1 at 66 µg (3 mg/kg), 22 µg (1 mg/kg), or 2.2 µg (0.1 mg/kg) and day 15 at 200 µg (10 mg/kg) or 66 µg (3mg/kg) i.p. in 100 µL PBS. ^{∗∗∗∗} p<0.0001, ^{∗∗∗} p<0.001, ^{∗∗} p <0.01, ^{∗} p<0.05, Two way ANOVA.

### Example 21

### MVA-BN-HER2 in Combination with anti-PD-1 reduces tumor burden with an increased second dosage

Mice are implanted with CT26-HER-2 cells and treated with MVA-BN-HER2 as described in Example 7. Mice are treated with anti-PD-1 on day 1 at 66 µg (3 mg/kg), 22 µg (1 mg/kg), or 2.2 µg (0.1 mg/kg) and day 15 at 200 µg (10 mg/kg) or 66 µg (3mg/kg) i.p. in 100 µL PBS. ^{∗∗∗∗} p<0.0001, ^{∗∗∗} p<0.001, ^{∗∗} p <0.01, ^{∗} p<0.05, Two way ANOVA.

### Example 22

### MVA-BN-HER2 in Combination with anti-ICOS reduces tumor burden with an increased second dosage

Mice are implanted with CT26-HER-2 cells and treated with MVA-BN-HER2 as described in Example 7. Mice are treated with anti-ICOS on day 1 at 66 µg (3 mg/kg), 22 µg (1 mg/kg), or 2.2 µg (0.1 mg/kg) and day 15 at 200 µg (10 mg/kg) or 66 µg (3mg/kg) i.p. in 100 µL PBS. ^{∗∗∗∗} p<0.0001, ^{∗∗∗} p<0.001, ^{∗∗} p <0.01, ^{∗} p<0.05, Two way ANOVA.

### Example 23

### MVA-BN-HER2 in Combination with anti-CTLA-4 and anti-PD-1 reduces tumor burden with an increased second dosage

Mice are implanted with CT26-HER-2 cells and treated with MVA-BN-HER2 as described in Example 7. Mice are treated with anti-CTLA-4 and anti-PD-1 on day 1 at 66 µg (3 mg/kg), 22 µg (1 mg/kg), or 2.2 µg (0.1 mg/kg) for each antibody and day 15 at 200 µg (10 mg/kg) or 66 µg (3mg/kg) for each antibody i.p. in 100 µL PBS. ^{∗∗∗∗} p<0.0001, ^{∗∗∗} p<0.001, ^{∗∗} p <0.01, ^{∗} p<0.05, Two way ANOVA.

### Example 24

### MVA-BN-HER2 in Combination with anti-PD-1 and anti-LAG-3 reduces tumor burden with an increased second dosage

Mice are implanted with CT26-HER-2 cells and treated with MVA-BN-HER2 as described in Example 7. Mice are treated with anti-PD-1 and anti-LAG-3 on day 1 at 66 µg (3 mg/kg), 22 µg (1 mg/kg), or 2.2 µg (0.1 mg/kg) for each antibody and day 15 at 200 µg (10 mg/kg) or 66 µg (3mg/kg) for each antibody i.p. in 100 µL PBS. ^{∗∗∗∗} p<0.0001, ^{∗∗∗} p<0.001, ^{∗∗} p <0.01, ^{∗} p<0.05, Two way ANOVA.

### Example 25

### MVA-BN-HER2 in Combination with anti-CTLA-4 and anti-ICOS reduces tumor burden with an increased second dosage

Mice are implanted with CT26-HER-2 cells and treated with MVA-BN-HER2 as described in Example 7. Mice are treated with anti-CTLA-4 and anti-ICOS on day 1 at 66 µg (3 mg/kg), 22 µg (1 mg/kg), or 2.2 µg (0.1 mg/kg) for each antibody and day 15 at 200 µg (10 mg/kg) or 66 µg (3mg/kg) for each antibody i.p. in 100 µL PBS. ^{∗∗∗∗} p<0.0001, ^{∗∗∗} p<0.001, ^{∗∗} p <0.01, ^{∗} p<0.05, Two way ANOVA.

### Example 26

### MVA-BN-HER2 in Combination with anti-CTLA-4 reduces tumor burden with poxvirus prior to anti-CTLA-4

Mice are implanted with CT26-HER-2 cells and treated with MVA-BN-HER2 as described in Example 7. Mice are treated with anti-CTLA-4 on day 3 at 66 µg (3 mg/kg), 22 µg (1 mg/kg), or 2.2 µg (0.1 mg/kg) and day 18 at 200 µg (10 mg/kg) or 66 µg (3mg/kg) i.p. in 100 µL PBS. ^{∗∗∗∗} p<0.0001, ^{∗∗∗} p<0.001, ^{∗∗} p <0.01, ^{∗} p<0.05, Two way ANOVA.

Mice are implanted with CT26-HER-2 cells and treated with MVA-BN-HER2 as described in Example 7. Mice are treated with anti-CTLA-4 on day 7 at 66 µg (3 mg/kg), 22 µg (1 mg/kg), or 2.2 µg (0.1 mg/kg) and day 21 at 200 µg (10 mg/kg) or 66 µg (3mg/kg) i.p. in 100 µL PBS. ^{∗∗∗∗} p<0.0001, ^{∗∗∗} p<0.001, ^{∗∗} p <0.01, ^{∗} p<0.05, Two way ANOVA.

### Example 27

### MVA-BN-HER2 in Combination with anti-PD-1 reduces tumor burden with poxvirus prior to anti-PD-1

Mice are implanted with CT26-HER-2 cells and treated with MVA-BN-HER2 as described in Example 7. Mice are treated with anti-PD-1 on day 3 at 66 µg (3 mg/kg), 22 µg (1 mg/kg), or 2.2 µg (0.1 mg/kg) and day 18 at 200 µg (10 mg/kg) or 66 µg (3mg/kg) i.p. in 100 µL PBS. ^{∗∗∗∗} p<0.0001, ^{∗∗∗} p<0.001, ^{∗∗} p <0.01, ^{∗} p<0.05, Two way ANOVA.

Mice are implanted with CT26-HER-2 cells and treated with MVA-BN-HER2 as described in Example 7. Mice are treated with anti-PD-1 on day 7 at 66 µg (3 mg/kg), 22 µg (1 mg/kg), or 2.2 µg (0.1 mg/kg) and day 21 at 200 µg (10 mg/kg) or 66 µg (3mg/kg) i.p. in 100 µL PBS. ^{∗∗∗∗} p<0.0001, ^{∗∗∗} p<0.001, ^{∗∗} p <0.01, ^{∗} p<0.05, Two way ANOVA.

### Example 28

### MVA-BN-HER2 in Combination with anti-ICOS reduces tumor burden with poxvirus prior to anti-ICOS

Mice are implanted with CT26-HER-2 cells and treated with MVA-BN-HER2 as described in Example 7. Mice are treated with anti-ICOS on day 3 at 66 µg (3 mg/kg), 22 µg (1 mg/kg), or 2.2 µg (0.1 mg/kg) and day 18 at 200 µg (10 mg/kg) or 66 µg (3mg/kg) i.p. in 100 µL PBS. ^{∗∗∗∗} p<0.0001, ^{∗∗∗} p<0.001, ^{∗∗} p <0.01, ^{∗} p<0.05, Two way ANOVA.

Mice are implanted with CT26-HER-2 cells and treated with MVA-BN-HER2 as described in Example 7. Mice are treated with anti-ICOS on day 7 at 66 µg (3 mg/kg), 22 µg (1 mg/kg), or 2.2 µg (0.1 mg/kg) and day 21 at 200 µg (10 mg/kg) or 66 µg (3mg/kg) i.p. in 100 µL PBS. ^{∗∗∗∗} p<0.0001, ^{∗∗∗} p<0.001, ^{∗∗} p <0.01, ^{∗} p<0.05, Two way ANOVA.

### Example 29

### MVA-BN-HER2 in Combination with anti-CTLA-4 and anti-PD-1 reduces tumor burden with poxvirus prior to anti-CTLA-4 and anti-PD-1

Mice are implanted with CT26-HER-2 cells and treated with MVA-BN-HER2 as described in Example 7. Mice are treated with anti-CTLA-4 and anti-PD-1 on day 3 at 66 µg (3 mg/kg), 22 µg (1 mg/kg), or 2.2 µg (0.1 mg/kg) for each antibody and day 18 at 200 µg (10 mg/kg) or 66 µg (3mg/kg) for each antibody i.p. in 100 µL PBS. ^{∗∗∗∗} p<0.0001, ^{∗∗∗} p<0.001, ^{∗∗} p <0.01, ^{∗} p<0.05, Two way ANOVA.

Mice are implanted with CT26-HER-2 cells and treated with MVA-BN-HER2 as described in Example 7. Mice are treated with anti-CTLA-4 and anti-PD-1 on day 7 at 66 µg (3 mg/kg), 22 µg (1 mg/kg), or 2.2 µg (0.1 mg/kg) for each antibody and day 21 at 200 µg (10 mg/kg) or 66 µg (3mg/kg) for each antibody i.p. in 100 µL PBS. ^{∗∗∗∗} p<0.0001, ^{∗∗∗} p<0.001, ^{∗∗} p <0.01, ^{∗} p<0.05, Two way ANOVA.

### Example 30

### MVA-BN-HER2 in Combination with anti-PD-1 and anti-LAG-3 reduces tumor burden with poxvirus prior to anti-PD-1 and anti-LAG-3

Mice are implanted with CT26-HER-2 cells and treated with MVA-BN-HER2 as described in Example 7. Mice are treated with anti-PD-1 and anti-LAG-3 on day 3 at 66 µg (3 mg/kg), 22 µg (1 mg/kg), or 2.2 µg (0.1 mg/kg) for each antibody and day 18 at 200 µg (10 mg/kg) or 66 µg (3mg/kg) for each antibody i.p. in 100 µL PBS. ^{∗∗∗∗} p<0.0001, ^{∗∗∗} p<0.001, ^{∗∗} p <0.01, ^{∗} p<0.05, Two way ANOVA.

Mice are implanted with CT26-HER-2 cells and treated with MVA-BN-HER2 as described in Example 7. Mice are treated with anti-PD-1 and anti-LAG-3 on day 7 at 66 µg (3 mg/kg), 22 µg (1 mg/kg), or 2.2 µg (0.1 mg/kg) for each antibody and day 21 at 200 µg (10 mg/kg) or 66 µg (3mg/kg) for each antibody i.p. in 100 µL PBS. ^{∗∗∗∗} p<0.0001, ^{∗∗∗} p<0.001, ^{∗∗} p <0.01, ^{∗} p<0.05, Two way ANOVA.

### Example 31

### MVA-BN-HER2 in Combination with anti-CTLA-4 and anti-ICOS reduces tumor burden with poxvirus prior to anti-CTLA-4 and ICOS

Mice are implanted with CT26-HER-2 cells and treated with MVA-BN-HER2 as described in Example 7. Mice are treated with anti-CTLA-4 and anti-ICOS on day 3 at 66 µg (3 mg/kg), 22 µg (1 mg/kg), or 2.2 µg (0.1 mg/kg) for each antibody and day 18 at 200 µg (10 mg/kg) or 66 µg (3mg/kg) for each antibody i.p. in 100 µL PBS. ^{∗∗∗∗} p<0.0001, ^{∗∗∗} p<0.001, ^{∗∗} p <0.01, ^{∗} p<0.05, Two way ANOVA.

Mice are implanted with CT26-HER-2 cells and treated with MVA-BN-HER2 as described in Example 7. Mice are treated with anti-CTLA-4 and anti-ICOS on day 7 at 66 µg (3 mg/kg), 22 µg (1 mg/kg), or 2.2 µg (0.1 mg/kg) for each antibody and day 21 at 200 µg (10 mg/kg) or 66 µg (3mg/kg) for each antibody i.p. in 100 µL PBS. ^{∗∗∗∗} p<0.0001, ^{∗∗∗} p<0.001, ^{∗∗} p <0.01, ^{∗} p<0.05, Two way ANOVA.

### Example 32

### Increase in Tim-3 Expression with MVA-BN-HER2 Treatment

Female BALB/c mice (6-8 weeks old, ~ 20 g, Simonsen Laboratories, Gilroy,CA) were treated on day 1 and day 15 with 7.1 µL of 1.0×10^7 Inf. U. MVA-BN-HER2 by tail scarification (t.s., produced by Bavarian Nordic [BN], Martinsried, Germany). Tissues were collected, processed, and stained as described in Example 2, and surface expression of Tim-3 was measured at various intervals.

*Results.* Shown in Figure 14, subjects were administered MVA-BN-HER-2 after which expression levels of Tim-3 were measured at regular intervals. In response to the treatment, there was an initial period of very little increase in immune checkpoint expression. From about day 3 to about day 12 T-cell expression of TIM-3 increased dramatically. Increased expression was still seen until about day 18. With a second treatment of MVA-BN-HER2 on day 15, Tim 3 expression increased starting at day 17. Even more significant, this increase in immune checkpoint expression was more profound in CD8 T-cells as compared to CD4 T-cells.

Shown in Figure 14, treatment with MVA-BN-HER2 transiently induced expression of Tim-3 on T cells. In one aspect, this induced expression of Tim-3 on T-cells is indicative that blocking the Tim-3 pathway via one or more TIM-3 antagonists can enhance a cancer patient's T-cell response.

### Example 33

### MVA-BN-HER2 Treatment Increases ICOS on CD8⁺ and CD4⁺ T Cells

Mice were treated with MVA-BN-HER2 (1E7 Inf.U., t.s.) on day 1 and 15. Tissues were collected, processed, and stained as described in Example 2, and surface expression of ICOS was measured at various intervals.

*Results.* Shown in Figure 15, subjects were administered MVA-BN-HER-2 after which expression levels of ICOS were measured at regular intervals. In response to the treatment, there was an initial period of very little increase in immune checkpoint expression. After approximately 1-2 days, T-cell expression of ICOS showed a slight increase. From about day 3 to about day 12 T-cell expression of ICOS increased dramatically. Increased expression was still seen until about day 18. With a second treatment of MVA-BN-HER2 on day 15, ICOS expression increased starting at day 17. Even more significant, this increase in immune checkpoint expression was more profound in CD8 T-cells as compared to CD4 T-cells.

Shown in Figure 15, treatment with MVA-BN-HER2 transiently induced expression of ICOS on T cells. In one aspect, this induced expression of ICOS on T-cells is indicative that activating the ICOS pathway via one or more ICOS agonists can enhance a cancer patient's T-cell response.

### Example 34

### PD-1 Expression Increases with MVA-BN-HER2 Treatment

Mice were treated with MVA-BN-HER2 (1E7 Inf.U., t.s.) on day 1 and 15. Tissues were collected, processed, and stained as described in Example 2, and surface expression of PD-1 was measured at various intervals.

*Results.* The results are shown in Figure 16. Subjects were administered MVA-BN-HER-2 after which expression levels of PD-1 were measured at regular intervals. In response to the treatment, there was an initial period of very little increase in immune checkpoint expression. After approximately 1-2 days, T-cell expression of PD-1 showed a slight increase. From about day 3 to about day 12 T-cell expression of PD-1 increased dramatically. Increased expression was still seen until about day 18. With a second treatment of MVA-BN-HER2 on day 15, PD-1 expression increased starting at day 17. Even more significant, this increase in immune checkpoint expression was more profound in CD8 T-cells as compared to CD4 T-cells.

Shown in Figure 16, treatment with MVA-BN-HER2 transiently induced expression of PD-1 on T cells. In one aspect, this induced expression of PD-1 on T-cells is indicative that blocking the PD-1/PDL-1 pathway via one or more PD-1/PDL-1 agonists can enhance a cancer patient's T-cell response.

It is further suggested that blocking PD-1 induced by MVA-BN-HER2 may lead to LAG-3 co-expression and therapeutic benefit may be achieved with dual blockade of the PD-1/PD-L1 and LAG-3 pathway in combination with MVA-BN-HER2.

### Example 35

### LAG-3 Immune Response to MVA-BN-HER2

Mice were treated with MVA-BN-HER2 (1E7 Inf.U., t.s.) on day 1 and 15. Tissues were collected, processed, and stained as described in Example 2, and surface expression of LAG-3 was measured at various intervals.

*Results.* The results are shown in Figure 17. Subjects were administered MVA-BN-HER-2 after which expression levels of LAG-3 were measured at regular intervals. There was an increase in LAG-3 expression on CD4 or CD8 T cells after MVA-BN-HER2 treatment.

### Example 36

### Induction of an anti-tumor response in mice treated with MVA-BN-CV301 and antibodies

Female C57BL/6 mice (6-8 weeks old, -20 g, Simonsen Laboratories, Gilroy CA) were implanted in day 1 with MC38-CEA cells (2×105, i.d. in the back flank). Mice were treated on day 1 and 15 with MVA-BN-CV301 (1E7 Inf.U., s.c. above the tail base). Mice were treated i.p. with anti-PD-1 and/or anti-LAG-3 as described each of the Figures and examples and as described in in Example 2.

### Example 37

### Induction of an anti-tumor response in mice treated with MVA-BN-CV301 and anti-PD-1

C57/BL6 mice were implanted i.d. with MC38-CEA tumors and treated as described in Example 36. The results are shown in Figure 18.

### Example 38

### Induction of an anti-tumor response in mice treated with MVA-BN-CV301 and anti-LAG-3

C57/BL6 mice were implanted i.d. with MC38-CEA tumors and treated as described in Example 36. The results are shown in Figure 19.

### Example 39

### Induction of an anti-tumor response in mice treated with MVA-BN-CV301 and anti-PD-1

### and anti-LAG-3

C57/BL6 mice were implanted i.d. with MC38-CEA tumors and treated as described in Example 36. The results are shown in Figure 20.

### Example 40

### Heterologous prime boost amplifies PSA-specific T cell responses

BALB/c males (5/group) were treated every two weeks with: Buffer (Control), PROSTVAC-V (VVV) (2E6 Inf. U., s.c. at the tail base), PROSTVAC-F (FFF) (1E7 Inf. U., s.c. at the tail base), or received a PROSTVAC-V prime followed by 2 PROSTVAC-F boosts (VFF). Pooled splenocytes were assayed for PSA-specific responses by IPNγ ELISPOT as described in Mandl et al. Cancer Immunol. Immunother (2012), 61:19-29, which is incorporated by reference herein.

(A, B) and cytotoxic activity by flow cytometry (% CD107+ IFNγ+ CD8 T cells) (C). Anti-PSA IgG titers were determined by ELISA for each individual mouse (D). For ELISPOT, splenocytes were restimulated with CD4 or CD8 PSA-specific peptides or controls (controls not shown at indicated concentrations. Responses that were too numerous to count were displayed as 1000 spots/million cells. Statistical significance was determined by RM-ANOVA with Tukey post-test at 0.01 µM. ^{∗∗∗∗}P < 0.001 compared to control (A & B). To identify cytotoxic CD8+ T cells, splenocytes were restimulated overnight with a PSA CD8-specific peptide in the presence of anti-CD107 antibody. Graphs show representative data of four independently performed experiments.

Shown in Figure 21, the heterologous prime-boost regimen with Vaccinia virus followed by one or more Fowlpoxvirus boost doses resulted in a much higher frequency of IFNγ-producing PSA-specific CD4 T cells (Figure 21A) and CD8 T cells (Figures 21B and 22A) compared to VVV or FFF homologous dosing regimens.

Moreover, PSA-specific T cells from VFF dosing were of higher avidity (Fig. 21A and 21B), as evidenced by higher frequencies of T cells responding at the lower 0.01 µM peptide concentrations in the ELISPOT. Importantly, the number of functionally active PSA-specific CD8 CTLs resulting from the VFF heterologous prime-boost regimen was 7 to 20 fold higher than those generated by either homologous dosing regimen (Figure 21C).

In contrast to the T cell responses, the heterologous prime-boost regimen did not improve PSA-specific antibody responses (Figure 21 D). These results indicate that heterologous VFF dosing generates CD4 and CD8 PSA-specific T cell responses of greater magnitude and higher quality as measured by higher avidity and increased CD8 CTL activity. As described herein, these contribute to improved anti-PSA specific anti-tumor responses following heterologous PROSTVAC-V/F dosing.

### Example 41

### Heterologous prime-boost improves the quality of PSA-specific T cell responses

BALB/c males (5/group) were treated as described in Example 40. Spleens were harvested 14 days after the last treatment, and pooled splenocytes were restimulated overnight with PSA OPL or controls (controls not shown). The cells were stained for intracellular IFNγ, TNFα, and IL-2 prior to flow cytometric analysis. (A) The pie charts are weighted in size to reflect the numbers of detected cells (total numbers of PSA-specific CD8 per million T cells are indicated below each chart). (B) Amount of IFNγ production on a per cell basis as measured by mean fluorescence intensity (MFI). Graphs show representative data of two independently performed experiments.

Shown in Figure 22, additional distinguishing features in the quality of the PSA-specific CD8 T cell response were observed when PSA-specific CD8 T cells were analyzed for the multicytokine-production of IFNγ, TNFα, and IL-2 by flow cytometry (Figure 22). Using cytokine expression, CD8 memory T cells have been classified as double-positive CD8 effector memory T cells (IFNγ+ TNFα+, TEM and as triple-positive CD8 central memory T cells (IFNγ+ TNFα+ IL-2+; TCM) *See, e.g.,* Nat Rev Immunol 2008, 8:247-258.

In addition to the increased magnitude of the CD8 T cell response (Fig. 21 and Fig. 22A), a pronounced shift in the quality of the CD8 T cell response was revealed by the higher proportion of double-positive TEM and triple-positive TCM (Fig. 22A) as a result of the heterologous PROSTVAC-V/F regimen compared to homologous dosing regimen. Priming with a 5 fold higher PROSTVAC-V dose did not yield any additional benefit in the magnitude or the quality of the CD8 T cell response (data not shown). Further double-positive TEM and triple-positive TCM CD8 T cells produced higher levels of IPNγ on a per cell basis than single positive cells (Fig. 22B). This increased IPNγ production was observed in TEM and TCM CD8 T cells regardless of dosing regimen.

Additionally, shown in Figure 22, MVA-BN-HER2 induces tumor antigen specific T cells that produce IFNγ. It is contemplated by the present disclosure that virus induced TILs (tumor infiltrating lymphocytes) that secrete IPNγ may lead to increased PD-L1 on tumor cells; supporting blockade of this pathway in combination with virus treatment.

### Example 42

### Immune focusing of T cell response towards PSA

Mice were treated as described in Example 40. Pooled splenocytes were assayed for vaccinia virus (VV)-specific (A and C panels on left) or PSA-specific (A and C panels on right) cytotoxic activity by flow cytometry (% CD107+ IFNγ+ CD8 T cells) 14 days after the last treatment. Splenocytes were restimulated overnight with vaccinia E3L and F2L peptides or with PSA OPL in the presence of anti-CD 107 antibody. The following day, cells were stained intracellularly for IPNγ and with the surface markers CD127 and KLRG1. % antigen-specific cytotoxic SLEC and DPEC were determined by gating on (CD8 +CD127-KLRG1+) and (CD8+CD127+KLRG1+) cells, respectively. Graphs show representative data of two independently performed experiments. Results are shown in Figure 23.

The impact of heterologous PROSTVAC vaccinavirus Fowlpox/F dosing regimen compared to homologous dosing on the cytotoxic capabilities of vector-specific vs. PSA-specific effector T cell subsets was analyzed. Homologous VVV dosing generated a relatively high number of vaccinia-specific cytotoxic SLEC (~50%) and DPEC (~20%) (Figure. 23A and 23C), yet less than 10% of SLEC or DPEC cytotoxic CD8 T cells were PSA-specific. Conversely, 65% of SLEC and 30% of the highly active DPEC effector memory T cells were PSA-specific CTL following heterologous VFF dosing, while less than 10% constituted vaccinia-specific CTL (Figures 23A ,and 23C). Therefore, the heterologous PROSTVAC-V/F regimen resulted in a 100 fold improvement in the ratio of PSA-targeted to vaccinia-targeted SLEC and DPEC T cell responses (Figures 23B and 23D). Again, priming with 5 fold more PROSTVAC-V did not yield any additional benefit (data not shown).

### Example 43

### Combination therapy with CTLA-4 after immune focusing

BALB/c males (5/group) are treated every two weeks with: Buffer (Control), PROSTVAC-V prime followed by 2 PROSTVAC-F boosts (VFF) as described in example 40. Mice are treated i.p. with anti-CTLA-4 (60 µg) on days 1, 15 and 29 (A), or on days 15 and 29 (B) , or on day s16 and 30 (C) or on days 17 and 31.(D). PSA specific T cell responses are analyzed as described in examples 40, 41 and 42.

As described herein and demonstrated by present application, administering a recombinant poxvirus comprising a TAA in combination with one or more immune checkpoint antagonists or agonists is therapeutically effective at various dosages. In particular, the disclosed combinations are effective at dosages that are lower than those dosages using one or more immune checkpoint antagonists or agonists or poxviral therapies by themselves.

The present application additionally demonstrates that efficacy of the recombinant poxvirus and immune checkpoint antagonist or agonist combination is greatly enhanced dependent upon the dosing regimen used during treatment. For example, administering one or more of the immune checkpoint antagonist or agonist after a recombinant poxviral treatment greatly enhances efficacy of the combination treatment. Additionally, increasing a dosage amount of an immune checkpoint antagonist or agonist after a second or subsequent recombinant poxviral treatment greatly enhances efficacy of the combination treatments described herein.

The present application additionally demonstrates the dosing regimens described herein are important for the greatly enhanced efficacy of the recombinant poxvirus and immune checkpoint antagonist or agonist combination therapy.

Significantly, the present disclosure demonstrates that a poxviral vector having a TAA when administered in combination with an immune checkpoint antagonist or agonist can provide increased therapeutic effect independent of the type of poxviral vector. In particular, the present disclosure demonstrates increased therapeutic effect when multiple types of poxviruses are used in the combination treatment. For example, therapeutic effects were achieved when a poxvirius, such as an orthopoxvirus or an avipoxvirus was administered with an immune checkpoint antagonist or agonist as described herein.

## Claims

1. A pharmaceutical combination or medicament for use in treating a human cancer patient, the combination or medicament comprising:
(a) a therapeutical effective amount of recombinant poxvirus encoding a polypeptide comprising at least one tumor-associated antigen (TAA) selected from the group consisting of carcinoembryonic (CEA), mucin 1 cell surface associated (MUC-1), prostate specific antigen (PSA), human epidermal growth factor 2 (HER-2), and Brachyury; and
(b) a subtherapeutically effective amount of an immune checkpoint antagonist that is a PD-1 antagonist or a CTLA-4 antagonist, wherein the pharmaceutical combination or medicament comprises a PD-1 antagonist and a CTLA-4 antagonist, and wherein each of said PD-1 antagonist and CTLA-4 antagonist is an antibody, an antisense RNA, or an siRNA.

2. The combination or medicament for use of claim 1, wherein the PD-1 antagonist comprises an anti-PD-1 antibody and the CTLA-4 antagonist comprises an anti-CTLA-4 antibody.

3. The combination or medicament for use of any of claims 1-2, wherein the poxvirus is selected from an orthopoxvirus and an avipoxvirus.

4. The combination or medicament for use of claim 3, wherein the orthopoxvirus is a vaccinia virus, preferably a modified vaccinia Ankara (MVA) virus, more preferably MVA-BN.

5. The combination or medicament for use of claim 3, wherein the avipoxvirus is a fowlpox virus.

6. The combination or medicament for use of any of claims 1-5, wherein the at least one TAA is human epidermal growth factor receptor 2 (HER-2).

7. The combination or medicament for use of any of claims 1-6, wherein the at least one TAA comprises a CEA antigen and MUC-1 antigen.

8. The combination or medicament for use of any of claims 1-7, wherein the at least one TAA is prostate specific antigen (PSA).

9. The combination or medicament for use of claim 1, wherein the cancer treatment is directed against breast cancer, lung cancer, head and neck cancer, thyroid, melanoma, gastric cancer, bladder cancer, kidney cancer, liver cancer, melanoma, pancreatic cancer, prostate cancer, ovarian cancer, or colorectal cancer.

10. The combination or medicament for use of any of claims 1-9, wherein at least one of the immune checkpoint antagonists is administered at an amount of about 1mg/kg to about 3mg/kg of the patient's body weight, preferably about 1mg/kg of the patient's body weight.

11. The combination or medicament for use of any of claims 1-10, wherein the therapeutically effective amount of a recombinant poxvirus in combination with a subtherapeutically effective amount of an immune checkpoint antagonist according to (b) is administered as part of a homologous or heterologous prime-boost regimen;
wherein the homologous prime-boost regimen comprises a first prime dose of the recombinant poxvirus in combination with the immune checkpoint antagonist and one or more subsequent boost doses of a same recombinant poxvirus in combination with the immune checkpoint antagonist; and
wherein the heterologous prime-boost regimen comprises a first prime dose of the recombinant poxvirus in combination with the immune checkpoint antagonist and one or more subsequent boost doses of a different recombinant poxvirus in combination with the immune checkpoint antagonist.

12. The combination or medicament for use of claim 11, wherein the recombinant poxvirus in combination with the immune checkpoint antagonist is administered as part of heterologous prime-boost regimen, wherein the recombinant poxvirus of the first prime dose comprises an orthopoxvirus and the recombinant poxvirus of one or more subsequent boosting doses is an avipoxvirus.

13. The combination or medicament for use of claim 11, wherein the orthopoxvirus is selected from vaccinia virus, MVA, or MVA-BN.

## Patentansprüche

1. Pharmazeutische Kombination oder Arzneimittel zur Verwendung bei der Behandlung eines menschlichen Krebspatienten, wobei die Kombination oder das Arzneimittel umfasst:
(a) eine therapeutisch wirksame Menge an rekombinantem Pockenvirus, das für ein Polypeptid kodiert, das mindestens ein tumorassoziiertes Antigen (TAA) umfasst, ausgewählt aus der Gruppe bestehend aus karzinoembryonalem (CEA), Mucin-1-Zelloberflächen-assoziiertem (MUC-1), Prostata-spezifischem Antigen (PSA), humanem epidermalen Wachstumsfaktor 2 (HER-2) und Brachyury; und
(b) eine subtherapeutisch wirksame Menge eines Immun-Checkpoint-Antagonisten, der ein PD-1-Antagonist oder ein CTLA-4-Antagonist ist, wobei die pharmazeutische Kombination oder das Arzneimittel einen PD-1-Antagonisten und einen CTLA-4-Antagonisten umfasst, und wobei sowohl der PD-1-Antagonist als auch der CTLA-4-Antagonist ein Antikörper, eine Antisense-RNA oder eine siRNA ist.

2. Kombination oder Arzneimittel zur Verwendung nach Anspruch 1, wobei der PD-1-Antagonist einen Anti-PD-1-Antikörper umfasst und der CTLA-4-Antagonist einen Anti-CTLA-4-Antikörper umfasst.

3. Kombination oder Arzneimittel zur Verwendung nach einem der Ansprüche 1-2, wobei das Pockenvirus ausgewählt ist aus einem Orthopoxvirus und einem Avipoxvirus.

4. Kombination oder Arzneimittel zur Verwendung nach Anspruch 3, wobei das Orthopoxvirus ein Vacciniavirus, vorzugsweise ein modifiziertes Vaccinia-Ankara-(MVA)-Virus, stärker bevorzugt MVA-BN ist.

5. Kombination oder Arzneimittel zur Verwendung nach Anspruch 3, wobei das Avipoxvirus ein Geflügelpockenvirus ist.

6. Kombination oder Arzneimittel zur Verwendung nach einem der Ansprüche 1-5, wobei das mindestens eine TAA humaner epidermaler Wachstumsfaktor-Rezeptor 2 (HER-2) ist.

7. Kombination oder Arzneimittel zur Verwendung nach einem der Ansprüche 1-6, wobei das mindestens eine TAA ein CEA-Antigen und ein MUC-1-Antigen umfasst.

8. Kombination oder Arzneimittel zur Verwendung nach einem der Ansprüche 1-7, wobei das mindestens eine TAA Prostata-spezifisches Antigen (PSA) ist.

9. Kombination oder Arzneimittel zur Verwendung nach Anspruch 1, wobei die Krebsbehandlung gegen Brustkrebs, Lungenkrebs, Kopf- und Halskrebs, Schilddrüsenkrebs, Melanom, Magenkrebs, Blasenkrebs, Nierenkrebs, Leberkrebs, Melanom, Bauchspeicheldrüsenkrebs, Prostatakrebs, Eierstockkrebs oder Darmkrebs gerichtet ist.

10. Kombination oder Arzneimittel zur Verwendung nach einem der Ansprüche 1-9, wobei mindestens einer der Immun-Checkpoint-Antagonisten in einer Menge von etwa 1 mg/kg bis etwa 3 mg/kg des Körpergewichts des Patienten, vorzugsweise etwa 1 mg/kg des Körpergewichts des Patienten verabreicht wird.

11. Kombination oder Arzneimittel zur Verwendung nach einem der Ansprüche 1-10, wobei die therapeutisch wirksame Menge eines rekombinanten Pockenvirus in Kombination mit einer subtherapeutisch wirksamen Menge eines Immun-Checkpoint-Antagonisten nach (b) als Teil eines homologen oder heterologen Prime-Boost-Regimes verabreicht wird;
wobei das homologe Prime-Boost-Regime eine erste Prime-Dosis des rekombinanten Pockenvirus in Kombination mit dem Immun-Checkpoint-Antagonisten und eine oder mehrere nachfolgende Boost-Dosen desselben rekombinanten Pockenvirus in Kombination mit dem Immun-Checkpoint-Antagonisten umfasst; und
wobei das heterologe Prime-Boost-Regime eine erste Prime-Dosis des rekombinanten Pockenvirus in Kombination mit dem Immun-Checkpoint-Antagonisten und eine oder mehrere nachfolgende Boost-Dosen eines anderen rekombinanten Pockenvirus in Kombination mit dem Immun-Checkpoint-Antagonisten umfasst.

12. Kombination oder Arzneimittel zur Verwendung nach Anspruch 11, wobei das rekombinante Pockenvirus in Kombination mit dem Immun-Checkpoint-Antagonisten als Teil eines heterologen Prime-Boost-Regimes verabreicht wird, wobei das rekombinante Pockenvirus der ersten Prime-Dosis ein Orthopoxvirus umfasst und das rekombinante Pockenvirus einer oder mehrerer nachfolgender Booster-Dosen ein Avipoxvirus ist.

13. Kombination oder Arzneimittel zur Verwendung nach Anspruch 11, wobei das Orthopoxvirus ausgewählt ist aus Vacciniavirus, MVA und MVA-BN.

## Revendications

1. Combinaison pharmaceutique ou médicament pour une utilisation dans le traitement d'un patient humain atteint d'un cancer, la combinaison ou le médicament comprenant :
(a) une quantité thérapeutique efficace d'un poxvirus recombinant codant pour un polypeptide comprenant au moins un antigène associé à une tumeur (AAT) choisi dans le groupe constitué par l'antigène carcinoembryonnaire (ACE), la mucine 1, associée à la surface cellulaire (MUC-1), l'antigène spécifique à la prostate (ASP), le facteur de croissance épidermique humain 2 (HER-2), et Brachyury ; et
(b) une quantité efficace de manière sous-thérapeutique d'un antagoniste de point de contrôle immunitaire qui est un antagoniste de PD-1 ou un antagoniste de CTLA-4, la combinaison pharmaceutique ou le médicament comprenant un antagoniste de PD-1 et un antagoniste de CTLA-4, et chacun parmi ledit un antagoniste de PD-1 et ledit antagoniste de CTLA-4 étant un anticorps, un ARN antisens ou un ARNsi.

2. Combinaison ou médicament pour une utilisation selon la revendication 1, l'antagoniste de PD-1 comprenant un anticorps anti-PD-1 et l'antagoniste de CTLA-4 comprenant un anticorps anti-CTLA-4.

3. Combinaison ou médicament pour une utilisation selon l'une quelconque des revendications 1 et 2, le poxvirus étant choisi parmi un orthopoxvirus et un avipoxvirus.

4. Combinaison ou médicament pour une utilisation selon la revendication 3, l'orthopoxvirus étant un virus de la vaccine, préférablement un virus Ankara de la vaccine modifié (MVA), plus préférablement l'MVA-BN.

5. Combinaison ou médicament pour une utilisation selon la revendication 3, l'avipoxvirus étant un virus de la variole aviaire.

6. Combinaison ou médicament pour une utilisation selon l'une quelconque des revendications 1 à 5, l'au moins un AAT étant le facteur de croissance épidermique humain 2 (HER-2).

7. Combinaison ou médicament pour une utilisation selon l'une quelconque des revendications 1 à 6, l'au moins un AAT comprenant l'antigène AEC et l'antigène MUC-1.

8. Combinaison ou médicament pour une utilisation selon l'une quelconque des revendications 1 à 7, l'au moins un AAT étant l'antigène spécifique à la prostate (ASP).

9. Combinaison ou médicament pour une utilisation selon la revendication 1, le traitement du cancer étant dirigé contre un cancer du sein, un cancer du poumon, un cancer de la tête et du cou, un cancer de la thyroïde, un mélanome, un cancer gastrique, un cancer de la vessie, un cancer du rein, un cancer du foie, un mélanome, un cancer pancréatique, un cancer de la prostate, un cancer de l'ovaire ou un cancer colorectal.

10. Combinaison ou médicament pour une utilisation selon l'une quelconque des revendications 1 à 9, au moins l'un parmi les antagonistes de point de contrôle immunitaire étant administré à raison d'une quantité d'environ 1 mg/kg à environ 3 mg/kg du poids corporel du patient, préférablement d'environ 1 mg/kg du poids corporel du patient.

11. Combinaison ou médicament pour une utilisation selon l'une quelconque des revendications 1 à 10, la quantité thérapeutiquement efficace d'un poxvirus recombinant en combinaison avec une quantité efficace de manière sous-thérapeutique d'un antagoniste de point de contrôle immunitaire selon (b) étant administrée selon un régime de primo-immunisation-rappel homologue ou hétérologue ;
le régime de primo-immunisation-rappel homologue comprenant une première dose de primo-immunisation du poxvirus recombinant en combinaison avec l'antagoniste de point de contrôle immunitaire et une ou plusieurs doses de rappel subséquentes d'un même poxvirus recombinant en combinaison avec l'antagoniste de point de contrôle immunitaire ; et
le régime de primo-immunisation-rappel hétérologue comprenant une première dose de primo-immunisation du poxvirus recombinant en combinaison avec l'antagoniste de point de contrôle immunitaire et une ou plusieurs doses de rappel subséquentes d'un poxvirus recombinant différent en combinaison avec l'antagoniste de point de contrôle immunitaire.

12. Combinaison ou médicament pour une utilisation selon la revendication 11, le poxvirus recombinant en combinaison avec l'antagoniste de point de contrôle immunitaire étant administré comme partie d'un régime de primo-immunisation-rappel hétérologue, le poxvirus recombinant de la première dose de primo-immunisation comprenant un orthopoxvirus et le poxvirus recombinant d'une ou plusieurs doses de rappel subséquentes étant un avipoxvirus.

13. Combinaison ou médicament pour une utilisation selon la revendication 11, l'orthopoxvirus étant choisi parmi le virus de la vaccine, MVA et MVA-BN.
